# EUROPÄISCHE PATENTSCHRIFT

(11) **EP 1 940 808 B1**
(45) Veröffentlichungstag und Bekanntmachung des Hinweises auf die Patenterteilung: **27.11.2013**
(21) Anmeldenummer: 06792404.3
(22) Anmeldetag: 09.10.2006
(51) Int. Cl.: C07D 257/04, C07C 229/14, C07C 57/42, A61K 31/41, A61K 31/195, A61P 9/00

(54) **DIFLUORPHENOL-DERIVATE UND IHRE VERWENDUNG**
DIFLUOROPHENOL DERIVATIVES AND THEIR USE
DERIVES DE DIFLUOROPHENOL ET LEUR UTILISATION

(30) Priorität: 21.10.2005 DE 102005050497
(43) Veröffentlichungstag der Anmeldung: 09.07.2008
(73) Patentinhaber: Bayer Intellectual Property GmbH, 40789 Monheim (DE)
(72) Erfinder: BARTEL, Stephan, 51515 Kürten (DE); HAHN, Michael, 40764 Langenfeld (DE); MORADI, Wahed, Ahmed, 40789 Monheim (DE); BECKER, Eva-Maria, 42117 Wuppertal (DE); RÖLLE, Thomas, 51381 Leverkusen (DE); STASCH, Johannes-Peter, 42651 Solingen (DE); SCHLEMMER, Karl-Heinz, 42113 Wuppertal (DE); WUNDER, Frank, 42117 Wuppertal (DE); KNORR, Andreas, 40699 Erkrath (DE)
(74) Vertreter: BIP Patents
(86) Internationale Anmeldenummer: PCT/EP2006/009726
(87) Internationale Veröffentlichungsnummer: WO 2007/045369

(56) Entgegenhaltungen:
- WO-A2-01/19780
- US-A1- 2004 082 658
- US-A1- 2004 176 446

## Beschreibung

Die vorliegende Anmeldung betrifft neue Difluorphenol-Derivate, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prävention kardiovaskulärer Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch

Biokonversion gebildet und aktiviert die lösliche Guanylatcyclase durch Angriffe am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxy-methyl-2'-furyl)-1-benzylindazol [YC-1, Wu et al., Blood 84 (1994), 4226; Mülsch et al., Br. J. Pharmacol. 120 (1997), 681], Fettsäuren [Goldberg et al., J. Biol. Chem. 252 (1977), 1279], Diphenyliodonium-hexafluorophosphat [Pettibone et al., Eur. J. Pharmacol. 116 (1985), 307], Isoliquiritigenin [Yu et al., Br. J. Pharmacol. 114 (1995), 1587], sowie verschiedene substituierte Pyrazolderivate (WO 98/16223, WO 98/16507 und WO 98/23619).

Die vorstehend beschriebenen Stimulatoren der löslichen Guanylatcyclase stimulieren das Enzym entweder direkt über die Häm-Gruppe (Kohlenmonoxid, Stickstoffmonoxid oder Diphenyliodoniumhexafluorophosphat) durch Interaktion mit dem Eisenzentrum der Häm-Gruppe und eine sich daraus ergebende, zur Erhöhung der Enzymaktivität führende Konformationsänderung [Gerzer et al., FEBS Lett. 132 (1981), 71] oder über einen Häm-abhängigen Mechanismus, der unabhängig von NO ist, aber zu einer Potenzierung der stimulierenden Wirkung von NO oder CO führt [z.B. YC-1, Hoenicka et al., J. Mol. Med. 77 (1999) 14; oder die in WO 98/16223, WO 98/16507 und WO 98/23619 beschriebenen Pyrazolderivate].

Die in der Literatur behauptete stimulierende Wirkung von Isoliquiritigenin und von Fettsäuren, wie z.B. von Arachidonsäure, Prostaglandin-Endoperoxiden und Fettsäure-Hydroperoxiden auf die lösliche Guanylatcyclase konnte nicht bestätigt werden [vgl. z.B. Hoenicka et al., J. Mol. Med. 77 (1999), 14].

Entfernt man von der löslichen Guanylatcyclase die Häm-Gruppe, zeigt das Enzym immer noch eine nachweisbare katalytische Basalaktivität, d.h. es wird nach wie vor cGMP gebildet. Die verbleibende katalytische Basalaktivität des Häm-freien Enzyms ist durch keinen der vorstehend genannten bekannten Stimulatoren stimulierbar.

Es wurde eine Stimulation von Häm-freier löslicher Guanylatcyclase durch Protoporphyrin IX beschrieben [Ignarro et al., Adv. Pharmacol. 26 (1994), 35]. Allerdings kann Protoporphyrin IX als Mimik für das NO-Häm-Addukt angesehen werden, weshalb die Zugabe von Protoporphyrin IX zur löslichen Guanylatcyclase zur Bildung einer der durch NO stimulierten Häm-haltigen löslichen Guanylatcyclase entsprechenden Struktur des Enzyms führen dürfte. Dies wird auch durch die Tatsache belegt, dass die stimulierende Wirkung von Protoporphyrin IX durch den vorstehend beschriebenen NO-unabhängigen, aber Häm-abhängigen Stimulator YC-1 erhöht wird [Mülsch et al., Naunyn Schmiedebergs Arch. Pharmacol. 355, R47].

Im Gegensatz zu den vorstehend beschriebenen Stimulatoren der löslichen Guanylatcyclase sind die Verbindungen der vorliegenden Erfindung in der Lage, sowohl die Häm-haltige als auch die Häm-freie Form der löslichen Guanylatcyclase zu aktivieren. Die Stimulierung des Enzyms verläuft bei diesen neuen Aktivatoren also über einen Häm-unabhängigen Weg, was auch dadurch belegt wird, dass die neuen Aktivatoren am Häm-haltigen Enzym einerseits keine synergistische Wirkung mit NO zeigen und andererseits sich die Wirkung dieser neuartigen Aktivatoren nicht durch den Häm-abhängigen Inhibitor der löslichen Guanylatcyclase, 1*H-*1,2,4-Oxadiazol-(4,3-a)-chinoxalin-1-on (ODQ), blockieren lässt.

In EP 0 341 551-A1 werden Alkensäure-Derivate als Leukotrien-Antagonisten für die Behandlung von Erkrankungen des Kreislauf- und Atmungssystems offenbart. In WO 01/19355, WO 01/19776, WO 01/19778, WO 01/19780, WO 02/070462, WO 02/070461 und WO 02/070510 werden Dicarbonsäure- bzw. Aminodicarbonsäure-Derivate als Stimulatoren der löslichen Guanylatcyclase zur Behandlung von Herz-Kreislauf-Erkrankungen beschrieben. Allerdings zeigte es sich, dass diese Verbindungen hinsichtlich ihrer pharmakokinetischen Eigenschaften Nachteile aufweisen, wie insbesondere eine geringe Bioverfügbarkeit und/oder eine nur kurze Wirkdauer nach oraler Gabe.

Aufgabe der vorliegenden Erfindung war daher die Bereitstellung neuer Verbindungen, welche als Aktivatoren der löslichen Guanylatcyclase wirken, jedoch nicht die vorstehend aufgeführten Nachteile der Verbindungen aus dem Stand der Technik aufweisen.

Gegenstand der vorliegenden Erfindung sind Verbindungen der allgemeinen Formel (I) 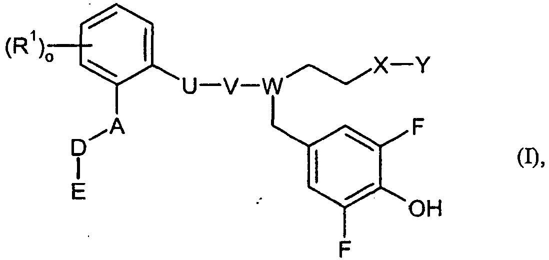 in welcher
U, V und W zusammen eine Gruppe der Formel *-CH=CH-CH<, *-CH₂-CH₂-CH< oder *-CH₂-CH₂-N< bilden, worin * die Verknüpfungsstelle mit dem Phenylring bedeutet,
- A: für O oder CH₂ steht,
- D: für eine Bindung oder für (C₁-C₇)-Alkandiyl, (C₂-C₇)-Alkendiyl oder (C₂-C₇)-Alkindiyl, welche jeweils ein- oder mehrfach mit Fluor substituiert sein können, steht, E für Wasserstoff, Trifluormethyl oder für eine Gruppe der Formel 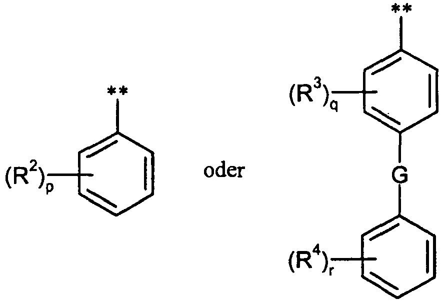 steht, worin ** die Verknüpfungsstelle mit der Gruppe D und
- G: eine Bindung, CH₂, -CH₂-CH₂- oder -CH=CH- bedeutet,
- X: für -CH₂-CH₂- oder eine Gruppe der Formel 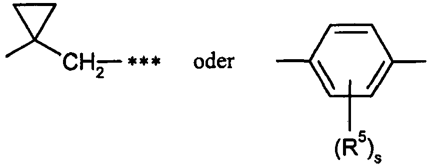 steht, worin *** die Verknüpfungsstelle mit der Gruppe Y bedeutet,
- Y: für Carboxyl oder eine Gruppe der Formel 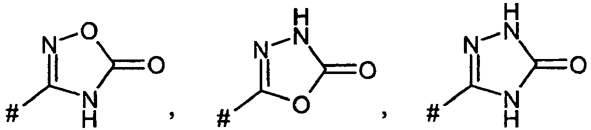 oder 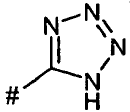 steht, worin # die jeweilige Verknüpfungsstelle bedeutet,
- R¹, R², R³, R⁴ und R⁵: unabhängig voneinander für einen Substituenten ausgewählt aus der Reihe Halogen, (C₁-C₆)-Alkyl, Trifluormethyl, (C₁-C₆)-Alkoxy, Trifluormethoxy, Cyano und Nitro stehen,
und
- o, p, q, r und s: unabhängig voneinander jeweils für die Zahl 0, 1, 2, 3 oder 4 stehen,
wobei für den Fall, dass R¹, R², R³, R⁴ oder R⁵ mehrfach auftreten, ihre Bedeutungen jeweils gleich oder verschieden sein können,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:

(C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, n-Propyl, Isopropyl, n-Butyl, iso-Butyl, sec.-Butyl, *tert*.-Butyl, 1-Ethylpropyl, n-Pentyl und n-Hexyl.

(C₁-C₇)-Alkandiyl steht im Rahmen der Erfindung für einen geradkettigen oder verzweigten divalenten Alkylrest mit 1 bis 7 Kohlenstoffatomen. Bevorzugt ist ein geradkettiger Alkandiylrest mit 1 bis 6 Kohlenstoffatomen. Beispielhaft und vorzugsweise seien genannt: Methylen, 1,2-Ethylen, Ethan-1,1-diyl, 1,3-Propylen, Propan-1,1-diyl, Propan-1,2-diyl, Propan-2,2-diyl, 1,4-Butylen, Butan-1,2-diyl, Butan-1,3-diyl, Butan-2,3-diyl, Pentan-1,5-diyl, Pentan-2,4-diyl, 3-Methyl-pentan-2,4-diyl und Hexan-1,6-diyl.

(C₂-C₇)-Alkendiyl steht im Rahmen der Erfindung für einen geradkettigen oder verzweigten divalenten Alkenylrest mit 2 bis 7 Kohlenstoffatomen und bis zu 3 Doppelbindungen. Bevorzugt ist ein geradkettiger Alkendiylrest mit 2 bis 6 Kohlenstoffatomen und bis zu 2 Doppelbindungen. Beispielhaft und vorzugsweise seien genannt: Ethen-1,1-diyl, Ethen-1,2-diyl, Propen-1,1-diyl, Propen-1,2-diyl, Propen-1,3-diyl, But-1-en-1,4-diyl, But-1-en-1,3-diyl, But-2-en-1,4-diyl, Buta-1,3-dien-1,4-diyl, Pent-2-en-1,5-diyl, Hex-3-en-1,6-diyl und Hexa-2,4-dien-1,6-diyl.

(C₂-C₇)-Alkindiyl steht im Rahmen der Erfindung für einen geradkettigen oder verzweigten divalenten Alkinylrest mit 2 bis 7 Kohlenstoffatomen und bis 3 Dreifachbindungen. Bevorzugt ist ein geradkettiger Alkindiylrest mit 2 bis 6 Kohlenstoffatomen und bis zu 2 Dreifachbindungen. Beispielhaft und vorzugsweise seien genannt: Ethin-1,2-diyl, Propin-1,3-diyl, But-1-in-1,4-diyl, But-1-in-1,3-diyl, But-2-in-1,4-diyl, Pent-2-in-1,5-diyl, Pent-2-in-1,4-diyl und Hex-3-in-1,6-diyl.

(C₁-C₆)-Alkoxy und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, n-Propoxy, Isopropoxy, n-Butoxy, *tert*.-Butoxy, n-Pentoxy und n-Hexoxy.

(C₁-C₄)-Alkoxycarbonyl steht im Rahmen der Erfindung für einen geradkettigen oder verzweigten Alkoxyrest mit 1 bis 4 Kohlenstoffatomen, der über eine Carbonylgruppe verknüpft ist. Beispielhaft und vorzugsweise seien genannt: Methoxycarbonyl, Ethoxycarbonyl, n-Propoxycarbonyl, Isopropoxycarbonyl und tert.-Butoxycarbonyl.

Halogen schließt im Rahmen der Erfindung Fluor, Chlor, Brom und Iod ein. Bevorzugt sind Chlor oder Fluor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Wenn ein Rest in den erfindungsgemäßen Verbindungen mehrfach mit Fluor substituiert sein kann, so schließt dies im Rahmen der vorliegenden Erfindung eine Perfluor-Substitution mit ein.

Bevorzugt im Rahmen der vorliegenden Erfindung sind Verbindungen der Formel (I), in welcher
- U, V und W: zusammen eine Gruppe der Formel *-CH=CH-CH< oder *-CH₂-CH₂-N< bilden, worin * die Verknüpfungsstelle mit dem Phenylring bedeutet,
- A: für O steht,
- D: für (C₁-C₇)-Alkandiyl, welches ein- oder mehrfach mit Fluor substituiert sein kann, steht,
- E: für Wasserstoff, Trifluormethyl oder für eine Gruppe der Formel 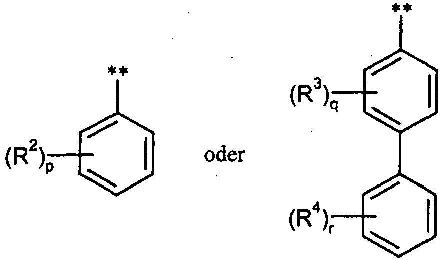 steht, worin ** die Verknüpfungsstelle mit der Gruppe D bedeutet,
- X: für -CH₂-CH₂- oder eine Gruppe der Formel 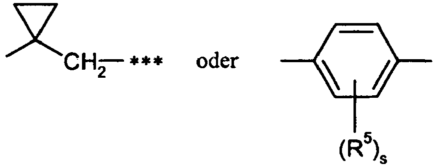 steht, worin *** die Verknüpfungsstelle mit der Gruppe Y bedeutet,
- Y: für Carboxyl oder eine Gruppe der Formel 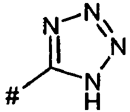 steht, worin # die Verknüpfungsstelle bedeutet,
- R¹, R², R³ und R⁴: unabhängig voneinander für einen Substituenten ausgewählt aus der Reihe Fluor, Chlor, Brom, (C₁-C₄)-Alkyl, Trifluormethyl, (C₁-C₄)-Alkoxy und Trifluormethoxy stehen,
- o, p, q und r: unabhängig voneinander jeweils für die Zahl 0, 1 oder 2 stehen, wobei für den Fall, dass R¹, R², R³ oder R⁴ mehrfach auftreten, ihre Bedeutungen jeweils gleich oder verschieden sein können,
- R⁵: für Fluor*
und
- s: für die Zahl 0 oder 1 steht,
sowie ihre Salze, Solvate und Solvate der Salze.

Besonders bevorzugt im Rahmen der vorliegenden Erfindung sind Verbindungen der Formel (I-A) 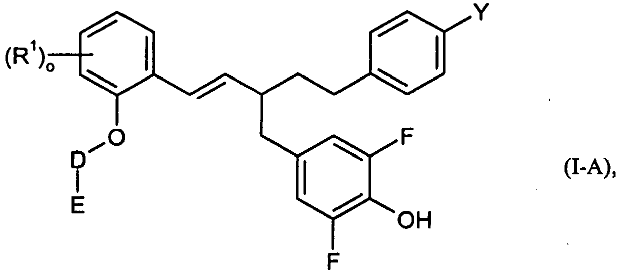 in welcher
- D: für (C₁-C₇)-Alkandiyl, welches ein- oder mehrfach mit Fluor substituiert sein kann, steht,
- E: für Wasserstoff, Trifluormethyl oder für eine Gruppe der Formel 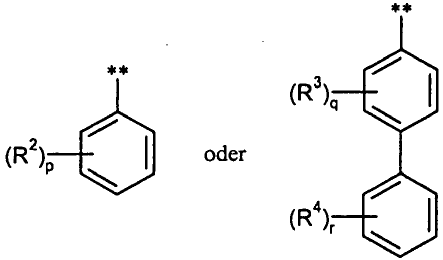 steht, worin ** die Verknüpfungsstelle mit der Gruppe D bedeutet,
- Y: für Carboxyl oder eine Gruppe der Formel 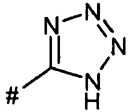 steht, worin # die Verknüpfungsstelle bedeutet,
- R¹, R², R³ und R⁴: unabhängig voneinander für einen Substituenten ausgewählt aus der Reihe Fluor, Chlor, Brom, Methyl, *tert*.-Butyl, Trifluormethyl und Methoxy stehen,
und
- o, p, q und r: unabhängig voneinander jeweils für die Zahl 0, 1 oder 2 stehen,
wobei für den Fall, dass R¹, R², R³ oder R⁴ mehrfach auftreten, ihre Bedeutungen jeweils gleich oder verschieden sein können,
sowie ihre Salze, Solvate und Solvate der Salze.

Besonders bevorzugt im Rahmen der vorliegenden Erfindung sind auch Verbindungen der Formel (I-B) 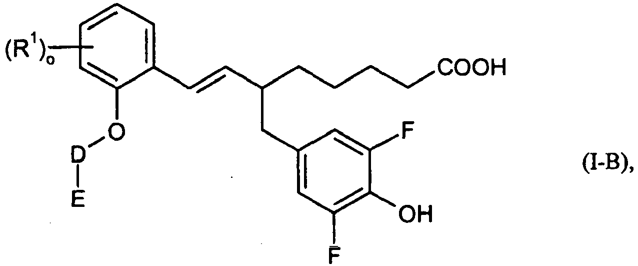 in welcher
- D: für (C₁-C₇)-Alkandiyl, welches ein- oder mehrfach mit Fluor substituiert sein kann, steht,
- E: für Wasserstoff, Trifluormethyl oder für eine Gruppe der Formel 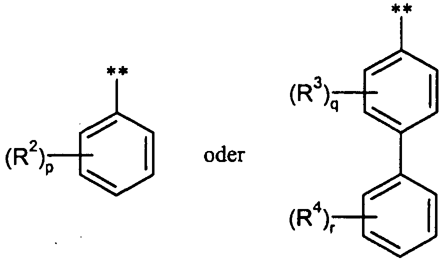 steht, worin ** die Verknüpfungsstelle mit der Gruppe D bedeutet,
- R¹, R², R³ und R⁴: unabhängig voneinander für einen Substituenten ausgewählt aus der Reihe Fluor, Chlor, Brom, Methyl, *tert*.-Butyl, Trifluormethyl und Methoxy stehen,
und
- o, p, q und r: unabhängig voneinander jeweils für die Zahl 0, 1 oder 2 stehen,
wobei für den Fall, dass R¹, R², R³ oder R⁴ mehrfach auftreten, ihre Bedeutungen jeweils gleich oder verschieden sein können,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), in welcher U, V und W zusammen eine Gruppe der Formel *-CH=CH-CH< bilden, worin * die Verknüpfungsstelle mit dem Phenylring bedeutet, und Y für Carboxyl steht,
dadurch gekennzeichnet, dass man Verbindungen der Formel (II) 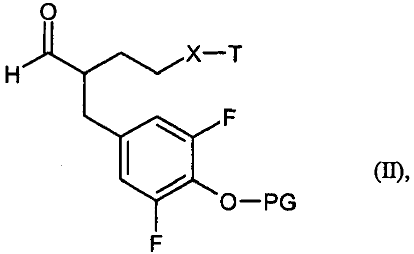 in welcher X die oben angegebene Bedeutung hat und
- PG: für eine Hydroxy-Schutzgruppe, insbesondere für eine Silylgruppe wie beispielsweise Trimethylsilyl, Triisopropylsilyl, tert.-Butyldimethylsilyl oder *tert*.-Butyldiphenylsilyl,
und
- T: für Cyano oder (C₁-C₄)-Alkoxycarbonyl steht,
entweder

[A-1] in einem inerten Lösungsmittel in Gegenwart einer Base mit einer Verbindung der Formel (III-A) 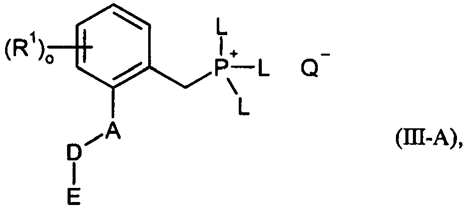 in welcher A, D, E, R¹ und o jeweils die oben angegebenen Bedeutungen haben und
- L: für Phenyl oder o-, m- oder p-Tolyl
und
- Q: für Halogenid oder Tosylat steht,
zu Verbindungen der Formel (IV-A) 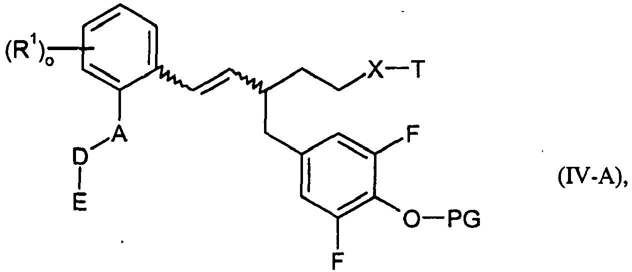 in welcher A, D, E, X, R¹, o, PG und T jeweils die oben angegebenen Bedeutungen haben,
umsetzt
oder

[A-2] in einem inerten Lösungsmittel in Gegenwart einer Base mit einer Verbindung der Formel (III-B) 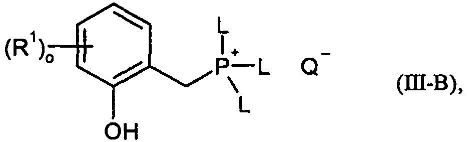 in welcher R¹, o, L und Q jeweils die oben angegebenen Bedeutungen haben,
zunächst zu Verbindungen der Formel (IV-B) 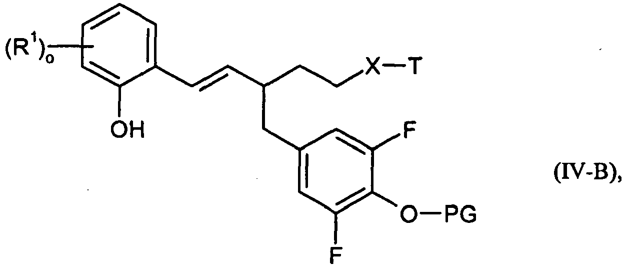 in welcher X, R¹, o, PG und T jeweils die oben angegebenen Bedeutungen haben,
umsetzt und diese anschließend in einem inerten Lösungsmittel in Gegenwart einer Base mit einer Verbindung der Formel (V)

E-D*-Z¹ (V),

in welcher E die oben angegebene Bedeutung hat,
- D*: die oben angegebene Bedeutung von D hat, jedoch nicht für eine Bindung steht,
und
- Z¹: für eine Abgangsgruppe, wie beispielsweise Halogen, Tosylat oder Mesylat, steht,
zu Verbindungen der Formel (IV-C) 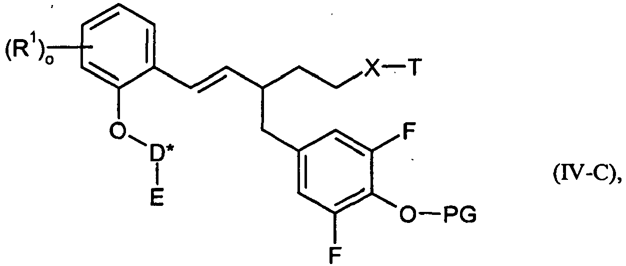 in welcher D*, E, X, R¹, o, PG und T jeweils die oben angegebenen Bedeutungen haben,
alkyliert,
die resultierenden Verbindungen der Formel (IV-A) bzw. (IV-C) dann durch Abspaltung der Schutzgruppe PG in Verbindungen der Formel (VI) 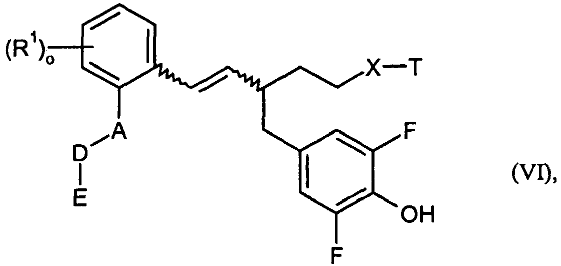 in welcher A, D, E, X, R¹, o und T jeweils die oben angegebenen Bedeutungen haben,
überführt und diese durch Hydrolyse der Ester- bzw. Nitril-Gruppe T zu Carbonsäuren der Formel (I-C) 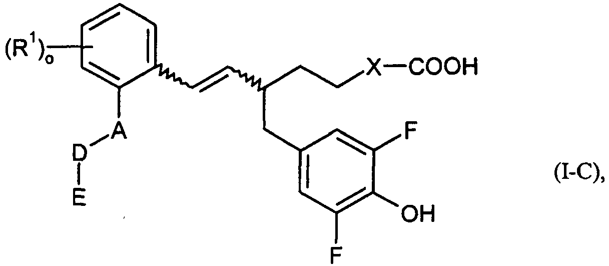 in welcher A, D, E, X, R¹ und o jeweils die oben angegebenen Bedeutungen haben,
umsetzt
und die Verbindungen der Formel (I-C) gegebenenfalls nach dem Fachmann bekannten Methoden in ihre Enantiomere und/oder Diastereomere trennt und/oder gegebenenfalls mit den entsprechenden (i) Lösungsmitteln und/oder (ii) Basen oder Säuren in ihre Solvate, Salze und/oder Solvate der Salze überführt.

Inerte Lösungsmittel für die Verfahrensschritte (II) + (III-A) → (IV-A) und (II) + (III-B) → (IV-B) sind beispielsweise Ether wie Diethylether, Tetrahydrofuran, Glykoldimethylether oder Diethylenglykoldimethylether, oder Kohlenwasserstoffe wie Benzol, Toluol, Xylol, Pentan, Hexan, Heptan, Cyclohexan oder Erdölfraktionen, oder Gemische dieser Lösungsmittel. Bevorzugt wird Tetrahydrofuran im Gemisch mit Hexan verwendet.

Als Basen für diese Verfahrensschritte sind die für eine Wittig-Reaktion üblichen Basen geeignet. Hierzu zählen insbesondere starke Basen wie *n-, sek.-* oder *tert*.-Butyllithium, Lithiumdiisopropylamid (LDA) oder Lithium-, Natrium- oder Kalium-bis(trimethylsilyl)amid. Bevorzugt ist *n*-Butyllithium.

Die Umsetzungen (II) + (III-A) → (IV-A) und (II) + (III-B) → (IV-B) werden im Allgemeinen in einem Temperaturbereich von -78°C bis +20°C, bevorzugt bei -20°C bis +10°C durchgeführt.

Inerte Lösungsmittel für den Verfahrensschritt (IV-B) + (V) → (IV-C) sind beispielsweise Ether wie Diethylether, Dioxan, Tetrahydrofuran, Glykoldimethylether oder Diethylenglykoldimethylether, oder andere Lösungsmittel wie Acetonitril, Dimethylformamid, Dimethylsulfoxid, *N,N'-*Dimethylpropylenharnstoff (DMPU) oder *N*-Methylpyrrolidon (NMP). Ebenso ist es möglich, Gemische der genannten Lösungsmittel einzusetzen. Bevorzugt wird Acetonitril verwendet.

Für diesen Verfahrensschritt geeignete Basen sind insbesondere Kaliumcarbonat, Natrium- oder Kaliumhydrid, Lithiumdiisopropylamid oder *n*-Butyllithium. Bevorzugt wird Kaliumcarbonat verwendet.

Die Reaktion (IV-B) + (V) → (IV-C) wird im Allgemeinen in einem Temperaturbereich von +20°C bis +120°C, bevorzugt bei +50°C bis +100°C durchgeführt.

Als Hydroxy-Schutzgruppe PG wird bevorzugt eine Silylgruppe wie beispielsweise Trimethylsilyl, Triisopropylsilyl, *tert*.-Butyldimethylsilyl oder *tert*.-Butyldiphenylsilyl verwendet. Besonders bevorzugt ist Triisopropylsilyl. Die Abspaltung der Silylgruppe im Verfahrensschritt (IV-A) bzw. (IV-C) → (VI) erfolgt vorzugsweise mit Hilfe von Tetra-n-butylammoniumfluorid (TBAF) oder Fluorwasserstoff. Die Umsetzung wird im Allgemeinen in Tetrahydrofuran als Lösungsmittel in einem Temperaturbereich von 0°C bis +40°C durchgeführt.

Die Hydrolyse der Ester- bzw. Nitril-Gruppe T im Verfahrensschritt (VI) → (I-C) erfolgt nach üblichen Methoden, indem man die Ester bzw. Nitrile in inerten Lösungsmitteln mit Säuren oder Basen behandelt, wobei bei letzterem die zunächst entstehenden Salze durch Behandeln mit Säure in die freien Carbonsäuren überführt werden. Im Falle der *tert*.-Butylester erfolgt die Esterspaltung bevorzugt mit Säuren.

Als inerte Lösungsmittel eignen sich für diese Reaktionen Wasser oder die für eine Esterspaltung üblichen organischen Lösungsmittel. Hierzu gehören bevorzugt Alkohole wie Methanol, Ethanol, n-Propanol, Isopropanol, n-Butanol oder *tert*.-Butanol, oder Ether wie Diethylether, Tetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran, Methanol und/oder Ethanol, bei der Nitril-Hydrolyse bevorzugt Wasser oder n-Propanol eingesetzt. Im Falle der Umsetzung mit Trifluoressigsäure wird bevorzugt Dichlormethan und im Falle der Umsetzung mit Chlorwasserstoff bevorzugt Tetrahydrofuran, Diethylether, Dioxan oder Wasser verwendet.

Als Basen sind die üblichen anorganischen Basen geeignet. Hierzu gehören bevorzugt Alkali- oder Erdalkalihydroxide wie beispielsweise Natrium-, Lithium-, Kalium- oder Bariumhydroxid, oder Alkali- oder Erdalkalicarbonate wie Natrium-, Kalium- oder Calciumcarbonat. Besonders bevorzugt sind Natrium-, Kalium- oder Lithiumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der tert.-Butylester und Salzsäure im Falle der Methylester.

Die Esterspaltung erfolgt im Allgemeinen in einem Temperaturbereich von 0°C bis +100°C, bevorzugt bei +20°C bis +60°C. Die Nitril-Hydrolyse wird im Allgemeinen in einem Temperaturbereich von +50°C bis +150°C, bevorzugt bei +90°C bis +110°C durchgeführt.

Die Verfahrenssequenz (IV-A) bzw. (IV-C) → (VI) → (I-C) kann gegebenenfalls auch als Eintopf-Reaktion durchgeführt werden, bei der die Abspaltung der Schutzgruppe PG und die Hydrolyse der Gruppe T gleichzeitig erfolgen. Hierfür sind insbesondere starke Säuren oder Basen wie Chlorwasserstoff oder Trifluoressigsäure bzw. Natrium- oder Kaliumhydroxid geeignet.

Die genannten Umsetzungen können bei normalem, erhöhtem oder bei erniedrigtem Druck durchgeführt werden (z.B. von 0.5 bis 5 bar). Im Allgemeinen arbeitet man jeweils bei Normaldruck.

Die Aldehyde der Formel (II) können in Analogie zu literaturbekannten Verfahren beispielsweise über eine sequentielle Dialkylierung von Malonsäurediallylester mit Verbindungen der Formeln (VII) und (VIII) 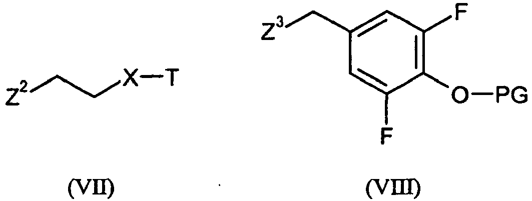 in welchen X, PG und T jeweils die oben angegebenen Bedeutungen haben und
Z² und Z³ gleich oder verschieden sind und für eine Abgangsgruppe, wie beispielsweise Halogen, Mesylat oder Tosylat, stehen,
zu Verbindungen der Formel (IX) 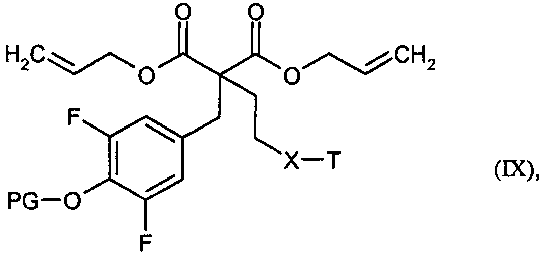 in welcher X, PG und T jeweils die oben angegebenen Bedeutungen haben,
anschließende Esterspaltung zu Verbindungen der Formel (X) 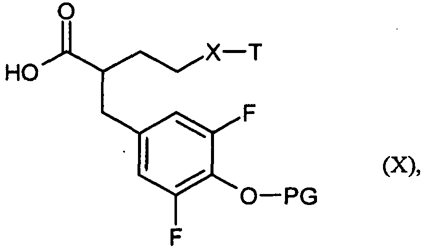 in welcher X, PG und T jeweils die oben angegebenen Bedeutungen haben,
und nachfolgende Reduktion der Carbonsäure-Gruppierung hergestellt werden (siehe auch nachfolgende Reaktionsschemata 3 und 6).

Die Verbindungen der Formeln (III-A) und (III-B) können nach literaturüblichen Verfahren durch Umsetzung von Verbindungen der Formel (XI-A) bzw. (XI-B) 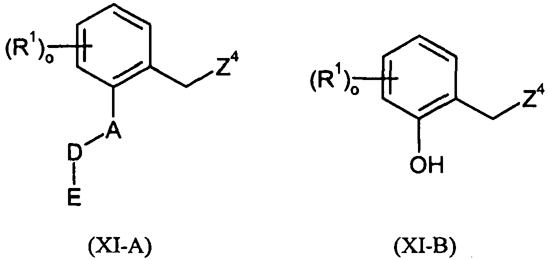 in welchen A, D, E, R¹ und o jeweils die oben angegebenen Bedeutungen haben und
- Z⁴: für eine Abgangsgruppe, wie beispielsweise Halogen oder Tosylat, oder für Hydroxy steht,
mit beispielsweise Triphenylphosphin bzw. (im Falle von Z⁴ = OH) Triphenylphosphin-Hydrobromid erhalten werden (siehe auch nachfolgendes Reaktionsschema 1).

Weiterer Gegenstand der Erfindung ist ein Verfahren zur Herstellung der erfindungsgemäßen Verbindungen der Formel (I), in welcher U, V und W zusammen eine Gruppe der Formel *-CH₂-CH₂-N< bilden, worin * die Verknüpfungsstelle mit dem Phenylring bedeutet, und Y für Carboxyl steht,
dadurch gekennzeichnet, dass man Verbindungen der Formel (XII) 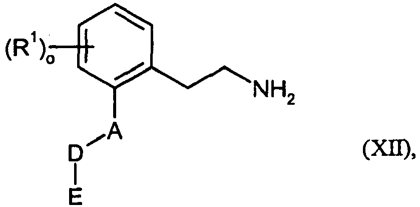 in welcher A, D, E, R¹ und o jeweils die oben angegebenen Bedeutungen haben,
zunächst in einem inerten Lösungsmittel in Gegenwart einer Base mit einer Verbindung der Formel (VII) 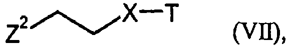 in welcher X die oben angegebene Bedeutung hat und
- T: für Cyano oder (C₁-C₄)-Alkoxycarbonyl
und
- Z²: für eine Abgangsgruppe, wie beispielsweise Halogen, Mesylat oder Tosylat, steht,
zu Verbindungen der Formel (XIII) 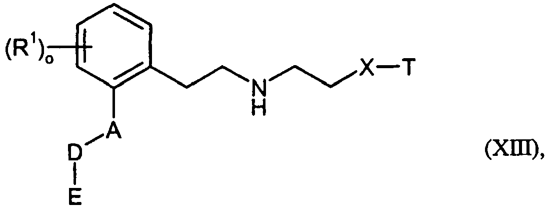 in welcher A, D, E, X, R¹, o und T jeweils die oben angegebenen Bedeutungen haben,
alkyliert, anschließend in einem inerten Lösungsmittel in Gegenwart einer Base mit einer Verbindung der Formel (VIII) 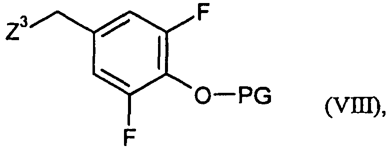 in welcher
- PG: für eine Hydroxy-Schutzgruppe, insbesondere für eine Silylgruppe wie beispielsweise Trimethylsilyl, Triisopropylsilyl, *tert*.-Butyldimethylsilyl oder *tert*.-Butyldiphenylsilyl,
und
- Z³: für eine Abgangsgruppe, wie beispielsweise Halogen, Mesylat oder Tosylat, steht,
zu Verbindungen der Formel (XIV) 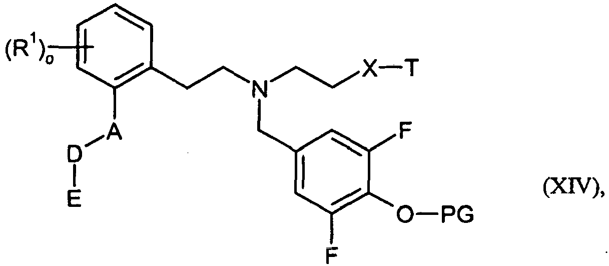 in welcher A, D, E, X, R¹, o, PG und T jeweils die oben angegebenen Bedeutungen haben,
umsetzt, dann durch Abspaltung der Schutzgruppe PG in Verbindungen der Formel (XV) 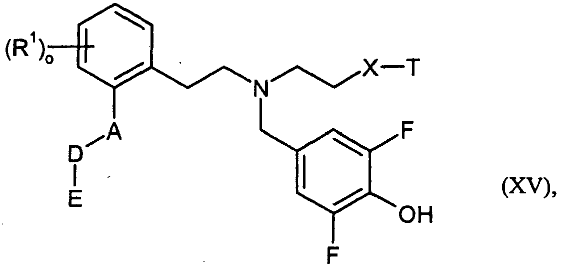 in welcher A, D, E, X, R¹, o und T jeweils die oben angegebenen Bedeutungen haben,
überführt und diese durch Hydrolyse der Ester- bzw. Nitril-Gruppe T zu Carbonsäuren der Formel (I-D) 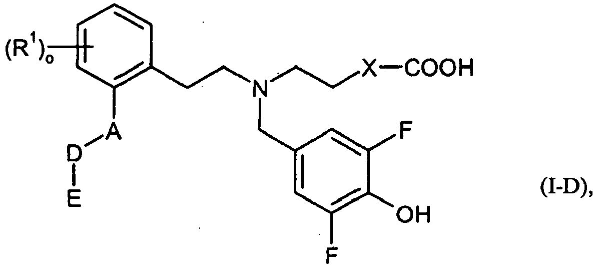 in welcher A, D, E, X, R¹ und o jeweils die oben angegebenen Bedeutungen haben,
umsetzt
und die Verbindungen der Formel (I-D) gegebenenfalls mit den entsprechenden (i) Lösungsmitteln und/oder (ii) Basen oder Säuren in ihre Solvate, Salze und/oder Solvate der Salze überführt.

Bei der zuvor beschriebenen Verfahrenssequenz (XII) → (I-D) können, falls zweckmäßig, einzelne Umsetzungsschritte in ihrer Reihenfolge auch vertauscht werden. So kann beispielsweise das Amin (XII) zunächst mit der Verbindung (VIII) und danach mit der Verbindung (VII) zu den Verbindungen der Formel (XIV) dialkyliert werden. Ebenso können die Hydrolyse der Gruppe T und/oder die Abspaltung der Schutzgruppe PG bereits zu früheren Zeitpunkten innerhalb der Verfahrenssequenz (XII) → (I-D) erfolgen (vgl. auch nachfolgendes Reaktionsschema 10). Solche Variationen sind dem Fachmann geläufig und werden vom erfindungsgemäßen Verfahren umfaßt.

Inerte Lösungsmittel für die Verfahrensschritte (XII) + (VII) → (XIII) und (XIII) + (VIII) → (XIV) sind beispielsweise Ether wie Diethylether, tert.-Butylmethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Acetonitril, Pyridin, Dimethylsulfoxid, Dimethylformamid, *N,N'*-Dimethylpropylenharnstoff (DMPU) oder *N*-Methylpyrrolidon (NMP). Ebenso ist es möglich, Gemische der genannten Lösungsmittel zu verwenden. Bevorzugt sind Dimethylformamid und Aceton.

Als Basen sind für diese Verfahrensschritte die üblichen anorganischen oder organischen Basen geeignet. Hierzu gehören bevorzugt Alkali- oder Erdalkalicarbonate wie Lithium-, Natrium-, Kalium-, Calcium- oder Cäsiumcarbonat, Alkalihydride wie Natriumhydrid, Amide wie Lithium-diisopropylamid oder Lithium- oder Kalium-bis(trimethylsilyl)amid, oder organische Amine wie Triethylamin, *N*-Methylmorpholin, *N*-Methylpiperidin, *N,N*-Diisopropylethylamin oder Pyridin. Besonders bevorzugt sind Natrium- oder Kaliumcarbonat und Triethylamin.

Die Umsetzungen (XII) + (VII) → (XIII) und (XIII) + (VIII) → (XIV) können gegebenenfalls vorteilhaft in Gegenwart eines Alkylierungskatalysators wie Lithium-, Natrium- oder Kaliumiodid durchgeführt werden.

Die Verfahrensschritte (XII) + (VII) → (XIII) und (XIII) + (VIII) → (XIV) erfolgen im Allgemeinen in einem Temperaturbereich von +20°C bis +100°C, bevorzugt bei +50°C bis +80°C.

Als Hydroxy-Schutzgruppe PG wird bevorzugt eine Silylgruppe wie beispielsweise Trimethylsilyl, Triisopropylsilyl, tert.-Butyldimethylsilyl oder *tert*.-Butyldiphenylsilyl verwendet. Besonders bevorzugt ist Triisopropylsilyl. Die Abspaltung der Silylgruppe im Verfahrensschritt (XIV) → (XV) erfolgt vorzugsweise mit Hilfe von Tetra-n-butylammoniumfluorid (TBAF) oder Fluorwasserstoff. Die Umsetzung wird im Allgemeinen in Tetrahydrofuran als Lösungsmittel in einem Temperaturbereich von 0°C bis +40°C durchgeführt.

Die Hydrolyse der Ester- bzw. Nitril-Gruppe T im Verfahrensschritt (XV) → (I-D) erfolgt nach üblichen Methoden, indem man die Ester bzw. Nitrile in inerten Lösungsmitteln mit Säuren oder Basen behandelt, wobei bei letzterem die zunächst entstehenden Salze durch Behandeln mit Säure in die freien Carbonsäuren überführt werden. Im Falle der *tert*.-Butylester erfolgt die Esterspaltung bevorzugt mit Säuren.

Als inerte Lösungsmittel eignen sich für diese Reaktionen Wasser oder die für eine Esterspaltung üblichen organischen Lösungsmittel. Hierzu gehören bevorzugt Alkohole wie Methanol, Ethanol, n-Propanol, Isopropanol, n-Butanol oder tert.-Butanol, oder Ether wie Diethylether, Tetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran, Methanol und/oder Ethanol, bei der Nitril-Hydrolyse bevorzugt Wasser oder n-Propanol eingesetzt. Im Falle der Umsetzung mit Trifluoressigsäure wird bevorzugt Dichlormethan und im Falle der Umsetzung mit Chlorwasserstoff bevorzugt Tetrahydrofuran, Diethylether, Dioxan oder Wasser verwendet.

Als Basen sind die üblichen anorganischen Basen geeignet. Hierzu gehören bevorzugt Alkali- oder Erdalkalihydroxide wie beispielsweise Natrium-, Lithium-, Kalium- oder Bariumhydroxid, oder Alkali- oder Erdalkalicarbonate wie Natrium-, Kalium- oder Calciumcarbonat. Besonders bevorzugt sind Natrium-, Kalium- oder Lithiumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der tert.-Butylester und Salzsäure im Falle der Methyl- oder Ethylester.

Die Esterspaltung erfolgt im Allgemeinen in einem Temperaturbereich von 0°C bis +100°C, bevorzugt bei +20°C bis +60°C. Die Nitril-Hydrolyse wird im Allgemeinen in einem Temperaturbereich von +50°C bis +150°C, bevorzugt bei +90°C bis +110°C durchgeführt.

Die Verfahrenssequenz (XIV) → (XV) → (I-D) kann gegebenenfalls auch als Eintopf-Reaktion durchgeführt werden, bei der die Abspaltung der Schutzgruppe PG und die Hydrolyse der Gruppe T gleichzeitig erfolgen. Hierfür sind insbesondere starke Säuren oder Basen wie Chlorwasserstoff oder Trifluoressigsäure bzw. Natrium- oder Kaliumhydroxid geeignet.

Die genannten Umsetzungen können bei normalem, erhöhtem oder bei erniedrigtern Druck durchgeführt werden (z.B. von 0.5 bis 5 bar). Im Allgemeinen arbeitet man jeweils bei Normaldruck.

Die erfindungsgemäßen Verbindungen der Formel (I), in welcher Y für eine Gruppe der Formel 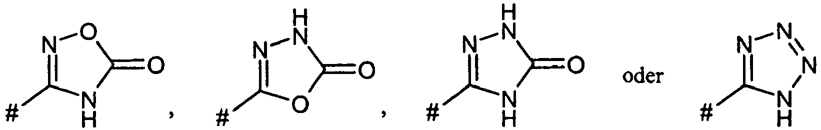 steht, worin # die jeweilige Verknüpfungsstelle bedeutet, können hergestellt werden, indem man
[B] Verbindungen der Formel (XVI) 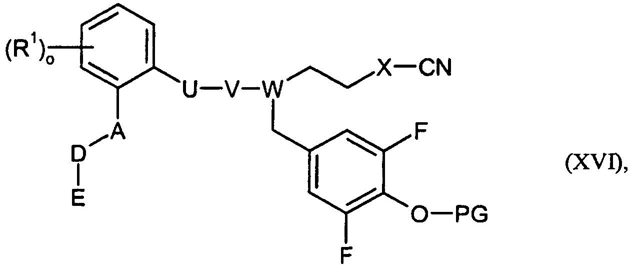 in welcher A, D, E, U, V, W, X, R¹, o und PG jeweils die oben angegebenen Bedeutungen haben,
zunächst in einem inerten Lösungsmittel mit Hydroxylamin in Verbindungen der Formel (XVII) 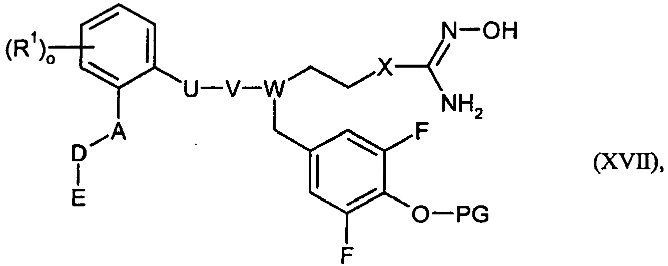 in welcher A, D, E, U, V, W, X, R¹, o und PG jeweils die oben angegebenen Bedeutungen haben,
überführt, anschließend in einem inerten Lösungsmittel in Gegenwart einer Base mit einem Chlorameisensäureester der Formel (XVIII) 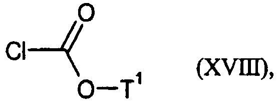 in welcher
- T¹: für (C₁-C₁₀)-Alkyl steht,
sowie gegebenenfalls durch nachfolgendes Erhitzen in einem inerten Lösungsmittel zu Verbindungen der Formel (XIX) 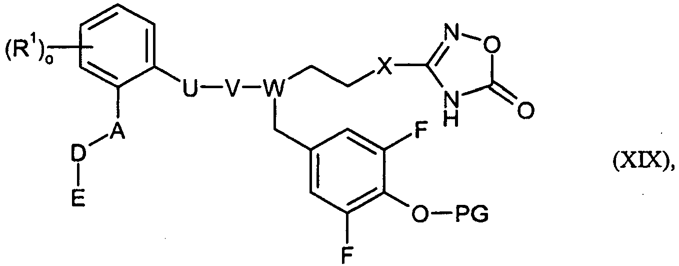 in welcher A, D, E, U, V, W, X, R¹, o und PG jeweils die oben angegebenen Bedeutungen haben,
umsetzt und dann durch Abspaltung der Schutzgruppe PG in Verbindungen der Formel (I-E) 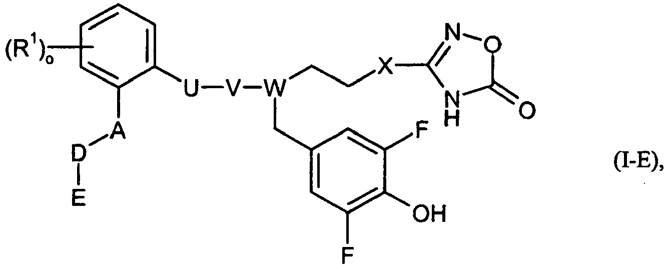 in welcher A, D, E, U, V, W, X, R¹ und o jeweils die oben angegebenen Bedeutungen haben,
überführt,
[C] Verbindungen der Formel (XX) 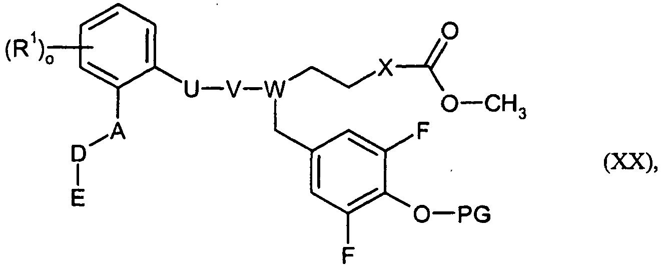 in welcher A, D, E, U, V, W, X, R¹, o und PG jeweils die oben angegebenen Bedeutungen haben,
zunächst in einem inerten Lösungsmittel mit Hydrazin in Verbindungen der Formel (XXI) 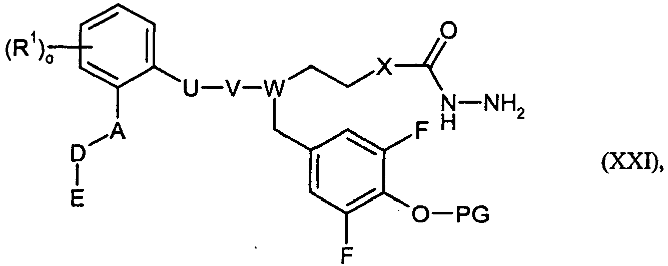 in welcher A, D, E, U, V, W, X, R¹, o und PG jeweils die oben angegebenen Bedeutungen haben,
überführt, anschließend in einem inerten Lösungsmittel mit Phosgen oder einem Phosgen-Derivat wie beispielsweise Di- oder Triphosgen zu Verbindungen der Formel (XXII) 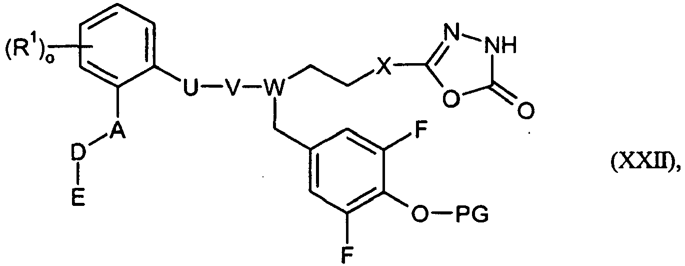 in welcher A, D, E, U, V, W, X, R¹, o und PG jeweils die oben angegebenen Bedeutungen haben,
umsetzt und dann durch Abspaltung der Schutzgruppe PG in Verbindungen der Formel (I-F) 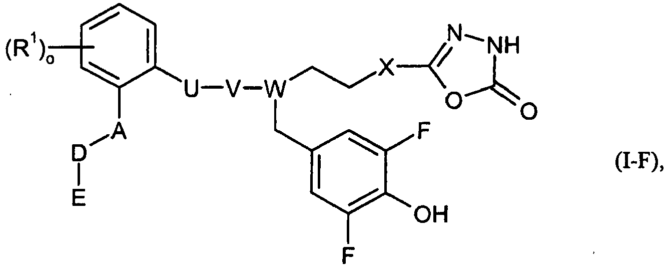 in welcher A, D, E, U, V, W, X, R¹ und o jeweils die oben angegebenen Bedeutungen haben,
überführt,
[D] Verbindungen der Formel (XXIII) 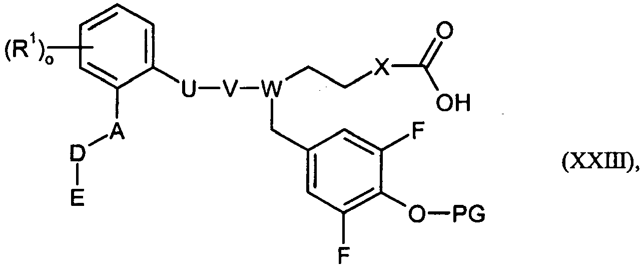 in welcher A, D, E, U, V, W, X, R¹, o und PG jeweils die oben angegebenen Bedeutungen haben,
zunächst in einem inerten Lösungsmittel mit Oxalylchlorid, Thionylchlorid oder Phosphorylchlorid in die entsprechenden Carbonsäurechloride der Formel (XXIV) 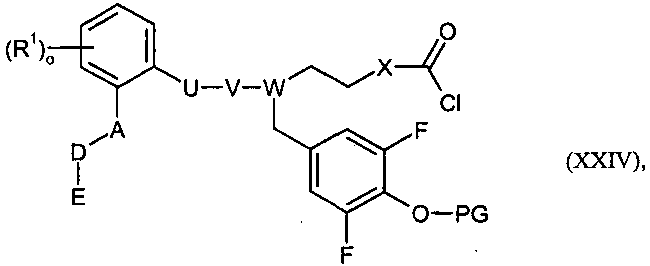 in welcher A, D, E, U, V, W, X, R¹, o und PG jeweils die oben angegebenen Bedeutungen haben,
überführt, diese dann in einem inerten Lösungsmittel mit Semicarbazid zu Verbindungen der Formel (XXV) 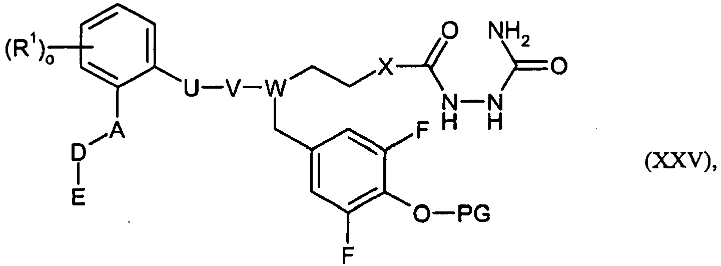 in welcher A, D, E, U, V, W, X, R¹, o und PG jeweils die oben angegebenen Bedeutungen haben,
umsetzt, anschließend in Gegenwart einer Base zu Verbindungen der Formel (XXVI) 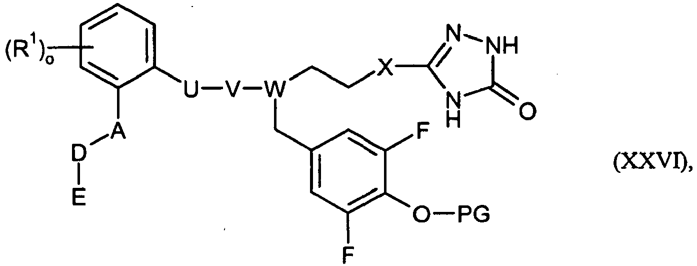 in welcher A, D, E, U, V, W, X, R¹, o und PG jeweils die oben angegebenen Bedeutungen haben,
cyclisiert und nachfolgend durch Abspaltung der Schutzgruppe PG in Verbindungen der Formel (I-G) 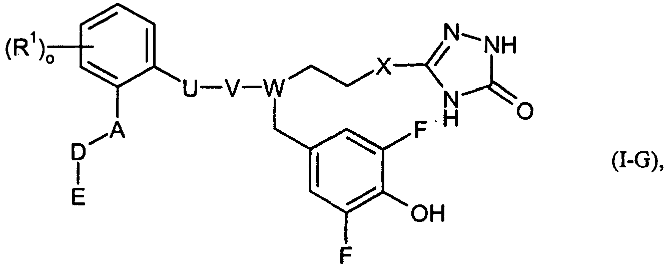 in welcher A, D, E, U, V, W, X, R¹ und o jeweils die oben angegebenen Bedeutungen haben,
überführt,
beziehungsweise
[E] Verbindungen der Formel (XVI) 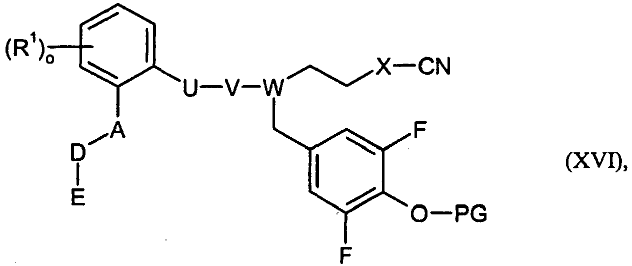 in welcher A, D, E, U, V, W, X, R¹, o und PG jeweils die oben angegebenen Bedeutungen haben,
in einem inerten Lösungsmittel mit einem Alkali-Azid in Gegenwart von Ammoniumchlorid oder mit Trimethylsilylazid gegebenenfalls in Gegenwart eines Katalysators zu Verbindungen der Formel (XXVII) 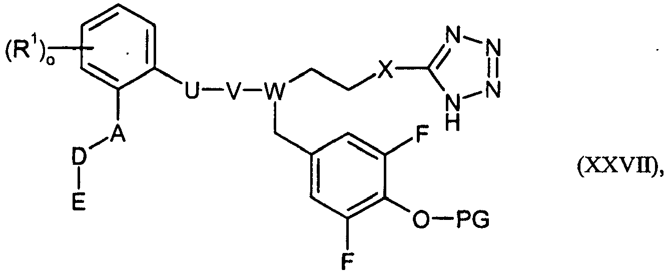 in welcher A, D, E, U, V, W, X, R¹, o und PG jeweils die oben angegebenen Bedeutungen haben,
umsetzt und dann durch Abspaltung der Schutzgruppe PG in Verbindungen der Formel (I-H) 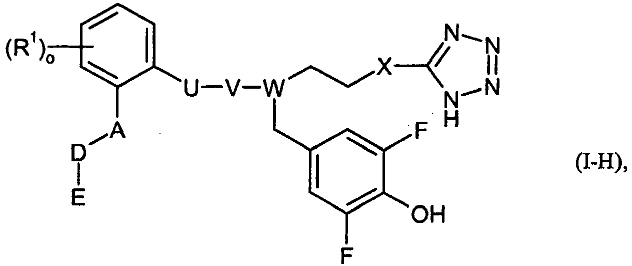 in welcher A, D, E, U, V, W, X, R¹ und o jeweils die oben angegebenen Bedeutungen haben,
überführt
und die jeweils resultierenden Verbindungen der Formeln (I-E), (I-F), (I-G) bzw. (I-H) gegebenenfalls nach dem Fachmann bekannten Methoden in ihre Enantiomere und/oder Diastereomere trennt und/oder gegebenenfalls mit den entsprechenden (i) Lösungsmitteln und/oder (ii) Basen oder Säuren zu ihren Solvaten, Salzen und/oder Solvaten der Salze umsetzt.

Die bei den Verfahrenssequenzen [B], [C], [D], [E] verwendeten Ausgangsverbindungen (XVI) und (XX) entsprechen den zuvor beschriebenen Verbindungen der Formeln (IV-A), (IV-B), (IV-C) bzw. (XIV).

Die erfindungsgemäßen Verbindungen der Formel (I), in welcher U, V und W zusammen eine Gruppe der Formel *-CH₂-CH₂-CH< bilden, worin * die Verknüpfungsstelle mit dem Phenylring bedeutet, können durch Hydrierung der olefinischen Doppelbindung auf der Stufe der Verbindungen (IV-A), (IV-B), (IV-C), (VI) oder (I-C) und weitere Umsetzung analog zu den zuvor beschriebenen Verfahren hergestellt werden.

Die Verbindungen der Formeln (V), (VII), (VIII), (XI-A), (XI-B), (XII) und (XVIII) sind kommerziell erhältlich, literaturbekannt oder können nach literaturbekannten Verfahren hergestellt werden (siehe auch nachfolgende Reaktionsschemata 1, 2 und 10).

Die Herstellung der erfindungsgemäßen Verbindungen kann durch die folgenden Syntheseschemata veranschaulicht werden: 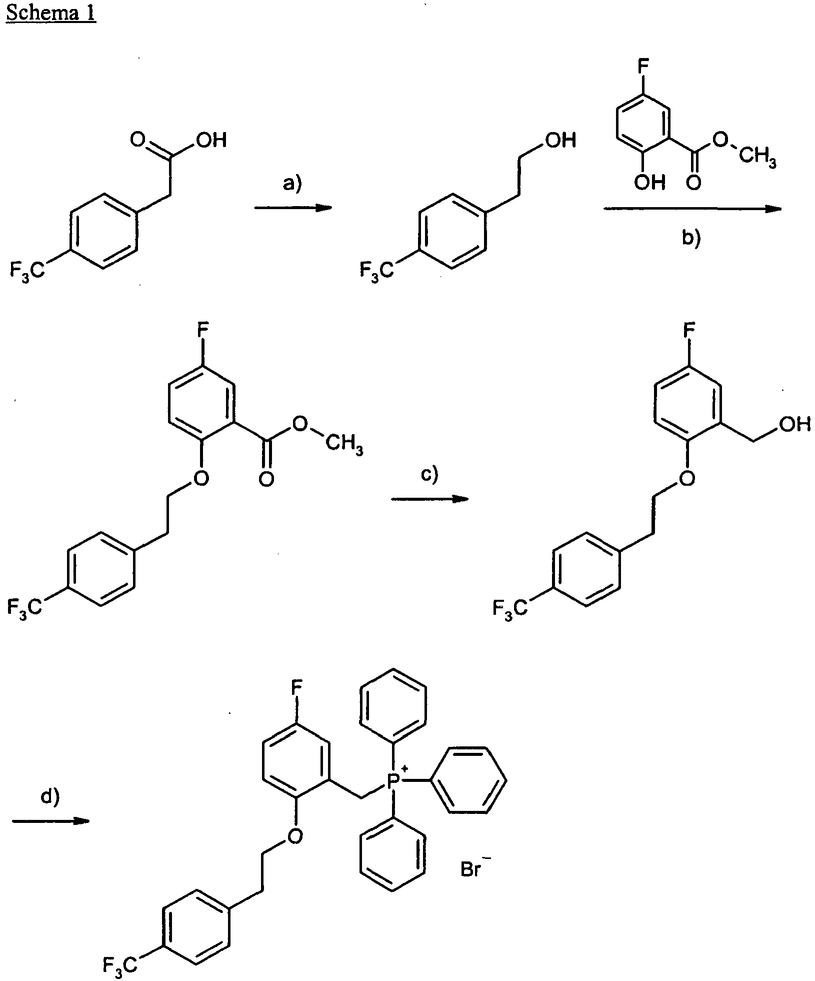 [a) Lithiumaluminiumhydrid; b) Diethylazodicarboxylat; c) Lithiumaluminiumhydrid; d) Triphenylphosphin-Hydrobromid]. 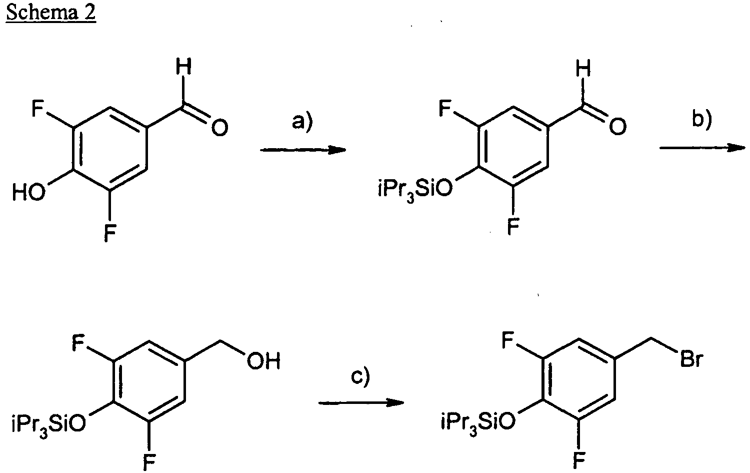 [a) Trifluormethansulfonsäure-triisopropylsilylester; b) Natriumborhydrid; c) Tetrabrommethan, Triphenylphosphin]. 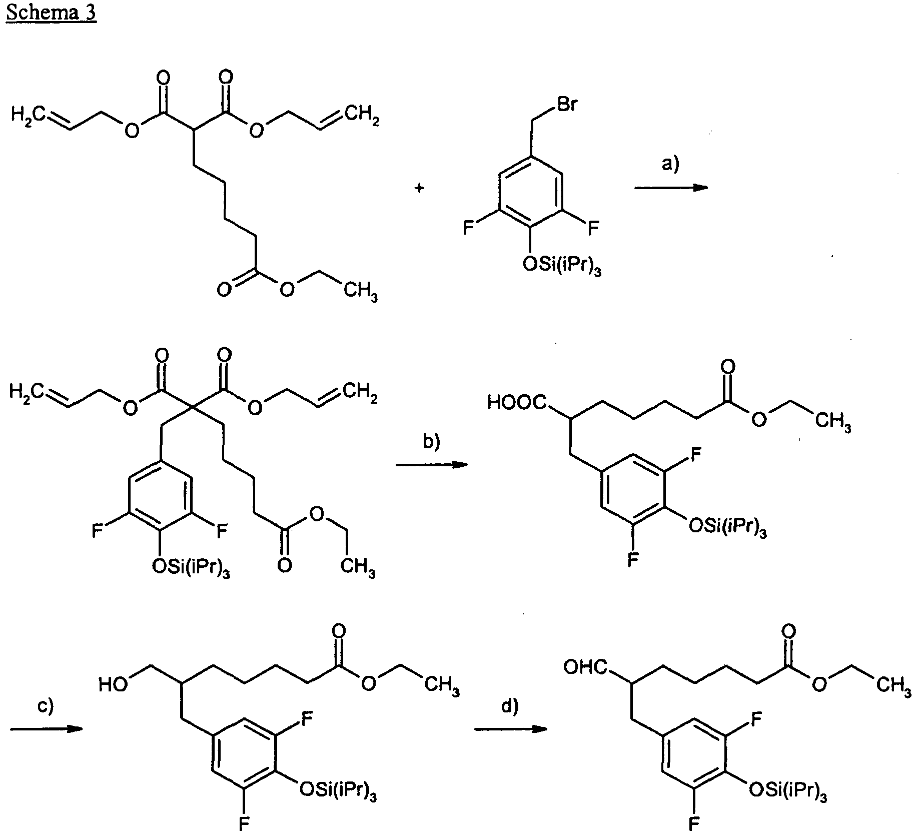 [a) Natriumhydrid; b) Palladium(II)acetat, Triphenylphosphin, Triethylamin, Ameisensäure; c) Boran-THF-Komplex; d) *N*-Methylmorpholin-*N*-oxid, Tetrapropylammoniumperruthenat]. 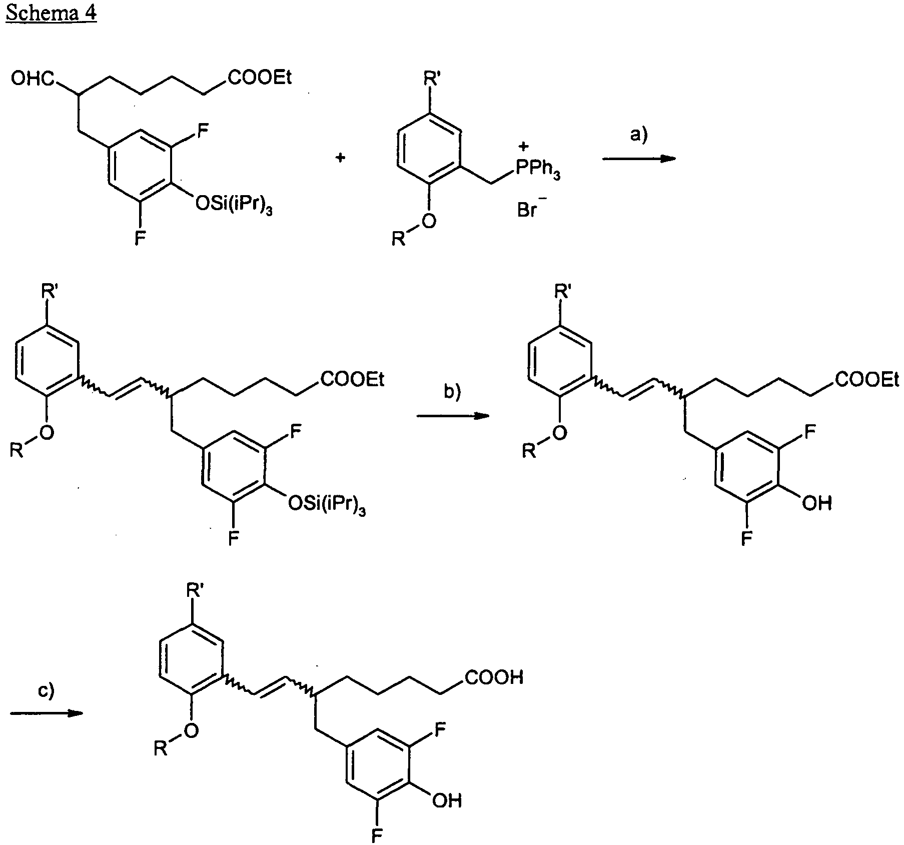 [a) n-Butyllithium; b) Tetra-n-butylammoniumfluorid; c) Natronlauge]. 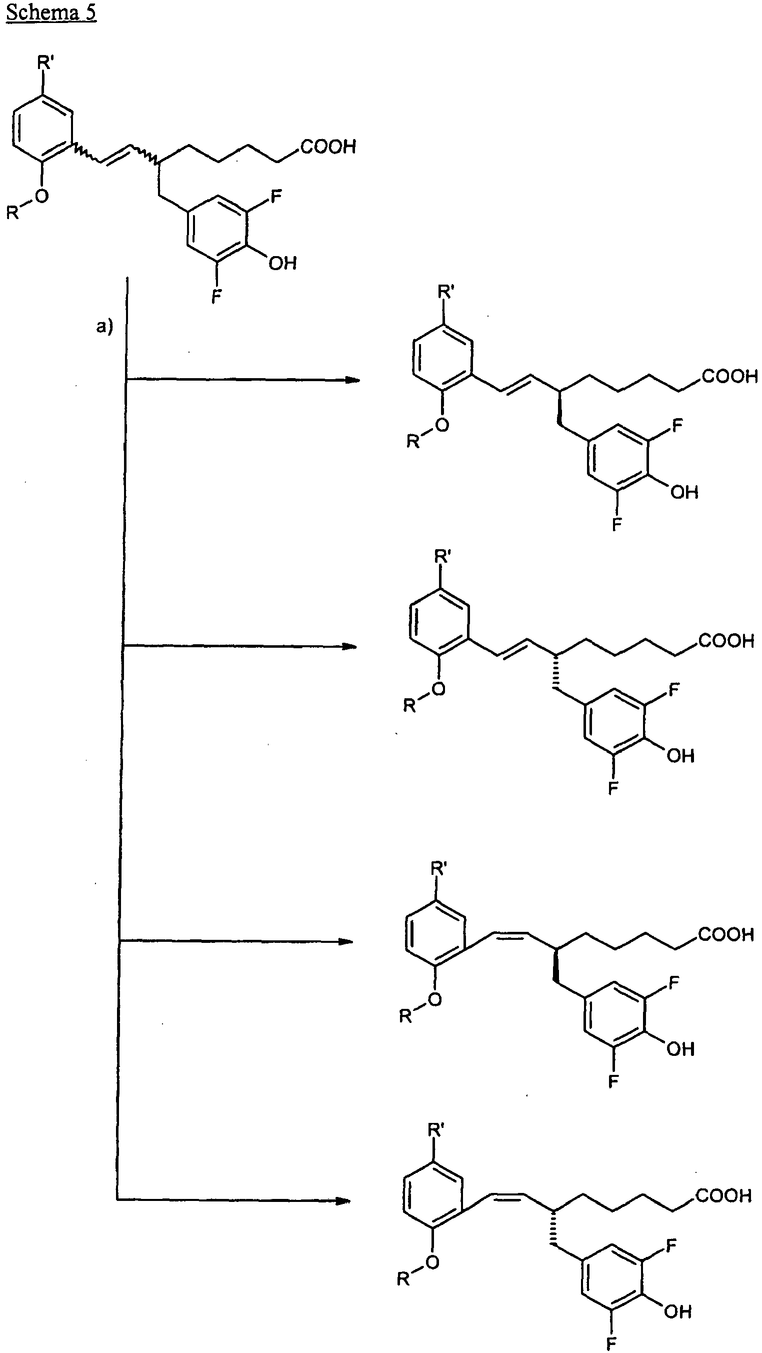 [a) präparative HPLC an chiraler Phase]. 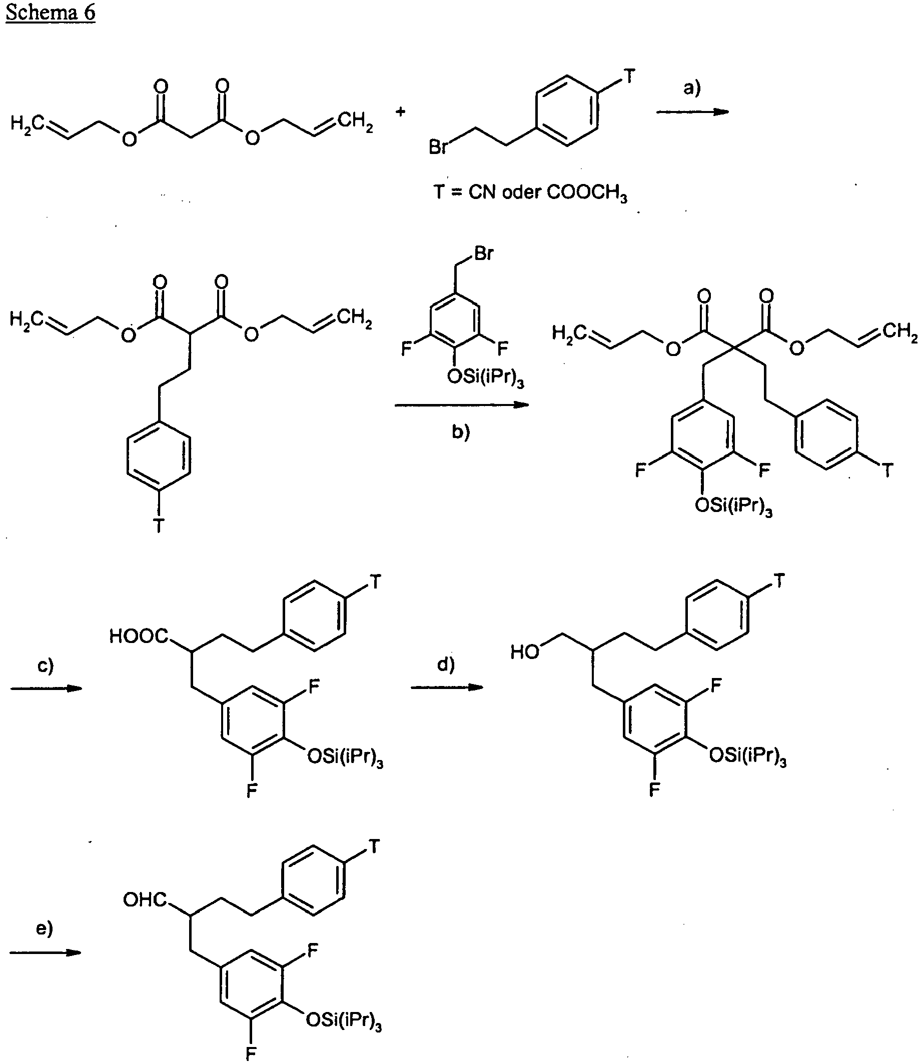 [a) Natriumhydrid; b) Natriumhydrid; c) Palladium(II)acetat, Triphenylphosphin, Triethylamin, Ameisensäure; d) Boran-THF-Komplex; e) Pyridiniumchlorochromat]. 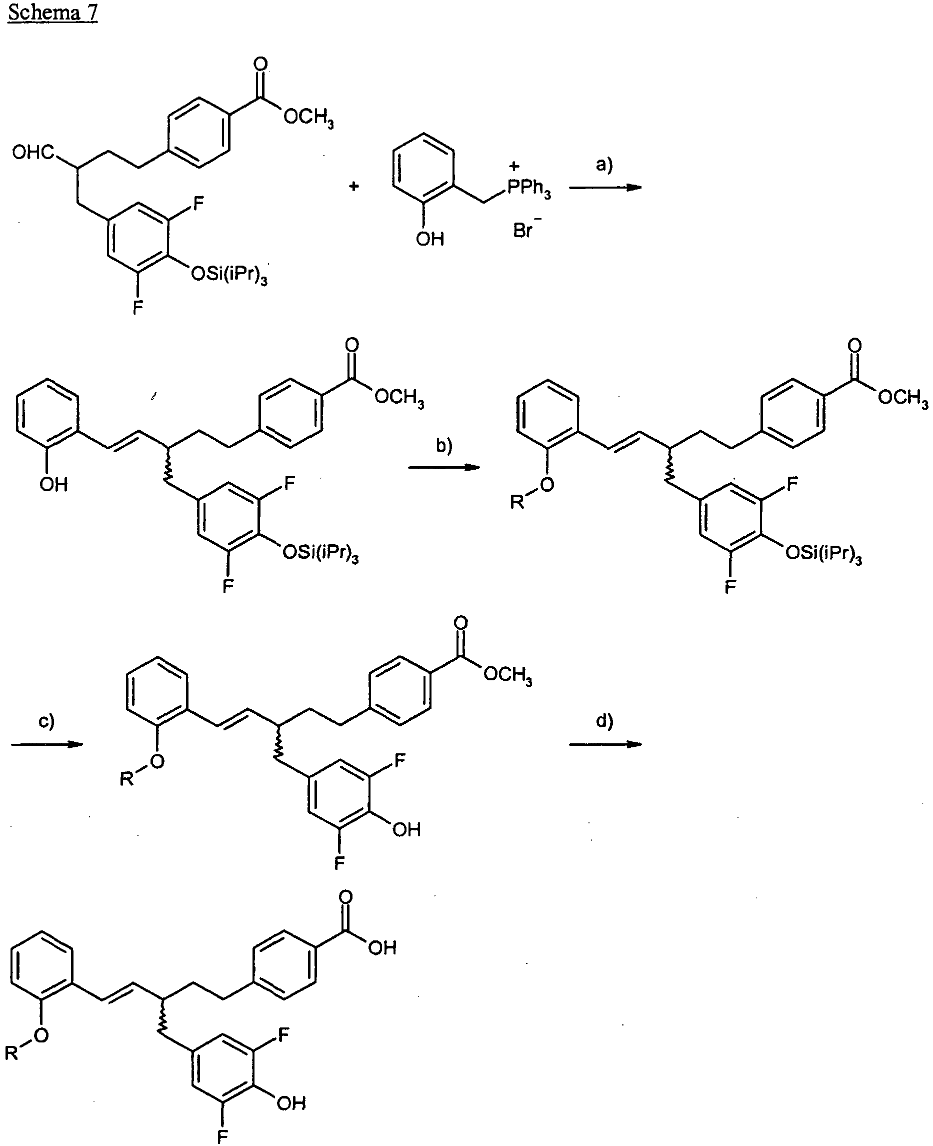 [a) n-Butyllithium; b) Alkylhalogenid, Kaliumcarbonat; c) Tetra-n-butylammoniumfluorid; d) Natronlauge]. 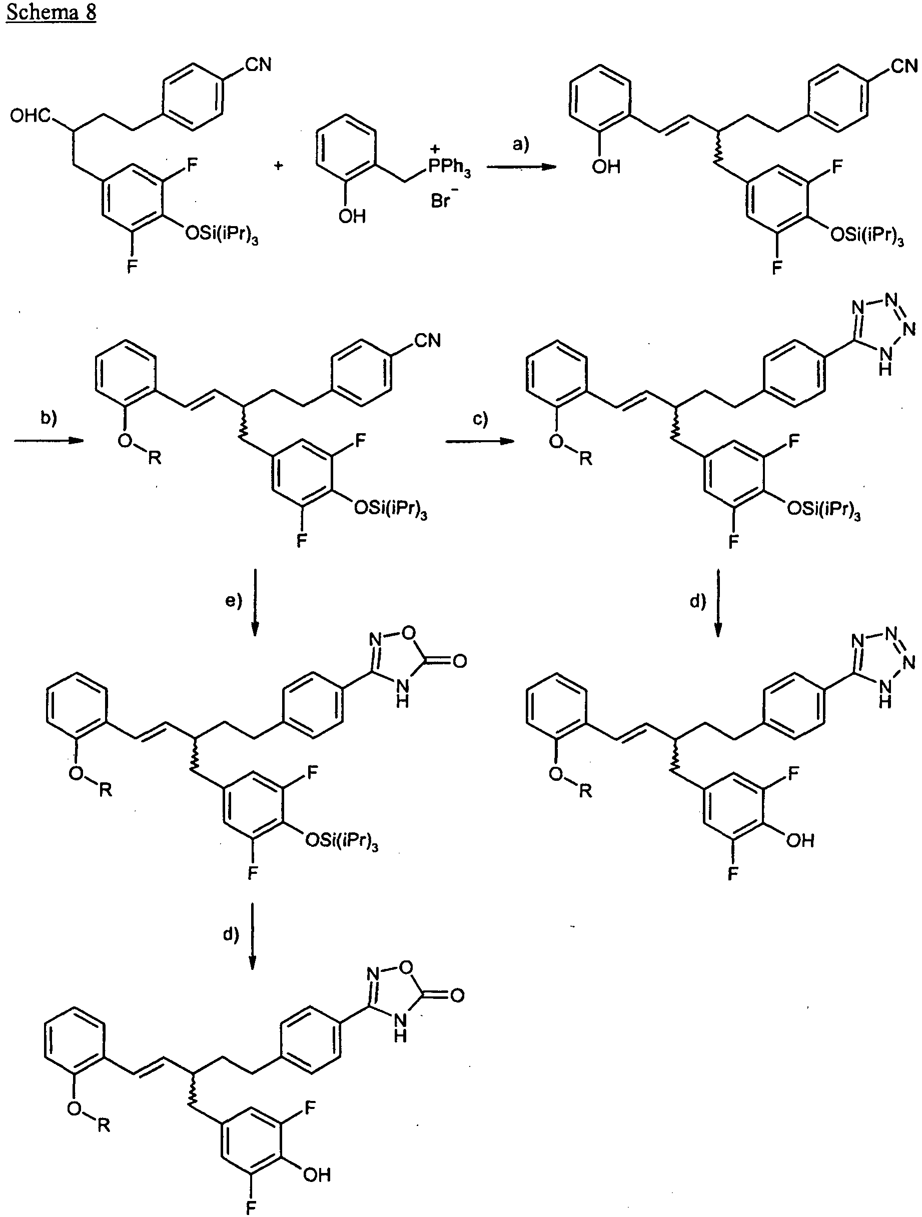 [a) n-Butyllithium; b) Alkylhalogenid, Kaliumcarbonat; c) Trimethylsilylazid, Di-n-butylzinnoxid; d) Tetra-n-butylammoniumfluorid; e) 1. Hydroxylamin-Hydrochlorid, 2. Chlorameisensäure-2-ethylhexylester].

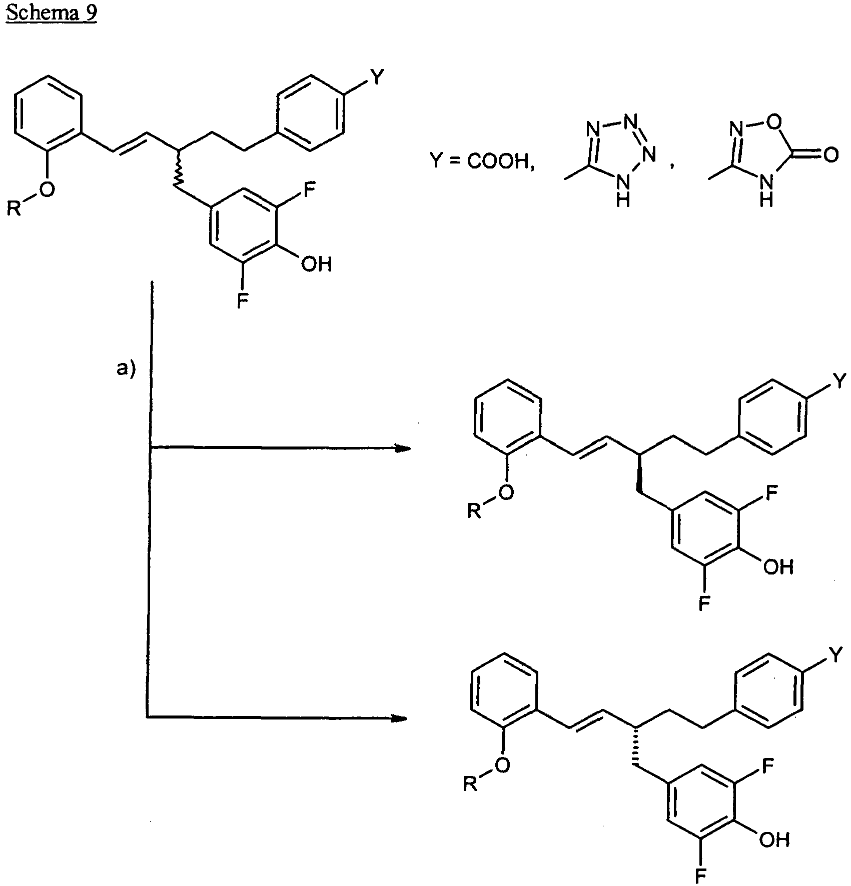

### [a) präparative HPLC an chiraler Phase].

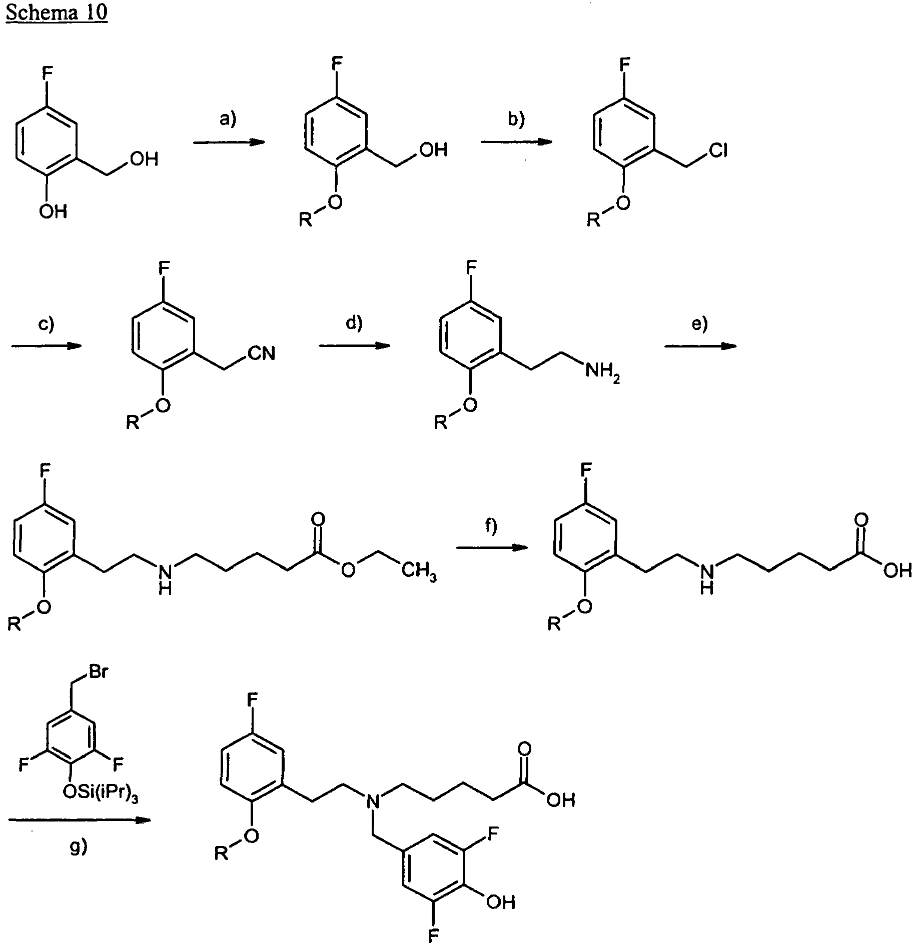

[a) Alkylhalogenid, Kaliumcarbonat; b) Thionylchlorid; c) Natriumcyanid; d) Aluminiumtrichlorid, Lithiumaluminiumhydrid; e) Bromvaleriansäureethylester, Triethylamin; f) Salzsäure; g) Kaliumcarbonat].

[Abkürzungen: Et = Ethyl; iPr = Isopropyl; Ph = Phenyl; THF = Tetrahydrofuran].

Die erfindungsgemäßen Verbindungen besitzen wertvolle pharmakologische Eigenschaften und können zur Vorbeugung und Behandlung von Erkrankungen bei Menschen und Tieren verwendet werden.

Als besonderes und überraschendes Merkmal weisen die Verbindungen der vorliegenden Erfindung vorteilhafte pharmakokinetische Eigenschaften wie beispielsweise eine erhöhte Bioverfügbarkeit und/oder eine verlängerte Wirkdauer nach oraler Gabe auf.

Die erfindungsgemäßen Verbindungen führen zu einer Gefäßrelaxation, zu einer Thrombozytenaggregationshemmung und zu einer Blutdrucksenkung sowie zu einer Steigerung des koronaren Blutflusses. Diese Wirkungen sind über eine direkte Aktivierung der löslichen Guanylatcyclase und einen intrazellulären cGMP-Anstieg vermittelt.

Die erfindungsgemäßen Verbindungen können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, pulmonaler Hypertonie, peripheren und kardialen Gefäßerkrankungen, Arrhythmien, zur Behandlung von thromboembolischen Erkrankungen und Ischämien wie Myokardinfarkt, Hirnschlag, transistorischen und ischämischen Attacken, peripheren Durchblutungsstörungen, zur Verhinderung von Restenosen wie nach Thrombolysetherapien, percutan-transluminalen Angioplastien (PTA), percutan-transluminalen Koronarangioplastien (PTCA) und Bypass sowie zur Behandlung von Arteriosklerose, asthmatischen Erkrankungen, Krankheiten des Urogenitalsystems wie beispielsweise Prostatahypertrophie, erektile Dysfunktion, weibliche sexuelle Dysfunktion und Inkontinenz, von Osteoporose, Glaukom und Gastroparese eingesetzt werden.

Außerdem können die erfindungsgemäßen Verbindungen zur Behandlung von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, CREST-Syndrom, Erythematose, Onychomykose sowie von rheumatischen Erkrankungen verwendet werden.

Ferner eignen sich die erfindungsgemäßen Verbindungen zur Behandlung von Respiratory Distress-Syndromen und chronisch-obstruktiven Atemwegserkrankungen (COPD), von akuter und chronischer Niereninsuffizienz sowie zur Förderung der Wundheilung.

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen eingesetzt werden.

Zudem besitzen die erfindungsgemäßen Verbindungen antiinflammatorische Wirkung und können daher als entzündungshemmende Mittel eingesetzt werden.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- NO-unabhängige, jedoch Häm-abhängige Stimulatoren der Guanylatcyclase, wie insbesondere die in WO 00/06568, WO 00/06569, WO 02/42301 und WO 03/095451 beschriebenen Verbindungen;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise BAY 59-7939, DU-176b, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptions-hemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

**Abkürzungen:**
- abs.: absolut
- aq.: wässrig
- Bsp.: Beispiel
- CI: chemische Ionisation (bei MS)
- DC: Dünnschichtchromatographie
- DCI: direkte chemische Ionisation (bei MS)
- DEAD: Diethylazodicarboxylat
- DMF: Dimethylformamid
- DMSO: Dimethylsulfoxid
- d. Th.: der Theorie (bei Ausbeute)
- ee: Enantiomerenüberschuss
- EI: Elektronenstoß-Ionisation (bei MS)
- eq.: Äquivalent(e)
- ESI: Elektrospray-Ionisation (bei MS)
- GC: Gaschromatographie
- h: Stunde(n)
- HPLC: Hochdruck-, Hochleistungsflüssigchromatographie
- LC/MS: Flüssigchromatographie-gekoppelte Massenspektroskopie
- min: Minute(n)
- MS: Massenspektroskopie
- NMR: Kernresonanzspektroskopie
- R_{f}: Retentionsindex (bei DC)
- RT: Raumtemperatur
- Rₜ: Retentionszeit (bei HPLC)
- THF: Tetrahydrofuran
- UV: Ultraviolett-Spektroskopie
- v/v: Volumen zu Volumen-Verhältnis (einer Lösung)

### LC/MS-Methoden:

### Methode 1 (LC-MS)

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 2 (LC-MS)

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 3 (LC-MS)

Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 4 (LC-MS)

Gerätetyp MS: Micromass ZQ; Gerätetyp HPLC: Waters Alliance 2795; Säule: Merck Chromolith SpeedROD RP-18e 50 mm x 4.6 mm; Eluent A: Wasser + 500 µl 50%-ige Ameisensäure / 1; Eluent B: Acetonitril + 500 µl 50%-ige Ameisensäure / 1; Gradient: 0.0 min 10% B → 3.0 min 95% B → 4.0 min 95% B; Ofen: 35°C; Fluss: 0.0 min 1.0 ml/min → 3.0 min 3.0 ml/min → 4.0 min 3.0 ml/min; UV-Detektion: 210 nm.

### Methode 5 (LC-MS)

Gerätetyp MS: Micromass ZQ; Gerätetyp HPLC: Waters Alliance 2790; Säule: Symmetry C 18, 50 mm x 2.1 mm, 3.5 µm; Eluent B: Acetonitril + 0.05% Ameisensäure, Eluent A: Wasser + 0.05% Ameisensäure; Gradient: 0.0 min 10% B → 3.5 min 90% B → 5.5 min 90% B; Ofen: 50°C; Fluss: 0.8 ml/min; UV-Detektion: 210 nm.

### GC/MS-Methoden:

### Methode 1 (GC-MS)

Instrument: Micromass GCT, GC6890; Säule: Restek RTX-35MS, 30 m x 250 µm x 0.25 µm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (1.7 min halten).

### Methode 2 (GC-MS)

Instrument: Micromass GCT, GC6890; Säule: Restek RTX-35MS, 30 m x 250 µm x 0.25 µm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (8.7 min halten).

### HPLC-Methoden:

### Methode 1 (HPLC)

Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 9 min 90% B → 9.2 min 2% B → 10 min 2% B; Fluss: 0.75 ml/min; Säulentemperatur: 30°C; UV-Detektion: 210 nm.

### Methode 2 (HPLC)

Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

### Methode 3 (HPLC)

Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 15 min 90% B → 15.2 min 2% B → 16 min 2% B; Fluss: 0.75 ml/min; Säulentemperatur: 30°C; UV-Detektion: 210 nm.

### Methode 4 (HPLC)

Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 30.0 min 90% B → 30.2 min 2% B → 32 min 2% B; Fluss: 0.75 ml/min; Säulentemperatur: 30°C; UV-Detektion: 210 nm.

### Ausgangsverbindungen und Intermediate:

### Beispiel 1A

### 2-(4-Trifluormethyl-phenyl)ethanol

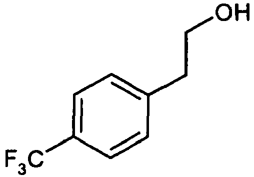

3.0 g (14.7 mmol) 4-Trifluormethylphenylessigsäure werden bei 0°C in 30 ml abs. THF vorgelegt, dann eine 1 M Lösung von 557 mg (14.7 mmol) Lithiumaluminiumhydrid in 14.7 ml THF zugetropft und die Mischung bei Raumtemperatur bis zur vollständigen Umsetzung gerührt. Der Ansatz wird auf Eis gegeben, mit Salzsäure angesäuert und mit Essigsäureethylester extrahiert. Die organische Phase wird eingeengt und das Rohprodukt an Kieselgel chromatographisch gereinigt. Es werden 2 g (92% d. Th.) der Titelverbindung erhalten.

¹H-NMR (400 MHz, CDCl₃, δ/ppm): 2.95 (t, 2H), 3.9 (t, 2H), 7.35 (d, 2H), 7.6 (t, 2H).

GC-MS (Methode 1): Rₜ 4.61 min; m/z 190 (M⁺).

### Beispiel 2A

### 5-Fluor-2-[2-(4-trifluormethyl-phenyl)ethoxy]benzoesäuremethylester

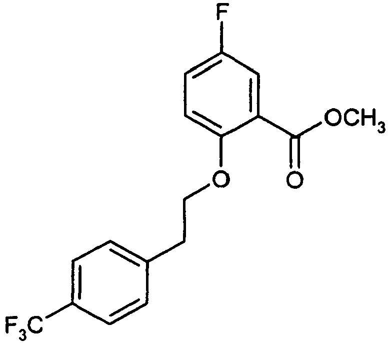

1.79 g (10.5 mmol) 5-Fluorsalicylsäuremethylester, 20 g (10.5 mmol) 2-(4-Trifluormethylphenyl)-ethanol und 2.76 g (10.5 mmol) Triphenylphosphin werden in 50 ml THF vorgelegt und dann bei 0°C eine Lösung von 1.83 g (10.5 mmol) DEAD in 10 ml THF zugetropft. Es wird über Nacht bei RT nachgerührt und die flüchtigen Komponenten anschließend im Vakuum entfernt. Das erhaltene Rohprodukt wird durch Chromatographie an Kieselgel (Laufmittel: Cyclohexan/Essigsäureethyl-ester 7:1) gereinigt. Es werden 2.09 g (58% d. Th.) der Titelverbindung erhalten.

¹H-NMR (300 MHz, CDCl₃, δ/ppm): 3.2 (t, 2H), 3.85 (s, 3H), 4.25 (t, 2H), 6.85 (dd, 1H), 7.15 (m, 1H), 7.5 (m, 3H), 7.6 (d, 2H).

GC-MS (Methode 2): Rₜ 10.54 min; m/z 342 (M⁺).

### Beispiel 3A

### 5-Fluor-2-[2-(4-trifluormethyl-phenyl)ethoxy]benzylalkohol

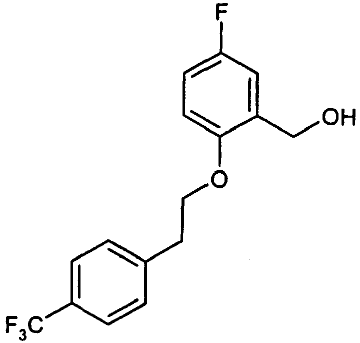

2.0 g (5.84 mmol) 5-Fluor-2-[2-(4-trifluormethyl-phenyl)ethoxy]benzoesäuremethylester werden in 25 ml THF vorgelegt, dann bei 0°C 4.38 ml (4.38 mmol) einer 1 M Lösung von Lithiumaluminiumhydrid in THF zugetropft und die Mischung bei RT bis zur vollständigen Umsetzung gerührt. Der Ansatz wird auf Eiswasser gegeben, mit Salzsäure angesäuert und mit Essigsäureethylester extrahiert. Alle flüchtigen Komponenten werden im Vakuum entfernt und das Rohprodukt chromatographisch an Kieselgel (Laufmittel: Cyclohexan/Essigsäureethylester 5:1) gereinigt. Es werden 1.7 g (82% d. Th.) der Titelverbindung erhalten.

¹H-NMR (400 MHz, CDCl₃, δ/ppm): 3.2 (t, 2H), 4.25 (t, 2H), 4.6 (s, 2H), 6.75 (m, 1H), 6.9 (m, 1H), 7.05 (dd, 1H), 7.4 (d, 2H), 7.6 (d, 2H).

GC-MS (Methode 1): Rₜ 10.71 min; m/z 314 (M⁺).

### Beispiel 4A

### {5-Fluor-2-[2-(4-trifluormethyl-phenyl)ethoxy]benzyl}-triphenylphosphoniumbromid

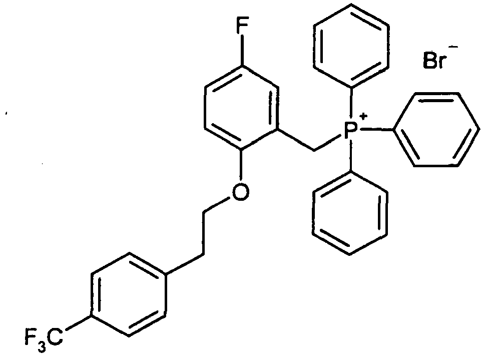

1.7 g (5.41 mmol) 5-Fluor-2-[2-(4-trifluormethyl-phenyl)ethoxy]benzylalkohol und 1.76 g (5.14 mmol) Triphenylphosphin-Hydrobromid werden in 20 ml Acetonitril drei Stunden unter Rückfluss erhitzt. Eine erste Produktfraktion wird bei -20°C auskristallisiert. Die Mutterlauge wird eingeengt, der Rückstand in Dichlormethan gelöst und mit Diethylether eine zweite Fraktion kristallisiert. Es werden insgesamt 2.71 g (76% d. Th.) der Titelverbindung erhalten.

¹H-NMR (400 MHz, DMSO-d₆, δ/ppm): 2.75 (t, 2H), 3.75 (t, 2H), 4.85 (d, 2H), 6.8 (m, 1H), 6.9 (m, 1H), 7.15 (m, 1H), 7.45 (d, 2H), 7.6 (m, 8H), 7.7 (m, 6H), 7.9 (t, 3H).

LC-MS (Methode 2): Pₜ 2.15 min; m/z 559 (M+H)⁺.

### Beispiel 5A

### 2-(2-Chlorbenzyloxy)-5-fluorbenzoesäuremethylester

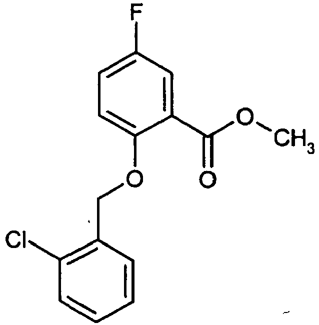

10.0 g (58 mmol) 5-Fluor-2-hydroxybenzoesäuremethylester [CAS 391-92-4], 13.3 g (64.5 mmol) 2-Chlorbenzylbromid [CAS 611-17-6], 40.6 g (293 mmol) Kaliumcarbonat und 14.6 g (88 mmol) Kaliumiodid werden in 50 ml Aceton gelöst und für 12 h unter Rückfluß gerührt. Das Lösungsmittel wird im Vakuum abdestilliert, der Rückstand in Ethylacetat aufgenommen und je zweimal mit 10%-iger Natronlauge, 1 N Salzsäure und Wasser gewaschen. Man trocknet über Magnesiumsulfat, filtriert, wäscht mit Ethylacetat nach und engt ein. Den Rückstand rührt man mit Petrolether aus, saugt den Feststoff ab und trocknet auf einem Tonteller. Es werden 10.0 g (33.9 mmol, 59% d. Th.) der Titelverbindung erhalten.

¹H-NMR (400 MHz, DMSO-d₆, δ/ppm): 3.81 (s, 3H), 5.22 (s, 2H), 7.31 (dd, 1H), 7.36-7.46 (m, 3H), 7.51 (m_{c}, 2H), 7.72 (m_{c}, 1H).

LC-MS (Methode 2): Rₜ 2.6 min; m/z 295 (M+H)⁺.

### Beispiel 6A

### 2-(2-Chlorbenzyloxy)-5-fluorbenzoesäure

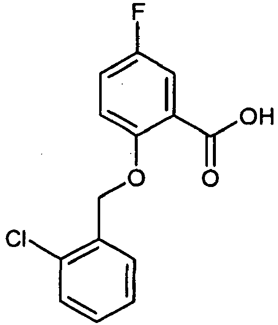

10.0 g (34 mmol) 2-(2-Chlorbenzyloxy)-5-fluorbenzoesäuremethylester werden in 80 ml Methanol und 40 ml 40%-iger Natronlauge gelöst und für 12 h bei RT gerührt. Das Lösungsmittel wird im Vakuum abdestilliert, der Rückstand mit so viel Wasser versetzt, bis alles gelöst ist, und dann mit Ethylacetat extrahiert. Die wässrige Phase stellt man mit Salzsäure sauer und extrahiert dreimal mit Ethylacetat. Man trocknet über Magnesiumsulfat, filtriert, wäscht mit Ethylacetat nach und engt ein. Den Rückstand versetzt man mit Petrolether, saugt die ausgefallenen Kristalle ab und trocknet auf einem Tonteller. Es werden 6.00 g (21.3 mmol, 63% d. Th.) der Titelverbindung erhalten.

¹H-NMR (400 MHz, DMSO-d₆, δ/ppm): 4.55 (d, 2H), 5.16 (s, 2H), 5.19 (t, 1H), 6.97-7.08 (m, 2H), 7.17 (dd, 1H), 7.42-7.3 (m, 2H), 7.51 (m_{c}, 1H), 7.61 (m_{c}, 1H).

HPLC (Methode 2): Rₜ 4.6 min.

MS (DCI): 284 (M+NH₄⁺).

### Beispiel 7A

### 2-(2-Chlorbenzyloxy)-5-fluorbenzylalkohol

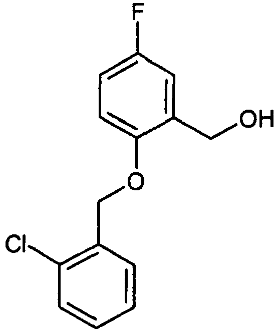

5.50 g (20 mmol) 2-(2-Chlorbenzyloxy)-5-fluorbenzoesäure werden in 25 ml THF gelöst, zum Rückfluß erhitzt und dann tropfenweise mit 20 ml 1 M Borandimethylsulfid-Komplex versetzt. Es wird 1 h bei Raumtemperatur nachgerührt. Das Lösungsmittel wird im Vakuum abdestilliert und der Rückstand mit Wasser versetzt. Anschließend wird vorsichtig (starke Gasentwicklung) mit 1 N Salzsäure sauer gestellt. Durch Zugabe von 1 N Natronlauge wird anschließend leicht basisch gestellt und die wässrige Phase dreimal mit Dichlormethan extrahiert. Die organische Phase trocknet man über Magnesiumsulfat, filtriert, wäscht mit Dichlormethan nach und engt ein. Den Rückstand versetzt man mit Petrolether, saugt das ausgefallene Kristallisat ab und trocknet auf einem Tonteller. Es werden 3.65 g (13.6 mmol, 68% d. Th.) der Titelverbindung erhalten.

¹H-NMR (400 MHz, DMSO-d₆, δ/ppm): 4.55 (d, 2H), 5.16 (s, 2H), 5.19 (t, 1H), 6.97-7.08 (m, 2H), 7.17 (dd, 1H), 7.37-7.42 (m, 2H), 7.51 (m_{c}, 1H), 7.61 (m_{c}, 1H).

HPLC (Methode 2): Rₜ 4.6 min.

MS (DCI): 284 (M+NH₄)⁺.

### Beispiel 8A

### [2-(2-Chlorbenzyloxy)-5-fluorbenzyl]-triphenylphosphoniumbromid

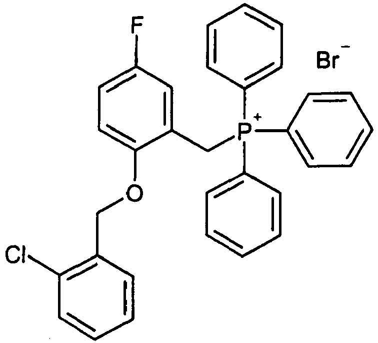

4.60 g (17 mmol) 2-(2-Chlorbenzyloxy)-5-fluorbenzylalkohol werden in 10 ml Acetonitril gelöst, dann 5.62 g (16 mmol) Triphenylphosphin-Hydrobromid zugegeben und die Suspension für 12 h unter Rückfluss gerührt. Weitere 2.80 g (8 mmol) Triphenylphosphin-Hydrobromid werden zugesetzt und erneut für 3 h unter Rückfluß gerührt. Das Lösungsmittel wird im Vakuum größtenteils abdestilliert, die ausgefallenen Kristalle mit Petrolether verrührt, abgesaugt und auf einem Tonteller getrocknet. Es werden 9.82 g (15.7 mmol, 92% d. Th.) der Titelverbindung erhalten.

¹H-NMR (300 MHz, DMSO-d₆, δ/ppm): 4.66 (d, 2H), 4.99 (d, 2H), 6.90 (dt, 1H), 6.96 (dd, 1H), 7.15 (m_{c}, 1H), 7.30-7.42 (m, 3H), 7.46-7.70 (m, 13H), 7.86 (m_{c}, 3H).

HPLC (Methode 1): Rₜ 5.0 min.

MS (ESI): 511 (M-Br)⁺.

### Beispiel 9A

### 4'-Trifluormethyl-biphenyl-4-carbonsäureethylester

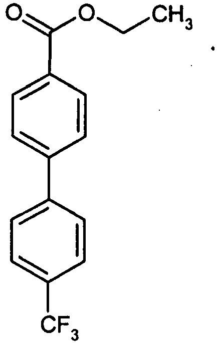

7.00 g (30.6 mmol) 4-Brombenzoesäureethylester werden in 60 ml 1,2-Dimethoxyethan gelöst und unter Argon mit 6.96 g (36.7 mmol) 4-Trifluormethylphenylboronsäure, 271 mg Bis(triphenylphosphin)palladium(II)chlorid und 40.7 ml einer 2 M wässrigen Natriumcarbonat-Lösung versetzt. Das Reaktionsgemisch wird anschließend 12 Stunden unter Rückfluß gerührt. Danach wird der Ansatz abgekühlt, über 1 g Extrelute filtriert, mit Dichlormethan gewaschen und das Lösungsmittel im Vakuum entfernt. Das erhaltene Rohprodukt wird durch Flash-Chromatographie an Kieselgel 60 (Laufmittel: Cyclohexan/Dichlormethan 2:1) gereinigt. Es werden 6.31 g (21.4 mmol, 70% d. Th.) der Titelverbindung erhalten.

¹H-NMR (300 MHz, CDCl₃, δ/ppm): 1.43 (t, 3H), 4.41 (q, 2H), 7.67 (d, 2H), 7.72 (s, 4H), 8.17 (d, 2H).

MS (EI): 294 (M⁺).

### Beispiel 10A

### (4'-Trifluormethyl-biphenyl-4-yl)methanol

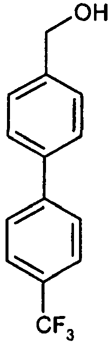

Eine Lösung von 6.24 g (21.2 mmol) 4'-Trifluormethyl-biphenyl-4-carbonsäureethylester in 60 ml trockenem THF wird bei 0°C langsam mit 12.7 ml (12.7 mmol) einer 1 M Lithiumaluminiumhydrid-Lösung in THF versetzt. Nach vollständiger Umsetzung wird mit gesättigter Ammoniumchlorid-Lösung hydrolysiert, in Essigsäureethylester aufgenommen, die organische Phase abgetrennt und über Natriumsulfat getrocknet. Nach Filtration wird das Lösungsmittel im Vakuum entfernt. Das erhaltene Rohprodukt wird durch Flash-Chromatographie an Kieselgel 60 (Laufmittel: Cyclohexan/Essigsäureethylester 5:1) gereinigt. Es werden 5.10 g (20.2 mmol, 95% d. Th.) der Titelverbindung erhalten.

¹H-NMR (300 MHz, DMSO-d₆, δ/ppm): 4.58 (d, 2H), 5.23 (t, 2H), 7.46 (d, 2H), 7.71 (d, 2H), 7.82 (d, 2H), 7.88 (d, 2H).

MS (EI): 252 (M⁺).

### Beispiel 11A

### 4-Chlormethyl-4'-trifluormethyl-biphenyl

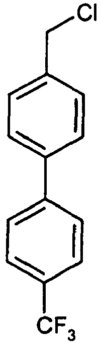

Eine Lösung von 5.00 g (19.8 mmol) (4'-Trifluormethyl-biphenyl-4-yl)methanol in 40 ml Chloroform wird mit 2.89 ml (39.7 mmol) Thionylchlorid, gelöst in 10 ml Chloroform, versetzt und über 12 Stunden bei Raumtemperatur gerührt. Nach vollständiger Umsetzung wird das Reaktionsgemisch bis zur Trockene eingeengt, der Rückstand in Essigsäureethylester aufgenommen und mit gesättigter Natriumcarbonat-Lösung gewaschen. Die organische Phase wird anschließend abgetrennt, über Natriumsulfat getrocknet und nach Filtration eingeengt. Das erhaltene Rohprodukt wird durch Flash-Chromatographie an Kieselgel 60 (Laufmittel: Cyclohexan/Essigsäureethylester 9:1) gereinigt. Es werden 5.26 g (19.4 mmol, 98% d. Th.) der Titelverbindung erhalten.

¹H-NMR (300 MHz, DMSO-d₆, δ/ppm): 4.83 (s, 2H), 7.58 (d, 2H), 7.78 (d, 2H), 7.91 (d, 2H), 7.82 (d, 2H).

MS (EI): 270 (M⁺).

### Beispiele 12A

### [5-Fluor-2-(4'-trifluormethyl-biphenyl-4-ylmethoxy)phenyl]methanol

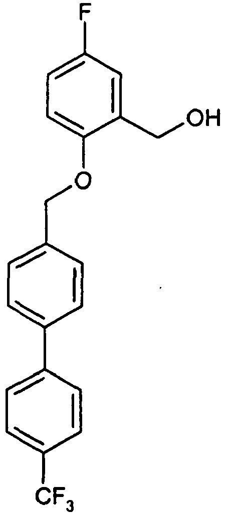

Eine Lösung von 4.59 g (36.9 mmol) 5-Fluor-2-hydroxybenzylalkohol [CAS 2357-33-7] in 200 ml trockenem Acetonitril wird mit 10.0 g (36.9 mmol) 4-Chlormethyl-4'-trifluormethyl-biphenyl und 6.13 g (44.3 mmol) wasserfreiem Kaliumcarbonat versetzt und 12 Stunden unter Rückfluß erhitzt. Anschließend wird der Ansatz zur Trockene eingeengt. Der Rückstand wird in Essigsäureethylester aufgenommen, mit Wasser und gesättigter Kochsalz-Lösung gewaschen und über Natriumsulfat getrocknet. Nach Einengen wird das erhaltene Rohprodukt durch Flash-Chromatographie an Kieselgel 60 (Laufmittel: Cyclohexan/Essigsäureethylester 5:1) gereinigt. Es werden 11.8 g (32.9 mmol, 89% d. Th.) der Titelverbindung erhalten.

¹H-NMR (300 MHz, DMSO-d₆, δ/ppm): 4.57 (d, 2H), 5.18 (m_{c}, 3H), 6.97-7.08 (m, 2H), 7.18 (dd, 1H), 7.58 (d, 2H), 7.75-7.83 (m, 4H), 7.91 (d, 2H).

MS (DCI): 394 (M+NH₄⁺)

HPLC (Methode 2): Rₜ 5.3 min.

### Beispiele 13A

### Triphenyl-[5-fluor-2-(4'-trifluormethyl-biphenyl-4-ylmethoxy)benzyl]-phosphoniumbromid

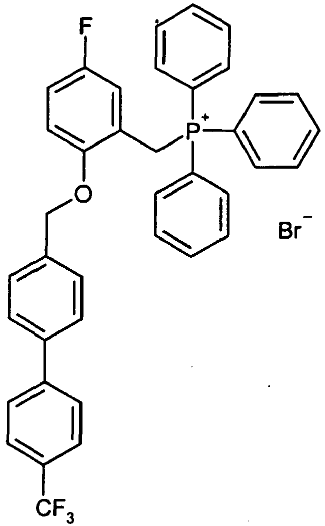

Eine Lösung von 3.00 g (7.97 mmol) [2-(4'-Trifluormethyl-biphenyl-4-ylmethoxy)phenyl]methanol in 20 ml Acetonitril wird mit 2.60 g (7.57 mmol) Triphenylphosphin-Hydrobromid versetzt und 20 Stunden unter Rückfluß erhitzt. Anschließend wird die Reaktionslösung bis zur Trockene eingeengt und das erhaltene Öl in Petrolether aufgenommen und verrieben. Dabei kristallisiert das Produkt als weißer Feststoff aus. Es werden 2.31 g (3.29 mmol, 41% d. Th.) der Titelverbindung erhalten.

¹H-NMR (300 MHz, DMSO-d₆, δ/ppm): 4.72 (s, 2H), 5.05-4.98 (d, 2H), 6.92-6.86 (m, 1H), 7.00-6.96 (m, 1H), 7.20-7.12 (m, 1H), 7.35-7.30 (m, 2H), 7.75-7.57 (m, 14H), 7.95-7.82 (m, 7H).

MS (ESI): 621 (M-Br)⁺.

### Beispiel 14A

### 3,5-Difluor-4-trüsopropylsilanoxy-benzaldehyd

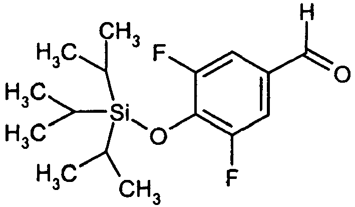

30.0 g (190 mmol) 3,5-Difluor-4-hydroxybenzaldehyd [CAS 118276-06-5] werden unter Argon in 600 ml Dichlormethan gelöst und bei 0°C mit 44.7 g (417 mmol) 2,6-Lutidin versetzt. Anschließend tropft man 87.2 g (284 mmol) Trifluormethansulfonsäuretriisopropylsilylester hinzu und rührt dann für 12 h bei RT. Das Lösungsmittel wird im Vakuum abdestilliert und der Rückstand mit Ammoniumchlorid-Lösung versetzt, 5 min gerührt und die organische Phase abgetrennt. Man extrahiert nochmals mit Dichlormethan, vereinigt die organischen Phasen, wäscht mit gesättigter Kochsalz-Lösung, trocknet über Magnesiumsulfat, filtriert, wäscht mit Dichlormethan nach und destilliert das Lösungsmittel im Vakuum ab. Nach Chromatographie an Kieselgel 60 (Laufmittel: Cyclohexan) werden 34.9 g (111 mmol, 58% d. Th.) der Titelverbindung erhalten.

¹H-NMR (400 MHz, DMSO-d₆, δ/ppm): 1.06 (d, 18H), 1.29 (sept, 3H), 7.68-7.74 (m, 2H), 9.86 (s, 1H).

HPLC (Methode 3): Rₜ 3.2 min.

MS (EI): 314 (M⁺).

### Beispiel 15A

### 1,3-Difluor-5-hydroxymethyl-2-triisopropylsilanoxy-benzol

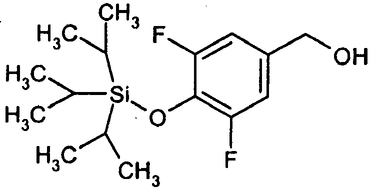

Man legt 35.0 g (111 mmol) 3,5-Difluor-4-trüsopropylsilanoxy-benzaldehyd in 150 ml Wasser und 40 ml Methanol vor, kühlt auf 0°C ab, gibt 8.42 g (222 mmol) Natriumborhydrid portionsweise hinzu und rührt anschließend über Nacht bei RT. Man stellt vorsichtig mit 1 N Salzsäure sauer, extrahiert mit Ethylacetat, wäscht die vereinigten organischen Phasen mit gesättigter Kochsalz-Lösung, trocknet über Magnesiumsulfat und destilliert das Lösungsmittel im Vakuum ab. Nach Chromatographie an Kieselgel 60 (Laufmittel: Cyclohexan/Ethylacetat 4:1) werden 29.5 g (93.2 mmol, 84% d. Th.) der Titelverbindung erhalten.

¹H-NMR (400 MHz, DMSO-d₆, δ/ppm): 1.05 (d, 18H), 1.25 (sept, 3H), 4.42 (d, 2H), 5.31 (t, 1H), 6.97-7.04 (m, 2H).

HPLC (Methode 1): Rₜ 5.9 min.

MS (DCI): 333 (M+NH₄⁺).

### Beispiel 16A

### 5-Brommethyl-1,3-difluor-2-triisopropylsilanoxy-benzol

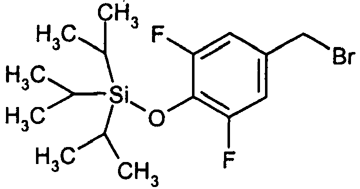

Unter Argon werden 7.40 g (23.4 mmol) 1,3-Difluor-5-hydroxymethyl-2-triisopropylsilanoxybenzol in 90 ml trockenem Dichlormethan unter Rühren bei 0°C gelöst. Zu dieser Lösung gibt man nacheinander 10.1 g (30.4 mmol) Tetrabromkohlenstoff sowie 7.97 g (30.4 mmol) Triphenylphosphin hinzu und rührt anschließend für 12 h unter Argon bei Rückfluß. Das Reaktionsgemisch wird mit Wasser gewaschen, die organische Phase abgetrennt, über Magnesiumsulfat getrocknet, filtriert und das Lösungsmittel im Vakuum abdestilliert. Der ölige Rückstand wird in Diethylether aufgenommen und bei RT ausgerührt, dann abfiltriert und das Filtrat eingeengt. Nach Chromatographie des Rückstands an Kieselgel 60 (Laufmittel: Cyclohexan/Ethylacetat 9:1) werden 7.42 g (15.6 mmol, 84% d. Th.) der Titelverbindung erhalten.

¹H-NMR (300 MHz, DMSO-d₆, δ/ppm): 1.05 (d, 18H), 1.25 (sept, 3H), 4.61 (s, 2H), 7.19-7.28 (m, 2H).

HPLC (Methode 1): Rₜ 7.4 min.

### Beispiel 17A

### 2-Allyloxycarbonyl-2-(3,5-difluor-4-triisopropylsilanyloxy-benzyl)heptandisäure-1-allylester-7-ethylester

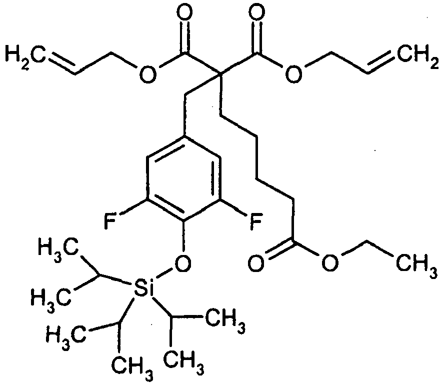

3.17 g (10.14 mmol) 2-Allyloxycarbonyl-heptandisäure-1-allylester-7-ethylester (Beispiel 48A) werden in 30 ml DMF gelöst, bei 0°C portionsweise mit 0.45 g (11.15 mmol) 60%-igem Natriumhydrid versetzt und die Mischung 30 Minuten bei RT nachgerührt. Nach erneutem Abkühlen auf 0°C wird eine Lösung von 5 g (13.18 mmol) 3,5-Difluor-4-triisopropylsilanyloxy-benzylbromid in 20 ml DMF zugetropft und das Gemisch unter Erwärmung auf Raumtemperatur über Nacht nachgerührt. Der Ansatz wird auf Eis gegeben, mit Salzsäure schwach angesäuert und mit Essigsäureethylester extrahiert. Die vereinigten organischen Phasen werden über Natriumsulfat getrocknet und eingeengt. Das erhaltene Rohprodukt wird chromatographisch an Kieselgel (Laufmittel: Cyclohexan, dann Cyclohexan/Essigsäureethylester 10:1) gereinigt. Es werden 4.57 g (73% d. Th.) der Titelverbindung erhalten.

¹H-NMR (400 MHz, CDCl₃, δ/ppm): 1.1 (d, 18H), 1.25 (m, 8H), 1.65 (q, 2H), 1.8 (m, 2H), 2.3 (t, 2H), 3.1 (s, 2H), 4.15 (t, 2H), 4.6 (d, 4H), 5.25 (d, 2H), 5.35 (d, 2H), 5.85 (m, 2H), 6.6 (d, 2H).

LC-MS (Methode 1): Rₜ 3.75 min; m/z 611 (M+H)⁺.

### Beispiel 18A

### 2-(3,5-Difluor-4-triisopropylsilanyloxy-benzyl)heptandisäure-7-ethylester

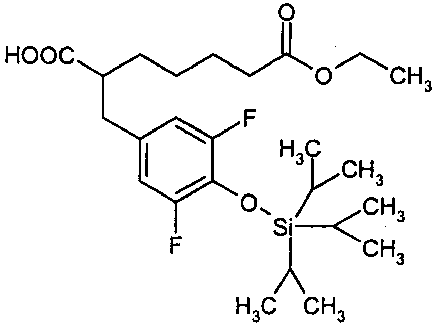

4.2 g (6.88 mmol) 2-Allyloxycarbonyl-2-(3,5-difluor-4-triisopropylsilanyloxy-benzyl)-heptandisäure-1-allylester-7-ethylester, 39 mg (0.17 mmol) Palladium(II)acetat und 135 mg (0.52 mmol) Triphenylphosphin werden in 30 ml Dioxan unter Argon gerührt. Anschließend wird eine Lösung von 2.3 g (22.69 mmol) Triethylamin und 0.79 g (17.19 mmol) Ameisensäure in 20 ml Dioxan zugetropft und das Gemisch über Nacht auf 100°C erwärmt. Der abgekühlte Ansatz wird eingeengt und das Rohprodukt an Kieselgel chromatographiert (Laufmittel: Cyclohexan/Essigsäureethylester 10:1, 3:1). Es werden 2.07 g (52% d. Th.) der Titelverbindung erhalten.

¹H-NMR (300 MHz, CDCl₃, δ/ppm): 1.1 (d, 18H), 1.25 (t, 3H), 1.3-1.7 (m, 8H), 2.3 (t, 2H), 2.65 (m, 2H), 2.9 (m, 1H), 4.15 (q, 2H), 6.7 (d, 2H).

LC-MS (Methode 3): Rₜ 3.42 min; m/z 485 (M-H)⁻.

### Beispiel 19A

### 6-(3,5-Difluor-4-triisopropylsilanyloxy-benzyl)-7-hydroxyheptansäureethylester

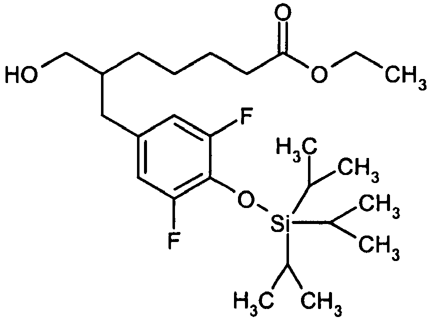

1.6 g (3.29 mmol) 2-(3,5-Difluor-4-triisopropylsilanyloxy-benzyl)heptandisäure-7-ethylester werden in 30 ml THF gelöst und unter Argon auf -10°C gekühlt. Anschließend werden 4.27 ml (4.27 mmol) einer 1 M Boran-THF-Komplex-Lösung zugetropft und das Gemisch bei 0°C bis zur vollständigen Umsetzung nachgerührt. Der Ansatz wird mit Wasser hydrolysiert, mit Essigsäureethylester extrahiert und die vereinigten organischen Phasen eingeengt. Das erhaltene Rohprodukt wird chromatographisch an Kieselgel (Laufmittel: Cyclohexan/Essigsäureethylester 7:1, 3:1) gereinigt. Es werden 1.18 g (76% d. Th.) der Titelverbindung erhalten.

¹H-NMR (300 MHz, CDCl₃, δ/ppm): 1.1 (d, 18H), 1.25 (t, 3H), 1.3-1.8 (m, 10H), 2.3 (t, 2H), 2.55 (ddd, 2H), 3.5 (d, 2H), 4.15 (q, 2H), 6.7 (d, 2H).

LC-MS (Methode 3): Rₜ 3.53 min; m/z 473 (M+H)⁺.

### Beispiel 20A

### 7-(3,5-Difluor-4-triisopropylsilanyloxy-phenyl)-6-formylheptansäureethylester

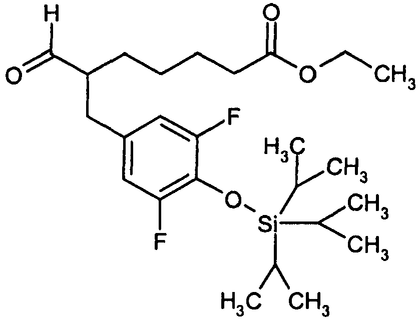

1.19 g (2.52 mmol) 6-(3,5-Difluor-4-triisopropylsilanyloxy-benzyl)-7-hydroxyheptansäureethylester werden mit 5 g 4Å-Molekularsieb und 442 mg (3.78 mmol) *N*-Methylmorpholin-*N*-oxid in 50 ml Dichlormethan zehn Minuten bei Raumtemperatur gerührt. Es werden 44 mg (0.13 mmol) Tetrapropylammoniumperruthenat zugegeben und das Gemisch bis zur vollständigen Umsetzung bei Raumtemperatur gerührt. Der Ansatz wird filtriert und das Filtrat mit Wasser und gesättigter Natriumchlorid-Lösung gewaschen. Die organische Phase wird über Natriumsulfat getrocknet und eingeengt. Das erhaltene Rohprodukt wird chromatographisch an Kieselgel (Laufmittel: Cyclohexan/Essigsäureethylester 3:1) gereinigt. Es wird 1.0 g (84% d. Th.) der Titelverbindung erhalten.

¹H-NMR (400 MHz, CDCl₃, δ/ppm): 1.1 (d, 18H), 1.25 (t, 3H), 1.3-1.8 (m, 9H), 2.3 (t, 2H), 2.6 (dd, 2H), 2.9 (dd, 1H), 4.1 (q, 2H), 6.65 (d, 2H), 9.65 (d, 1H).

LC-MS (Methode 3): Rₜ 3.63 min; m/z 488 (M+NH₄)⁺.

### Beispiel 21A

### 6-(3,5-Difluor-4-triisopropylsilanyloxy-benzyl)-8-[5-fluor-2-(4'-trifluormethyl-biphenyl-4-yl-methoxy)phenyl]oct-7-ensäureethylester

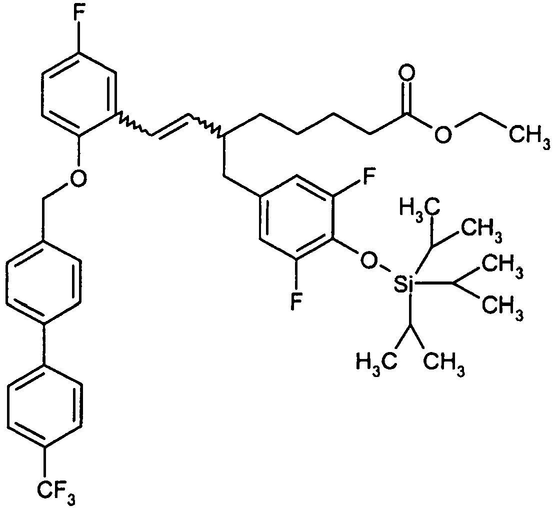

Zu einer Suspension von 784 mg (1.06 mmol) [5-Fluor-2-(4'-trifluormethyl-biphenyl-4-yl-methoxy)benzyl]-triphenylphosphoniumbromid in 10 ml THF werden bei 0°C tropfenweise 0.7 ml (1.12 mmol) einer 1.6 M Lösung von n-Butyllithium in Hexan getropft und anschließend 45 min nachgerührt. Danach wird eine Lösung von 500 mg (1.06 mmol) 7-(3,5-Difluor-4-triisopropyl-silanyloxy-phenyl)-6-formylheptansäureethylester in 10 ml THF bei 0°C zugetropft und bei dieser Temperatur 2 h nachgerührt. Der Ansatz wird mit gesättigter Ammoniumchlorid-Lösung versetzt und mit Essigsäureethylester extrahiert. Die vereinigten organischen Phasen werden über Natriumsulfat getrocknet und eingeengt. Das erhaltene Rohprodukt wird chromatographisch an Kieselgel (Laufmittel: Cyclohexan/Essigsäureethylester 7:1) gereinigt. Es werden 813 mg (84% d. Th.) der Titelverbindung als *E*/*Z*-Gemisch (2:1) erhalten.

¹H-NMR (400 MHz, CDCl₃, δ/ppm): 1.1 (m, 18H), 1.2 (m, 3H), 1.3-1.7 (m, 9H), 2.35 (m, 2H), 2.4-2.85 (m, 3H), 4.1 (q, 2H), 5.0 (s, 2H (*Z*)), 5.05 (s, 2H (*E*)), 5.45 (t, 1H (*Z*)), 5.95 (dd, 1H (*E*)), 6.5-6.7 (m, 3H), 6.85 (m, 2H), 7.05 (dd, 1H (*E*)), 7.5 (m, 2H), 7.65 (t, 2H), 7.7 (d, 4H).

HPLC (Methode 4): Rₜ 18.4 und 18.9 min.

MS (ESI): m/z 830 (M+NH₄)⁺.

### Beispiel 22A

### 6-(3,5-Difluor-4-triisopropylsilanyloxy-benzyl)-8-[5-fluor-2-(2-chlorbenzyloxy)phenyl]oct-7-ensäureethylester

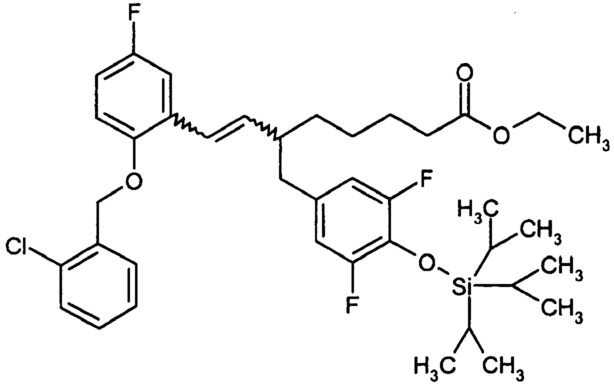

Analog zu Beispiel 21A wird bei der Umsetzung von [2-(2-Chlorbenzyloxy)-5-fluorbenzyl]-triphenylphosphoniumbromid und 7-(3,5-Difluor-4-triisopropylsilanyloxy-phenyl)-6-formylheptansäureethylester die Titelverbindung mit 70% d. Th. als *E*/*Z* Gemisch (2:1) erhalten.

¹H-NMR (400 MHz, CDCl₃, δ/ppm): 1.1 (m, 18H), 1.25 (m, 3H), 1.3-1.7 (m, 9H), 2.15-2.3 (m, 2H), 2.4-2.85 (m, 3H), 4.1 (dq, 2H), 5.05 (s, 2H (*Z*)), 5.15 (s, 2H (*E*)), 5.45 (t, 1H (*Z*)), 5.95 (dd, 1H (*E*)), 6.5-6.7 (m, 3H), 6.8 (m, 2H), 7.05 (dd, 1H (*E*)), 7.3 (m, 2H), 7.4 (m, 1H), 7.45 (m, 1H).

LC-MS (Methode 2): Rₜ 4.15 min; m/z 720 (M+NH₄)⁺.

### Beispiel 23A

### E-6-(3,5-Difluor-4-triisopropylsilanyloxy-benzyl)-8-[{5-fluor-2-[2-(4-trifluormethyl-phenyl)-ethoxy]phenyl}oct-7-ensäureethylester

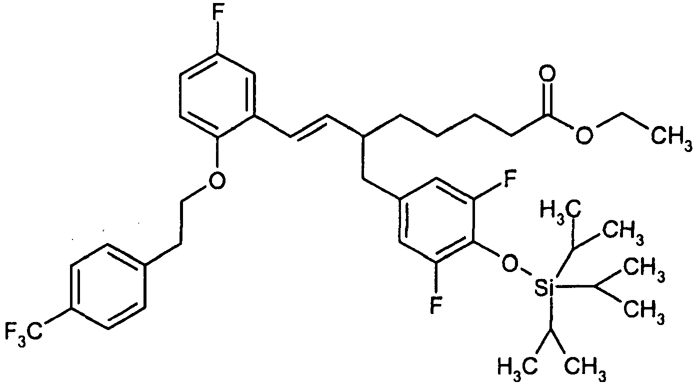

Analog zu Beispiel 21A wird bei der Umsetzung von {5-Fluor-2-[2-(4-trifluormethyl-phenyl)-ethoxy]benzyl}-triphenylphosphoniumbromid und 7-(3,5-Difluor-4-triisopropylsilanyloxy-phenyl)-6-formylheptansäureethylester die Titelverbindung mit 70% d. Th. erhalten.

¹H-NMR (300 MHz, CDCl₃, δ/ppm): 1.1 (m, 18H), 1.2 (m, 3H), 1.3-1.7 (m, 9H), 2.25 (m, 3H), 2.4-2.6 (m, 2H), 3.15 (m, 2H), 4.15 (m, 4H), 5.85 (dd, 1H), 6.35 (d, 1H), 6.65 (m, 2H), 6.7-6.9 (m, 2H), 7.0 (dd, 1H), 7.4 (m, 2H), 7.55 (m, 2H).

LC-MS (Methode 1): Rₜ 4.34 min; m/z 593 (M-C₉H₂₁Si)⁻.

### Beispiel 24A

### 6-(3,5-Difluor-4-hydroxybenzyl)-8-[5-fluor-2-(4'-trifluormethyl-biphenyl-4-ylmethoxy)phenyl]oct-7-ensäureethylester

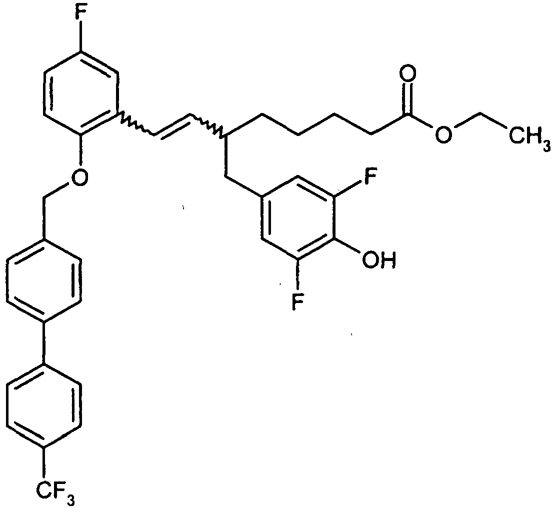

720 mg (0.89 mmol) 6-(3,5-Difluor-4-triisopropylsilanyloxy-benzyl)-8-[5-fluor-2-(4'-trifluor-methyl-biphenyl-4-ylmethoxy)phenyl]oct-7-ensäureethylester werden in 15 ml THF bei 0°C unter Argon vorgelegt, mit 1.77 ml (1.77 mmol) einer 1 M Lösung von Tetra-n-butylammoniumfluorid in THF versetzt und die Mischung bei Raumtemperatur bis zur vollständigen Umsetzung nachgerührt. Alle flüchtigen Komponenten werden im Vakuum entfernt. Das erhaltene Rohprodukt wird chromatographisch an Kieselgel (Laufmittel: Cyclohexan/Essigester 7:1) gereinigt. Es werden 400 mg (68% d. Th.) der Titelverbindung als *E*/*Z*-Gemisch (2: 1) erhalten.

¹H-NMR (400 MHz, CDCl₃, δ/ppm): 1.25 (dt, 3H), 1.3-1.7 (m, 6H), 2.25 (m, 2H), 2.4-2.8 (m, 3H), 4.1 (dq, 2H), 5.0 (s, 2H (*Z*)), 5.05 (s, 2H (*E*)), 5.45 (t, 1H (*Z*)), 5.95 (dd, 1H (*E*))*,* 6.55-6.9 (m, 5H), 7.1 (dd, 1H (*E*)), 7.45 (m, 2H), 7.65 (t, 2H), 7.7 (d, 4H).

LC-MS (Methode 1): Rₜ 3.42 min; m/z 674 (M+NH₄)⁺.

Auf analoge Weise werden die folgenden Verbindungen erhalten:

| **Beispiel Nr.** | **Struktur** | **Analytische Daten** |
|---|---|---|
| **25A** | 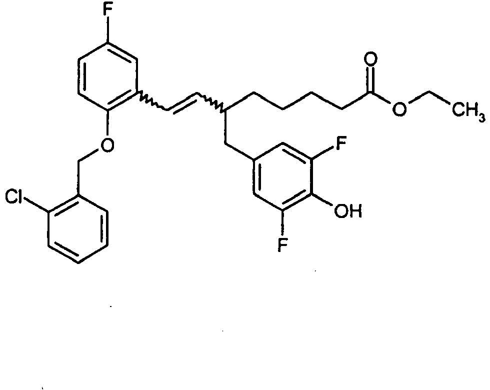 | **¹H-NMR** (300 MHz, CDCl₃, δ/ppm): 1.25 (m, 3H), 1.3-1.7 (m, 6H), 2.25 (m, 2H), 2.3-2.8 (m, 3H), 4.1 (q, 2H), 5.05 (s, 2H (*Z*)), 5.1 (s, 2H (*E*))*,* 5.45 (t, 1H (*Z*)), 5.95 (dd, 1H (*E*)), 6.55 (m, 2H), 6.7 (m, 2H), 6.85 (m, 2H), 7.05 (dd, 1H (*E*)), 7.3 (m, 2H), 7.45 (m, 2H). |
| | | LC-MS (Methode 2): Rₜ 3.12 min; m/z 564 (M+NH₄)⁺. |
| **26A** | 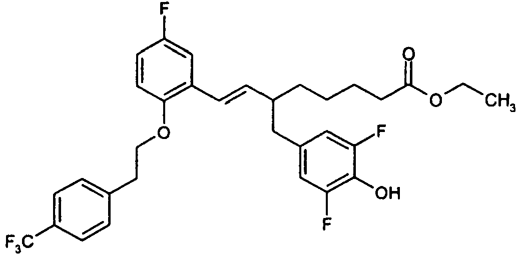 | ¹H-NMR (300 MHz, CDCl₃, δ/ppm): 1.2 (m, 3H), 1.3-1.7 (m, 6H), 2.3 (m, 3H), 2.6 (m, 2H), 3.15 (m, 2H), 4.1 (m, 4H), 5.85 (dd, 1H), 6.35 (d, 1H), 6.6 (m, 2H), 6.7-6.9 (m, 2H), 7.05 (dd, 1H), 7.4 (m, 2H), 7.55 (m, 2H). |
| | | LC-MS (Methode 2): Rₜ = 3.11 min; m/z 595 (M+H)⁺. |

### Beispiel 27A

### 2-[2-(4-Methoxycarbonyl-phenyl)ethyl]malonsäurediallylester

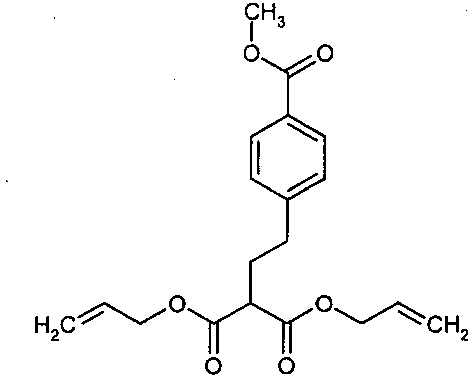

Eine Lösung von 5 g (27.15 mmol) Malonsäurediallylester in 25 ml Dioxan wird bei 0°C portionsweise mit 814 mg (20.36 mmol) Natriumhydrid versetzt (Vorsicht: Wasserstoffentwicklung). Nach Erwärmen auf Raumtemperatur wird der Ansatz 30 min bei 40°C nachgerührt. Anschließend werden 3.3 g (13.57 mol) 4-(2-Bromethyl)benzoesäuremethylester, gelöst in 25 ml Dioxan, langsam bei 40°C zugetropft und die Reaktionslösung über Nacht bei 110°C gerührt. Nach Abkühlen auf Raumtemperatur wird das Reaktionsgemisch auf 120 ml gesättigte Ammoniumchlorid-Lösung gegeben. Der Ansatz wird dreimal mit Essigsäureethylester extrahiert, die vereinigten organischen Phasen mit gesättigter Natriumchlorid-Lösung gewaschen und über Natriumsulfat getrocknet. Nach Filtration wird das Lösungsmittel im Vakuum bis zur Trockene eingeengt. Anschließend wird durch Hochvakuum-Destillation überschüssiger Malonsäurediallylester entfernt (Siedepunkt: 57°C, 0.074 mbar). Der Destillationsrückstand wird durch Flash-Chromatographie an Kieselgel (Laufmittel: Cyclohexan/Essigsäureethylester 9:1 → 1:1) gereinigt. Es werden 2.8 g (26% d. Th.) eines farblosen Feststoffes erhalten.

¹H-NMR (400 MHz, DMSO-d₆, δ/ppm): 7.90 (2H, d), 7.37 (2H, d), 5.98-5.81 (2H, m), 5.38-5.18 (4H, m), 4.68-4.54 (4H, m), 3.85 (3H, s), 3.59 (1H, t), 2.69 (2H, t), 2.17-2.04 (2H, m).

MS (DCI): 364 (M+NH₄⁺).

### Beispiel 28A

### 2-(3,5-Difluor-4-triisopropylsilanyloxy-benzyl)-2-[2-(4-methoxycarbonyl-phenyl)ethyl]malonsäurediallylester

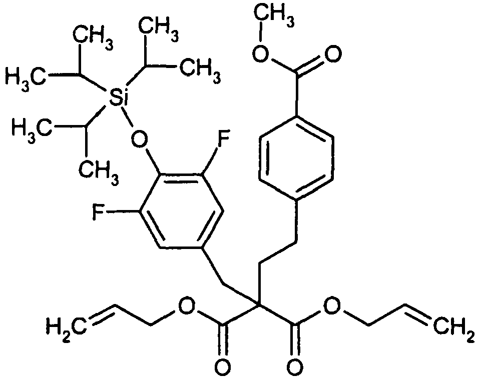

5.9 g (17.05 mmol) 2-[2-(4-Methoxycarbonyl-phenyl)ethyl]malonsäurediallylester werden in 125 ml Dioxan gelöst, bei 0°C portionsweise mit 818 mg (20.46 mmol) 60%-igem Natriumhydrid versetzt und die Mischung 30 min bei Raumtemperatur nachgerührt. Nach erneutem Abkühlen auf 0°C wird eine Lösung von 9.7 g (25.57 mmol) 3,5-Difluor-4-triisopropylsilanyloxy-benzylbromid in 125 ml Dioxan zugetropft und der Ansatz unter Erwärmung auf Raumtemperatur 2 h nachgerührt. Danach wird die Reaktionslösung auf 250 ml gesättigte Ammoniumchlorid-Lösung gegeben und mit Essigsäureethylester extrahiert. Die vereinigten organischen Phasen werden über Natriumsulfat getrocknet und eingeengt. Das erhaltene Rohprodukt wird mittels präparativer HPLC gereinigt. Es werden 7.2 g (65% d. Th.) der Titelverbindung erhalten.

¹H-NMR (300 MHz, DMSO-d₆, δ/ppm): 7.88 (2H, d), 7.30 (2H, d), 6.91 (2H, d), 5.98-5.81 (2H, m), 5.39-5.20 (4H, m), 4.63 (4H, d), 3.83 (3H, s), 3.27 (2H, s), 2.71-2.58 (2H, m), 1.98-1.83 (2H, m), 1.30-1.12 (3H, m), 1.02 (18H, d).

MS (EI): 645 (M+H⁺), 667 (M+Na⁺).

### Beispiel 29A

### 4-[3-Carboxy-4-(3,5-difluor-4-triisopropylsilanyloxy-phenyl)butyl]benzoesäuremethylester

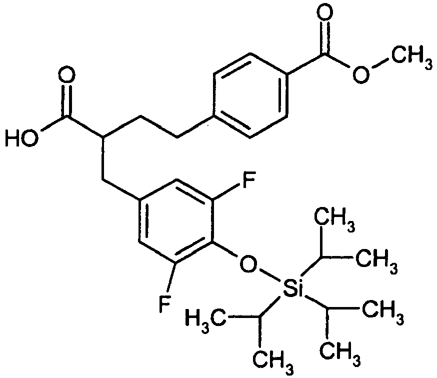

9.2 g (14.27 mmol) 2-(3,5-Difluor-4-triisopropylsilanyloxy-benzyl)-2-[2-(4-methoxycarbonyl-phenyl)ethyl]malonsäurediallylester, 64 mg (0.29 mmol) Palladium(II)acetat und 262 mg (1 mmol) Triphenylphosphin werden in 300 ml Dioxan unter Argon gerührt. Anschließend wird eine Lösung von 4.76 g (47.08 mmol) Triethylamin und 1.64 g (35.67 mmol) Ameisensäure in 200 ml Dioxan zugetropft und das Gemisch 2 h lang auf 100°C erwärmt. Der abgekühlte Ansatz wird eingeengt und das Rohprodukt an Kieselgel chromatographiert (Laufmittel: Cyclohexan/Essigsäureethylester 10:1 → 4:1). Es werden 3.69 g (49% d. Th.) der Titelverbindung erhalten.

¹H-NMR (300 MHz, CDCl₃, δ/ppm): 12.50-10.00 (1H, breit), 7.95 (2H, d), 7.20 (2H, d), 6.65 (2H, d), 3.91 (3H, s), 2.99-2.84 (1H, m), 2.83-2.49 (4H, m), 2.08-1.90 (1H, m), 1.89-1.71 (1H, m), 1.34-1.17 (3H, m), 1.09 (18H, m).

MS (EI): 519 (M-H⁻).

### Beispiel 30A

### 4-[3-(3,5-Difluor-4-triisopropylsilanyloxy-benzyl)-4-hydroxybutyl]benzoesäuremethylester

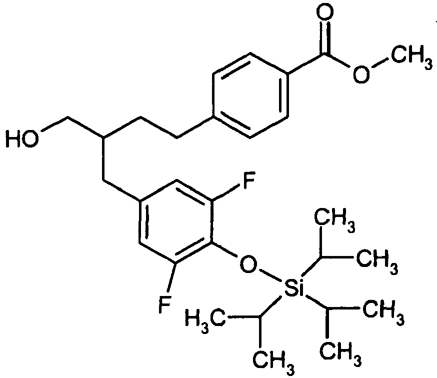

4.2 g (8.07 mmol) 4-[3-Carboxy-4-(3,5-difluor-4-triisopropylsilanyloxy-phenyl)butyl]benzoesäure-methylester werden in 200 ml THF gelöst und unter Argon auf -10°C gekühlt. Anschließend werden 16.13 ml (16.13 mmol) einer 1 M Boran-THF-Komplex-Lösung zugetropft und die Mischung bei 0°C bis zur vollständigen Umsetzung nachgerührt. Der Ansatz wird mit gesättigter Ammoniumchlorid-Lösung hydrolysiert, mit Diethylether extrahiert und die vereinigten organischen Phasen eingeengt. Das erhaltene Rohprodukt wird chromatographisch an Kieselgel (Lauf-mittel: Cyclohexan/Essigsäureethylester 20:1 → 10:1 → 4:1) gereinigt. Es werden 2.92 g (71% d. Th.) der Titelverbindung erhalten.

¹H-NMR (400 MHz, DMSO-d₆, δ/ppm): 7.85 (2H, d), 7.28 (2H, d), 6.89 (2H, d), 4.54 (1H, t), 3.83 (3H, s), 2.75-2.54 (3H, m), 1.72-1.52 (3H, m), 1.50-1.37 (3H, m), 1.30-1.15 (3H, m), 1.04 (18H, d).

MS (DCI): 524 (M+NH₄⁺).

### Beispiel 31A

### 4-[3-(3,5-Difluor-4-triisopropylsilanyloxy-benzyl)-4-oxo-butyl]benzoesäuremethylester

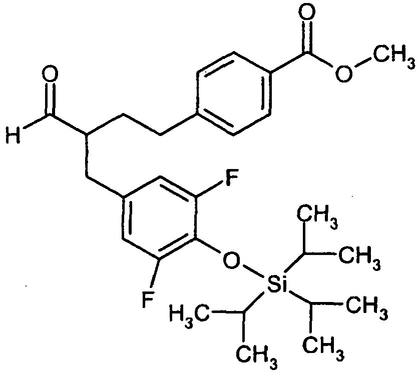

Eine Lösung von 2.9 g (5.72 mmol) 4-[3-(3,5-Difluor-4-triisopropylsilanyloxy-benzyl)-4-hydroxybutyl]benzoesäuremethylester in 50 ml Dichlormethan wird mit 1.48 g (6.87 mmol) Pyridiniumchlorochromat (PCC) versetzt und 12 h bei Raumtemperatur gerührt. Nach vollständigem Umsatz werden ca. 10 g Kieselgel zugegeben und das Lösungsmittel im Vakuum bis zur Trockene entfernt. Der Rückstand wird durch Flash-Chromatographie an Kieselgel (Laufmittel: Cyclohexan/Essigsäureethylester 10:1 → 4:1) gereinigt. Es werden 1.96 g (68% d. Th.) eines farblosen Feststoffs erhalten.

¹H-NMR (300 MHz, DMSO-d₆, δ/ppm): 9.64 (1H, s), 7.86 (2H, d), 7.29 (2H, d), 6.98 (2H, d), 3.83 (3H, s), 3.32 (2H, d), 3.01-2.82 (1H, m), 2.76-2.54 (2H, m), 1.94-1.73 (1H, m), 1.72-1.56 (1H, m), 1.31-1.11 (3H, m), 1.04 (18H, d).

MS (DCI): 522 (M+NH₄⁺).

### Beispiel 32A

### E-4-[3-(3,5-Difluor-4-triisopropylsilanyloxy-benzyl)-5-(2-hydroxyphenyl)pent-4-enyl]benzoesäuremethylester

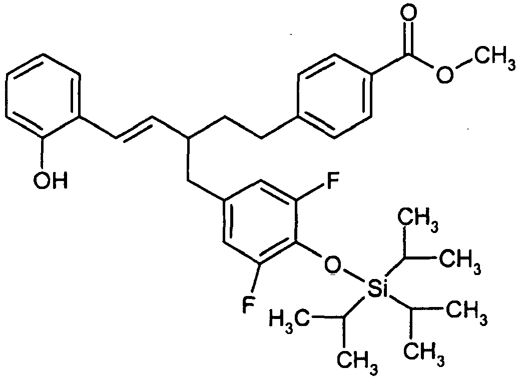

Zu einer Lösung von 2.38 g (5.14 mmol) (2-Hydroxybenzyl)-triphenylphosphoniumbromid in 50 ml THF werden bei 0°C 7.49 ml (11.98 mmol) einer 1.6 M Lösung von n-Butyllithium in Hexan langsam hinzugefügt. Anschließend werden bei dieser Temperatur 2.16 g (4.28 mmol) 4-[3-(3,5-Difluor-4-triisopropylsilanyloxy-benzyl)-4-oxo-butyl]benzoesäuremethylester langsam zudosiert. Die Reaktionslösung wird bei 0°C 2 Stunden nachgerührt, dann mit 100 ml gesättigte Ammoniumchlorid-Lösung versetzt und mit Diethylether extrahiert. Die vereinigten organischen Phasen werden mit gesättigter Natriumchlorid-Lösung gewaschen und über Natriumsulfat getrocknet. Nach Filtration wird das Lösungsmittel bis zur Trockene eingeengt. Das erhaltene Rohprodukt wird durch Flash-Chromatographie an Kieselgel (Laufmittel: Cyclohexan/Essigsäureethylester 10:1) gereinigt. Es werden 2.37 g (90% d. Th.) eines farblosen Feststoffes erhalten.

¹H-NMR (400 MHz, DMSO-d₆, δ/ppm): 9.44 (1H, s), 7.85 (2H, d), 7.30 (2H, d), 7.01 (1H, t), 6.91 (2H, d), 6.79 (1H, d), 6.72 (1H, t), 6.45 (1H, d), 6.08-5.97 (1H, m), 3.84 (3H, s), 2.80-2.67 (2H, m), 2.66-2.54 (2H, m), 2.48-2.36 (1H, m), 1.80-1.67 (1H, m), 1.67-1.54 (1H, m), 1.29-1.11 (3H, m), 1.01 (18H, d).

MS (EI): 595 (M+H⁺).

### Beispiel 33A

### E-4-(5-[2-(4-tert.-Butylbenzyloxy)phenyl]-3-(3,5-difluor-4-triisopropylsilanyloxy-benzyl)pent-4-enyl]benzoesäuremethylester

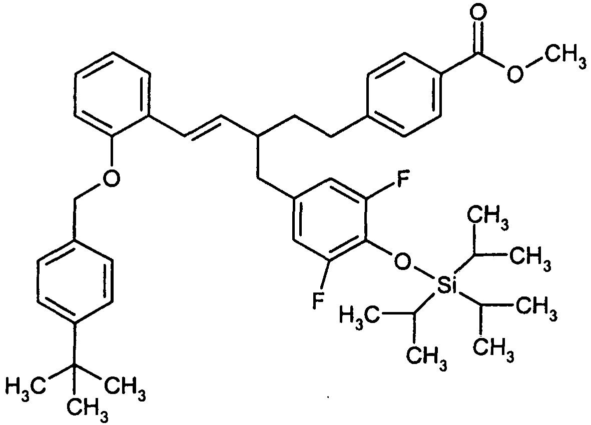

Eine Lösung von 1.3 g (2.19 mmol) *E*-4-[3-(3,5-Difluor-4-triisopropylsilanyloxy-benzyl)-5-(2-hydroxyphenyl)pent-4-enyl]benzoesäuremethylester in 20 ml trockenem Acetonitril wird mit 600 mg (2.62 mmol) 4-*tert*.-Butylbenzylbromid und 450 mg (3.28 mmol) wasserfreiem Kaliumcarbonat versetzt und 12 Stunden unter Rückfluss erhitzt. Anschließend wird der Ansatz zur Trockene eingeengt. Der Rückstand wird in Essigsäureethylester aufgenommen, mit Wasser und gesättigter Kochsalz-Lösung gewaschen, über Natriumsulfat getrocknet und eingeengt. Das erhaltene Rohprodukt wird mittels präparativer HPLC gereinigt. Es werden 780 mg (48% d. Th.) eines farblosen Öls erhalten.

LC-MS (Methode 4): Rₜ 4.23 min; m/z 759 (M+NH₄⁺).

### Beispiel 34A

### E-4-[5-[2-(4-tert.-Butylbenzyloxy)phenyl]-3-(3,5-difluor-4-hydroxybenzyl)pent-4-enyl]benzoesäuremethylester

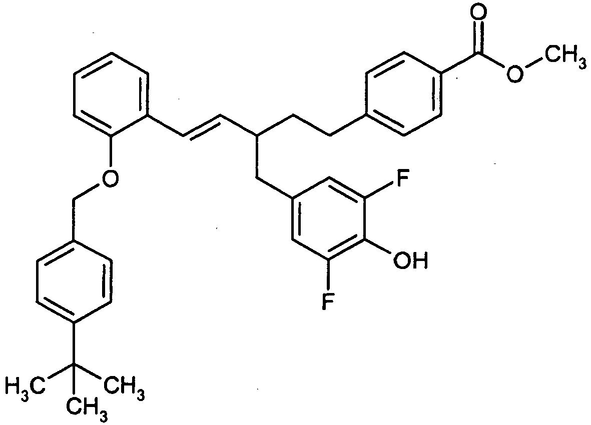

780 mg (1.05 mmol) *E*-4-[5-[2-(4-*tert*.-Butylbenzyloxy)phenyl]-3-(3,5-difluor-4-triisopropyl-silanyloxy-benzyl)pent-4-enyl]benzoesäuremethylester werden in 20 ml THF bei Raumtemperatur unter Argon vorgelegt, mit 2.11 ml (2.11 mmol) einer 1 M Lösung von Tetra-n-butylammoniumfluorid in THF versetzt und die Mischung bei Raumtemperatur bis zur vollständigen Umsetzung nachgerührt. Alle flüchtigen Komponenten werden dann im Vakuum entfernt. Das erhaltene Rohprodukt wird mittels präparativer HPLC gereinigt. Es werden 229 mg (37% d. Th.) der Titelverbindung erhalten.

¹H-NMR (400 MHz, DMSO-d₆, δ/ppm): 9.79 (1H, s), 7.83 (2H, d), 7.41 (1H, d), 7.39-7.24 (6H, m), 7.19 (1H, t), 7.06 (1H, d), 6.98-6.87 (1H, m), 6.81 (2H, d), 6.52 (1H, d), 6.14-6.02 (1H, m), 5.08 (2H, s), 3.82 (3H, s), 2.79-2.52 (4H, m), 2.47-2.35 (1H, m), 1.82-1.69 (1H, m), 1.68-1.54 (1H, m), 1.24 (9H, s).

MS (EI): 607 (M+Na⁺).

### Beispiel 35A

### 2-[2-(4-Cyanophenyl)ethyl]malonsäurediallylester

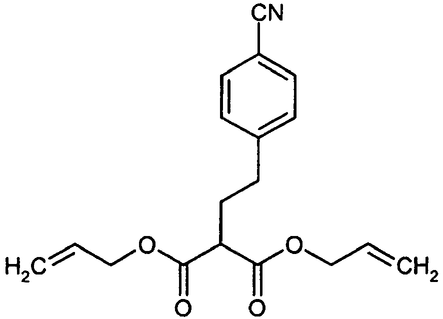

Eine Lösung von 34.35 g (186.51 mmol) Malonsäurediallylester in 300 ml Dioxan wird bei 0°C portionsweise mit 5.59 g (139.88 mmol) 60%-igem Natriumhydrid versetzt (Vorsicht: Wasserstoffentwicklung). Nach Erwärmen auf Raumtemperatur wird der Ansatz 30 min bei 40°C nachgerührt. Anschließend werden 19.59 g (93.25 mol) 4-(2-Bromethyl)benzonitril, gelöst in 200 ml Dioxan, langsam bei 40°C zugetropft und die Reaktionslösung dann über Nacht bei 110°C gerührt. Nach Abkühlen auf Raumtemperatur wird das Reaktionsgemisch auf 200 ml gesättigte Ammoniumchlorid-Lösung gegeben. Der Ansatz wird dann dreimal mit Essigsäureethylester extrahiert, die vereinigten organischen Phasen mit gesättigter Natriumchlorid-Lösung gewaschen und über Natriumsulfat getrocknet. Nach Filtration wird das Lösungsmittel im Vakuum bis zur Trockene eingeengt. Anschließend wird durch Hochvakuumdestillation überschüssiger Malonsäurediallyl-ester entfernt (Siedepunkt: 57°C, 0.074 mbar). Der Destillationsrückstand wird durch Flash-Chromatographie an Kieselgel (Laufmittel: Cyclohexan/Essigsäureethylester 9:1 → 4:1) gereinigt. Es werden 14.18 g (24% d. Th.) eines farblosen Feststoffs erhalten.

¹H-NMR (300 MHz, DMSO-d₆, δ/ppm): 7.77 (2H, d), 7.41 (2H, d), 5.98-5.80 (2H, m), 5.39-5.16 (4H, m), 4.70-4.51 (4H, m), 3.59 (1H, t), 2.70 (2H, t), 2.18-2.02 (2H, m).

MS (DCI): 331 (M+NH₄⁺).

### Beispiel 36A

### 2-[2-(4-Cyanophenyl)ethyl]-2-(3,5-difluor-4-triisopropylsilanyloxy-benzyl)malonsäurediallylester

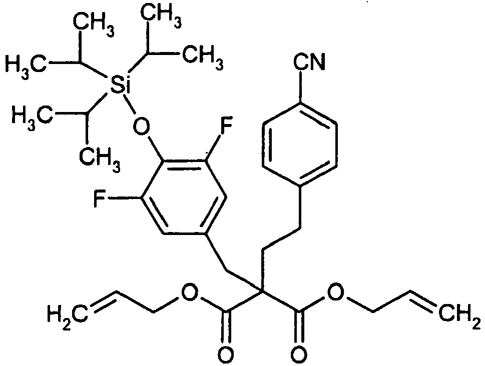

10 g (31.91 mmol) 2-[2-(4-Cyanophenyl)ethyl]malonsäurediallylester werden in 250 ml Dioxan gelöst, bei 0°C portionsweise mit 1.4 g (35.1 mmol) 60%-igem Natriumhydrid versetzt und die Mischung 30 min bei Raumtemperatur nachgerührt. Nach erneutem Abkühlen auf 0°C wird eine Lösung von 14.53 g (38.30 mmol) 3,5-Difluor-4-triisopropylsilanyloxy-benzylbromid in 250 ml Dioxan zugetropft und unter Erwärmung auf Raumtemperatur 2 h nachgerührt. Anschließend wird die Reaktionslösung auf 500 ml gesättigte Ammoniumchlorid-Lösung gegeben und mit Essigsäureethylester extrahiert. Die vereinigten organischen Phasen werden über Natriumsulfat getrocknet und eingeengt. Das erhaltene Rohprodukt wird durch Flash-Chromatographie an Kieselgel (Laufmittel: Cyclohexan/Essigsäureethylester 10:1 → 4:1) gereinigt. Es werden 14.3 g (73% d. Th.) der Titelverbindung erhalten.

¹H-NMR (400 MHz, DMSO-d₆, δ/ppm): 7.76 (2H, d), 7.37 (2H, d), 6.91 (2H, d), 5.98-5.82 (2H, m), 5.38-5.20 (4H, m), 4.68-4.56 (4H, m), 3.24 (2H, s), 2.74-2.61 (2H, m), 1.98-1.84 (2H, m), 1.29-1.13 (3H, m), 1.04 (18H, d).

MS (EI): 612 (M+H⁺), 634 (M+Na⁺).

### Beispiel 37A

### 4-(4-Cyanophenyl)-2-(3,5-difluor-4-triisopropylsilanyloxy-benzyl)butansäure

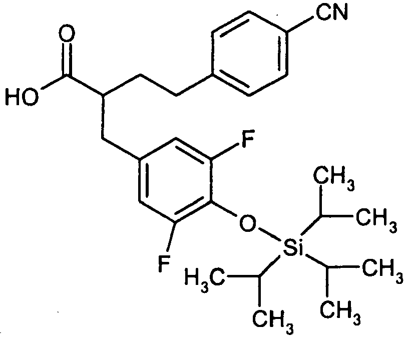

4720 mg (7.72 mmol) 2-[2-(4-Cyanophenyl)ethyl]-2-(3,5-difluor-4-triisopropylsilanyloxy-benzyl)-malonsäurediallylester, 34.6 mg (0.15 mmol) Panadium(II)acetat und 141.6 mg (0.54 mmol) Triphenylphosphin werden in 180 ml Dioxan unter Argon gerührt. Anschließend wird eine Lösung von 2576 mg (25.60 mmol) Triethylamin und 887.7 mg (19.29 mmol) Ameisensäure in 90 ml Dioxan zugetropft und die Mischung 2 h auf 100°C erwärmt. Der abgekühlte Ansatz wird eingeengt und das Rohprodukt an Kieselgel chromatographiert (Laufmittel: Cyclohexan/Essigsäureethylester 9:1 → 2:1 → 1:1). Es werden 3682 mg (97.9% d. Th.) der Titelverbindung erhalten.

¹H-NMR (400 MHz, DMSO-d₆, δ/ppm): 12.39-12.28 (1H, breit), 7.72 (2H, d), 7.35 (2H, d), 6.92 (2H, d), 2.84-2.59 (5H, m), 1.85-1.72 (1H, m), 1.71-1.60 (1H, m), 1.28-1.15 (3H, m), 1.04 (18H, d).

LC-MS (Methode 1): Rₜ 3.48 min; m/z 478 (M-H⁻).

### Beispiel 38A

### 4-[3-(3,5-Difluor-4-triisopropylsilanyloxy-benzyl)-4-hydroxybutyl]benzonitril

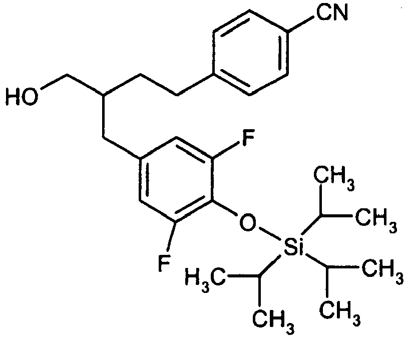

4879 mg (10 mmol) 4-(4-Cyanophenyl)-2-(3,5-difluor-4-triisopropylsilanyloxy-benzyl)butansäure werden in 244 ml THF gelöst und unter Argon auf -10°C gekühlt. Anschließend werden 20.01 ml (20.01 mmol) einer 1 M Boran-THF-Komplex-Lösung zugetropft und die Mischung bei 0°C bis zur vollständigen Umsetzung nachgerührt. Der Ansatz wird mit gesättigter Ammoniumchlorid-Lösung hydrolysiert, mit Diethylether extrahiert und die vereinigten organischen Phasen eingeengt. Das erhaltene Rohprodukt wird chromatographisch an Kieselgel (Laufmittel: Cyclohexan/Essigsäureethylester 9:1 → 4:1 → 2:1 → 1:1) gereinigt. Es werden 2705 mg (57% d. Th.) der Titelverbindung erhalten.

¹H-NMR (400 MHz, DMSO-d₆, δ/ppm): 7.71 (2H, d), 7.34 (2H, d), 6.89 (2H, d), 4.54 (1H, t), 2.75-2.44 (6H, m), 1.72-1.51 (2H, m), 1.49-1.35 (1H, m), 1.30-1.15 (3H, m), 1.04 (18H, d).

LC-MS (Methode 2): Rₜ 3.38 min; m/z 518 (M-H+HCO₂H)⁻, 316 (M-SiC₉H₂₁).

### Beispiel 39A

### 4-[3-(3,5-Difluor-4-triisopropylsilanyloxy-benzyl)-4-oxo-butyl]benzonitril

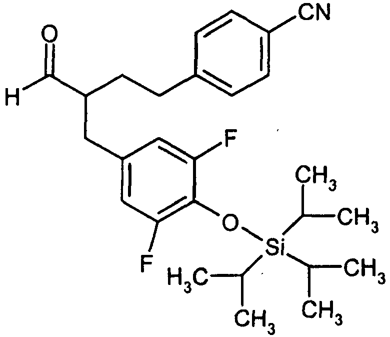

Eine Lösung von 750 mg (1.58 mmol) 4-[3-(3,5-Difluor-4-triisopropylsilanyloxy-benzyl)-4-hydroxybutyl]benzonitril in 9 ml Dichlormethan wird mit 409 mg (1.9 mmol) Pyridiniumchloro-chromat (PCC) versetzt und 12 h bei Raumtemperatur gerührt. Nach vollständigem Umsatz werden ca. 10 g Kieselgel zugegeben und das Lösungsmittel im Vakuum bis zur Trockene entfernt. Der Rückstand wird durch Flash-Chromatographie an Kieselgel (Laufmittel: Cyclohexan/Essigsäure-ethylester 10:1 → 5:1 → 2:1) gereinigt. Es werden 493 mg (64% d. Th.) eines farblosen Feststoffs erhalten.

¹H-NMR (300 MHz, DMSO-d₆, δ/ppm): 9.63 (1H, s), 7.72 (2H, d), 7.35 (2H, d), 6.98 (2H, d), 2.99-2.84 (1H, m), 2.76-2.55 (4H, m), 1.91-1.78 (1H, m), 1.71-1.57 (1H, m), 1.29-1.15 (3H, m), 1.04 (18H, d).

LC-MS (Methode 2): Rₜ 3.38 min; m/z 472 (M+H⁺).

### Beispiel 40A

### E-4-[3-(3,5-Difluor-4-triisopropylsilanyloxy-benzyl)-5-(2-hydroxyphenyl)pent-4-enyl]benzonitril

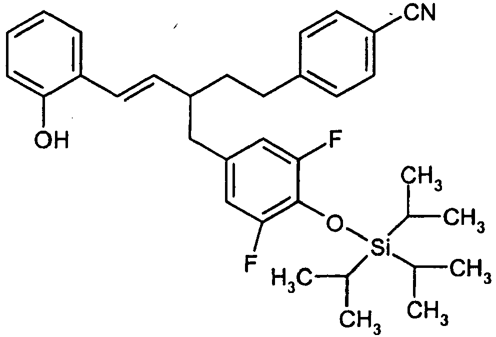

Zu einer Lösung von 579 mg (1.25 mmol) (2-Hydroxybenzyl)-triphenylphosphoniumbromid in 11.5 ml THF werden bei 0°C 1.82 ml (2.91 mmol) einer 1.6 M Lösung von n-Butyllithium in Hexan langsam hinzugefügt. Anschließend werden bei dieser Temperatur 490 mg (1.04 mmol) 4-[3-(3,5-Difluor-4-triisopropylsilanyloxy-benzyl)-4-oxobutyl]benzonitril langsam zudosiert. Die Reaktionslösung wird 2 h bei 0°C nachgerührt und dann mit 100 ml gesättigter Ammoniumchlorid-Lösung versetzt und mit Diethylether extrahiert. Die vereinigten organischen Phasen werden mit gesättigter Natriumchlorid-Lösung gewaschen und über Natriumsulfat getrocknet. Nach Filtration wird das Lösungsmittel bis zur Trockene eingeengt. Das erhaltene Rohprodukt wird durch Flash-Chromatographie an Kieselgel (Laufmittel: Cyclohexan/Essigsäureethylester 10:1) gereinigt. Es werden 379.7 mg (54% d. Th.) eines farblosen Feststoffes erhalten.

¹H-NMR (400 MHz, DMSO-d₆, δ/ppm): 9.42 (1H, s), 7.71 (2H, d), 7.35 (2H, d), 7.29 (2H, d), 7.05 (1H, m), 6.91 (2H, d), 6.79 (1H, d), 6.72 (1H, t), 6.44 (1H, d), 6.08-5.96 (1H, m), 2.79-2.65 (2H, m), 2.64-2.56 (2H, m), 2.47-2.36 (1H, m), 1.78-1.53 (2H, m), 1.29-1.10 (3H, m), 1.00 (18H, d).

MS (DCI): 579 (M+NH₄⁺).

### Beispiel 41A

### E-4-[5-[2-(4-tert.-Butylbenzyloxy)phenyl]-3-(3,5-difluor-4-triisopropylsilanyloxy-benzyl)pent-4-enyl]benzonitril

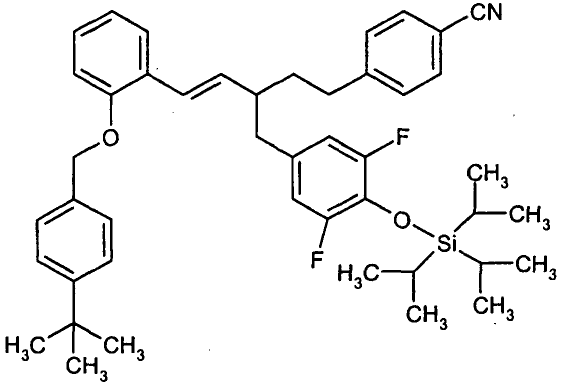

1.7 g (3 mmol) 4-[(4*E*)-3-{3,5-Difluor-4-[(triisopropylsilyl)oxy]benzyl}-5-(2-hydroxyphenyl)pent-4-en-1-yl]benzonitril in 35 ml Acetonitril werden mit 627 mg (4.5 mmol) Kaliumcarbonat und 1 g (4.5 mmol) 4-tert.-Butylbenzylbromid versetzt und 12 Stunden unter Rückfluss gerührt. Das Lösungsmittel wird im Vakuum abgezogen und der Rückstand in Essigsäureethylester aufgenommen. Die organische Phase wird einmal mit Natriumhydrogencarbonat-Lösung und einmal mit Natriumchlorid-Lösung gewaschen, über Natriumsulfat getrocknet und eingeengt. Das Rohprodukt wird mittels Flash-Chromatographie an Kieselgel gereinigt (Laufmittel: Cyclohexan/Essigsäure-ethylester 10:1). Es werden 628 mg (85% Reinheit, 0.76 mmol, 25% d. Th.) der Titelverbindung erhalten.

HPLC (Methode 3): Rₜ = 9.11 min

MS (ESIpos): m/z = 708 (M+H)⁺

¹H-NMR (400 MHz, DMSO-d₆, δ/ppm): 7.65 (d, 2H), 7.4-7.28 (m, 6H), 7.18 (t, 1H), 7.05 (d, 1H), 6.92-6.82 (m, 2H), 6.46 (d, 1H), 6.05 (dd, 1H), 5.1-5.0 (m, 2H), 2.77-2.5 (m, 4H), 2.46-2.31 (m, 1H), 1.8-1.55 (m, 2H), 1.25 (s, 9H), 1.22-1.1 (m, 3H), 0.98 (d, 18H).

### Beispiel 42A

### E-5-{4-[5-[2-(4-tert.-Butylbenzyloxy)phenyl]-3-(3,5-difluor-4-triisopropylsilanyloxy-benzyl)pent-4-enyl]phenyl}-1H-tetrazol

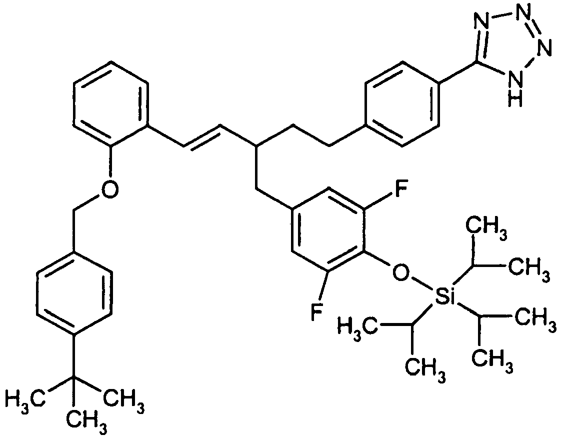

Eine Lösung von 625 mg (85%-ig, 0.75 mmol) E-4-[5-[2-(4-tert.-Butylbenzyloxy)phenyl]-3-(3,5-difluor-4-triisopropylsilanyloxy-benzyl)pent-4-enyl]benzonitril in 12 ml Toluol wird mit 1.76 ml (13.24 mmol) Trimethylsilylazid und 329.6 mg (1.3 mmol) Di-n-butylzinnoxid versetzt und 12 h bei 80°C gerührt. Nach vollständigem Umsatz wird das Gemisch mit Natriumhydrogencarbonat-Lösung ausgeschüttelt. Die wässrige Phase wird zweimal mit Essigsäureethylester rückextrahiert. Die vereinigten organischen Phasen werden mit Natriumchlorid-Lösung gewaschen, über Natriumsulfat getrocknet und eingeengt. Der erhaltene Rückstand wird mittels Flash-Chromatographie an Kieselgel gereinigt (Laufmittel: Cyclohexan/Essigsäureethylester 1:2 → Essigsäureethylester). Es werden 447.3 mg (0.51 mmol, 86% Reinheit, 58% d. Th.) der Titelverbindung erhalten.

HPLC (Methode 3): Rₜ = 7.97 min

MS (ESIpos): m/z = 751 (M+H)⁺

¹H-NMR (400 MHz, DMSO-d₆, δ/ppm): 7.92 (d, 2H), 7.42-7.26 (m, 7H), 7.18 (t, 1H), 7.05 (d, 1H), 6.93-6.84 (m, 3H), 6.5 (d, 1H), 6.08 (dd, 1H), 5.05 (s, 2H), 2.8-2.55 (m, 4H), 2.55-2.4 (m, 1H), 1.84-1.6 (m, 2H), 1.27-1.1 (m, 12H), 1.0 (d, 18H).

### Beispiel 43A

### 4-{[2-Chlormethyl)-4-fluorphenoxy]methyl}-4'-(trifluormethyl)-1,1'-biphenyl

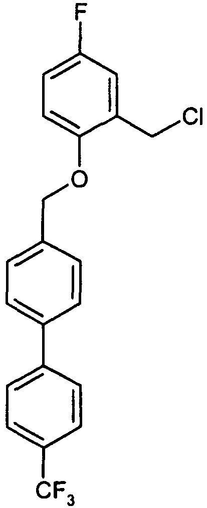

205 g (0.54 mol) (5-Fluor-2-{[4'-trifluormethyl-1,1'-biphenyl-4-yl]methoxy}phenyl]methanol werden in 1322 ml Dichlormethan bei Raumtemperatur unter Argon vorgelegt und mit einigen Tropfen Dimethylformamid versetzt. Anschließend werden 122.5 ml (1.68 mol) Thionylchlorid langsam zugetropft und die Mischung bei Raumtemperatur bis zur vollständigen Umsetzung nachgerührt. Alle flüchtigen Komponenten werden im Vakuum entfernt. Der erhaltene Rückstand wird unter Kühlung in Essigsäureethylester und Wasser aufgenommen. Die organische Phase wird mit gesättigter Natriumcarbonat-Lösung und Wasser gewaschen, über Natriumsulfat getrocknet und das Lösungsmittel im Vakuum entfernt. Es werden 211 g (93% d. Th.) der Titelverbindung erhalten.

¹H-NMR (300 MHz, CDCl₃, δ/ppm): 4.70 (2H, s), 5.20 (2H, s), 6.90 (2H, m), 7.10 (1H, m), 7.50 (2H, d), 7.60 (2H, d), 7.70 (4H, m).

### Beispiel 44A

### (5-Fluor-2-{[4'-(trifluormethyl)-1,1'-biphenyl-4-yl]methoxy}phenyl)acetonitril

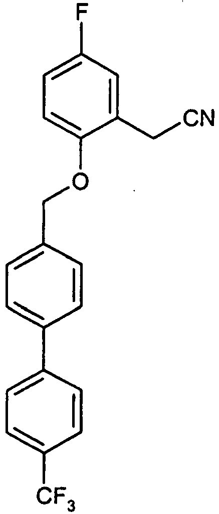

211 g (0.51 mol) 4-{[2-Chlormethyl)-4-fluorphenoxy]methyl}-4'-(trifluormethyl)-1,1'-biphenyl werden in 1.78 Liter Dimethylformamid/Wasser (5:1 v/v) gelöst. Anschließend werden bei Raumtemperatur 149.1 g (3.04 mol) Natriumcyanid und eine katalytische Menge Kaliumiodid zudosiert. Die Reaktionslösung wird über Nacht bei 120°C gerührt. Nach vollständiger Umsetzung (DC-Kontrolle mit Cyclohexan/Essigsäureethylester 2:1) wird die Reaktionslösung auf Raumtemperatur abgekühlt und auf ca. 60% des Lösungsmittelvolumens unter Vakuum eingeengt. Nach Zugabe von 5 Liter Wasser wird die Reaktionslösung dreimal mit jeweils 3 Liter Essigsäureethylester extrahiert. Die vereinigten organischen Phasen werden über Natriumsulfat getrocknet, filtriert und unter Vakuum eingeengt. Der erhaltene Rückstand wird zweimal über 10 kg Kieselgel (Laufmittel: Toluol) chromatographiert. Es werden 145.7 g (69% d. Th.) der Titelverbindung erhalten.

¹H-NMR (300 MHz, CDCl₃, δ/ppm): 3.70 (2H, s), 5.10 (2H, s), 6.90 (1H, m), 7.00 (1H, m), 7.15 (1H, m), 7.50 (2H, d), 7.60 (2H, d), 7.70 (4H, s).

### Beispiel 45A

### 2-(5-Fluor-2-{[4'-(trifluormethyl)-1,1'-biphenyl-4-yl]methoxy}phenyl)ethylamin

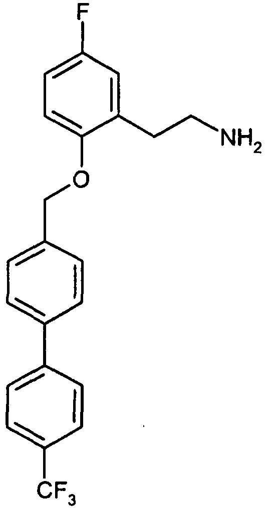

140.97 g (0.366 mol) (5-Fluor-2-{[4'-(trifluormethyl)-1,1'-biphenyl-4-yl]methoxy}phenyl)acetonitril werden unter Argon in 701 ml wasserfreiem THF gelöst, auf 0°C gekühlt und mit 361.6 ml (0.362 mol) einer 1 M Lösung von Lithiumaluminiumhydrid in THF versetzt. Anschließend werden 73.2 g (0.55 mol) Aluminiumtrichlorid in 701 ml THF langsam zu der Reaktionslösung getropft. Das Reaktionsgemisch wird 3.5 h bei Raumtemperatur gerührt. Nach vollständiger Umsetzung wird die Lösung auf 0°C gekühlt und langsam mit wässriger Natriumhydroxid-Lösung versetzt. Die wässrige Phase wird dreimal mit Essigsäureethylester extrahiert, die vereinigten organischen Phasen über Natriumsulfat getrocknet, filtriert und im Vakuum bis zur Trockene eingeengt. Das Rohprodukt wird über 10 kg Kieselgel (Laufmittel: Dichlormethan/Methanol 6:4) chromatographisch gereinigt. Es werden 90.28 g (61 % d. Th.) der Titelverbindung erhalten.

¹H-NMR (300 MHz, CDCl₃, δ/ppm): 1.50 (2H, br. s), 3.00 (4H, m), 5.10 (2H, s), 6.90 (3H, m), 7.50 (2H, d), 7.60 (2H, d), 7.70 (4H, s).

### Beispiel 46A

### 5-{[2-(5-Fluor-2-{[4'-(trifluormethyl)biphenyl-4-yl]methoxy}phenyl)ethyl]amino}pentansäure-ethylester

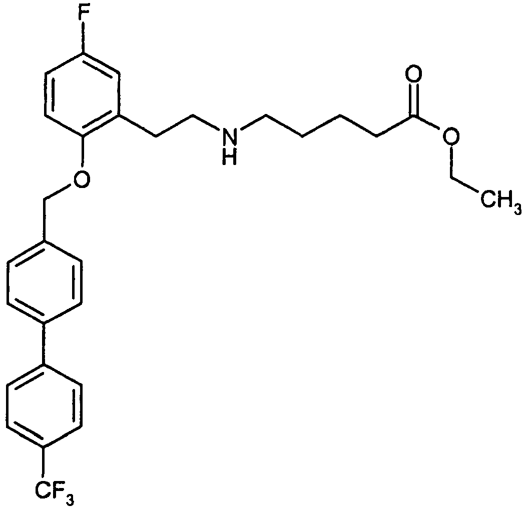

13.8 g (30 mmol) 2-(5-Fluor-2-{[4'-(trifluormethyl)biphenyl-4-yl]methoxy}phenyl)ethylamin-Hydrochlorid [erhältlich durch Aufnehmen der Verbindung aus Beispiel 45A in einer 1 M Lösung von Chlorwasserstoff in Dioxan, Wiedereinengen der Lösung und Trocknen des resultierenden Rückstands] werden in 200 ml DMF vorgelegt, mit 32.4 ml (0.23 mol) Triethylamin versetzt und auf 60-65°C erwärmt. Bei dieser Temperatur werden 9.48 g (0.05 mol) 5-Bromvaleriansäureethylester zugetropft und die Mischung für 12 Stunden gerührt. Nach dem Abkühlen gibt man den Ansatz auf Wasser, stellt mit 1 M Salzsäure auf pH 5 ein und extrahiert mit Essigsäureethylester. Die organische Phase wird über Natriumsulfat getrocknet und das Lösungsmittel im Vakuum entfernt. Der erhaltene Rückstand wird an Kieselgel chromatographisch gereinigt (Laufmittel: Dichlor-methan/Methanol 10:1). Es werden 4.5 g (8.9 mmol, 18% d. Th.) der Titelverbindung erhalten.

LC-MS (Methode 5): Rₜ 2.58 min; m/z 504 (M+H)⁺.

### Beispiel 47A

### 5-{[2-(5-Fluor-2-{[4'-(trifluormethyl)biphenyl-4-yl]methoxy}phenyl)ethyl]amino}pentansäure

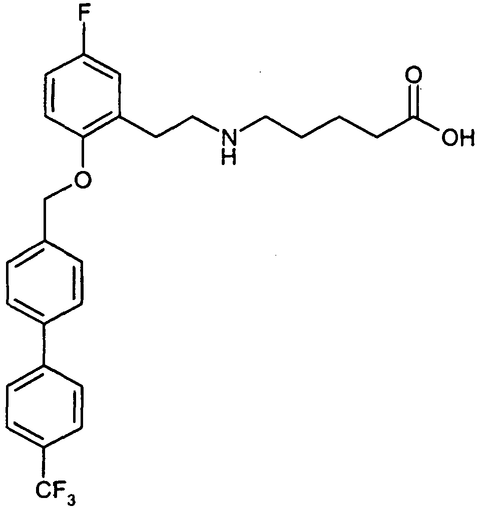

Eine Lösung von 4.5 g (6.93 mmol) 5-{[2-(5-Fluor-2-{[4'-(trifluormethyl)biphenyl-4-yl]methoxy}-phenyl)ethyl]amino}pentansäureethylester in 90 ml THF wird mit 18 ml halbkonzentrierter Salzsäure versetzt und für 20 min auf 100°C erhitzt. Nach dem Abkühlen gibt man den Ansatz auf Wasser und neutralisiert mit gesättigter Natriumhydrogencarbonat-Lösung. Es wird mit Essigsäureethylester extrahiert, die organische Phase über Natriumsulfat getrocknet und das Lösungsmittel im Vakuum entfernt. Der Rückstand wird aus Essigsäureethylester/Diethylether (1:1) kristallisiert. Die erhaltenen Kristalle werden abgesaugt und im Hochvakuum getrocknet. Es werden 2.4 g (4.9 mmol, 71 % d. Th.) der Titelverbindung erhalten.

MS (ESIpos): m/z 490 (M+H)⁺.

### Beispiel 48A

### 2-Allyloxycarbonyl-heptandisäure-1-allylester-7-ethylester

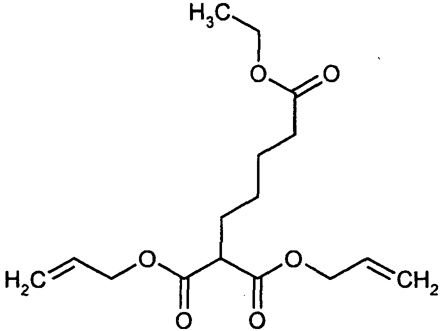

Zu einer Lösung von 100 g (542.92 mmol) Malonsäurediallylester in 900 ml trockenem Dioxan werden bei 5°C 16.29 g (407.19 mmol) Natriumhydrid portionweise zugegeben. Nach Beendigung der Gasentwicklung wird das Reaktionsgemisch auf 40°C erwärmt und 30 min nachgerührt. Anschließend werden 56.76 g (271.46 mmol) 5-Bromvaleriansäureethylester in 100 ml trockenem Dioxan zugetropft und die Mischung 12 Stunden bei 110°C gerührt. Nach Beendigung der Reaktion wird der Ansatz auf Raumtemperatur abgekühlt und auf ca. 400 ml Eiswasser gegeben. Nach Neutralisation des Reaktionsgemisches mit 1 N Salzsäure wird die organische Phase abgetrennt und die wässrige Phase dreimal mit jeweils 250 ml Essigsäureethylester extrahiert. Nach Vereinigung der organischen Phasen wird mit gesättigter Kochsalz-Lösung gewaschen und über Natriumsulfat getrocknet. Nach Filtration wird die Reaktionslösung im Vakuum eingeengt. Darauf folgend wird durch Hochvakuum-Destillation überschüssiger Malonsäurediallylester entfernt (Siedepunkt: 57°C, 0.074 mbar). Der Destillationsrückstand wird durch Flash-Chromatographie an Kieselgel (Laufmittel: Cyclohexan/Essigsäureethylester 2:1) gereinigt. Es werden 73.92 g (236.65 mmol, 44% d. Th.) einer farblosen Flüssigkeit erhalten.

¹H-NMR (400 MHz, DMSO-d₆, δ/ppm): 5.99-5.81 (2H, m), 5.38-5.16 (4H, m), 4.68-4.51 (4H, m), 4.04 (2H, q), 3.59 (1H, t), 2.28 (2H, t), 1.86-1.71 (2H, m), 1.61-1.45 (2H, m), 1.35-1.20 (2H, m), 1.17 (3H, t).

MS (DCI): m/z 330 (M+NH₄⁺).

### Ausführungsbeispiele:

### Beispiel 1

### 6-(3,5-Difluor-4-hydroxybenzyl)-8-[5-fluor-2-(4'-trifluormethyl-biphenyl-4-ylmethoxy)phenyl]oct-7-ensäure

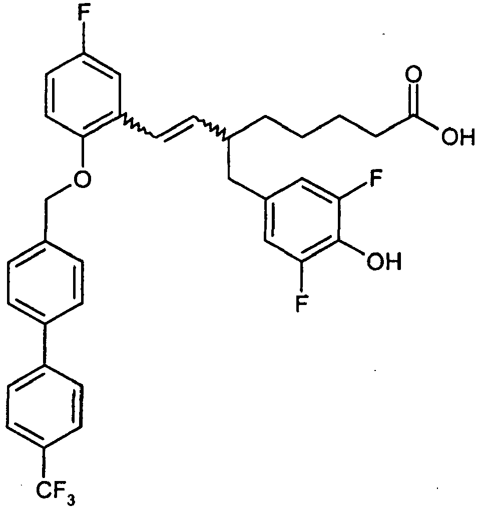

400 mg (0.61 mmol) 6-(3,5-Difluor-4-hydroxybenzyl)-8-[5-fluor-2-(4'-trifluormethyl-biphenyl-4-ylmethoxy)phenyl]oct-7-ensäurethylester werden mit 73 mg (1.83 mmol) Natriumhydroxid in einem Gemisch aus 20 ml THF und 10 ml Wasser über Nacht bei 50°C gerührt. Der abgekühlte Ansatz wird mit verdünnter Salzsäure angesäuert, mit Essigsäureethylester extrahiert, die vereinigten organischen Phasen über Natriumsulfat getrocknet und eingeengt. Es werden 333 mg (86% d. Th.) der Titelverbindung als *E*/*Z*-Gemisch (2:1) erhalten.

¹H-NMR (400 MHz, CDCl₃, δ/ppm): 1.3-1.7 (m, 6H), 2.2-2.8 (m, 5H), 5.0 (s, 2H (*Z*)), 5.05 (s, 2H (*E*)), 5.45 (t, 1H (*Z*)), 5.95 (dd, 1H (E)), 6.55-6.9 (m, 5H), 7.1 (dd, 1H (*E*)), 7.45 (m, 2H), 7.65 (t, 2H), 7.7 (d, 4H).

LC-MS (Methode 3): Rₜ 3.18 min; m/z 627 (M-H)⁻.

Auf analoge Weise werden die folgenden Verbindungen erhalten:

| **Beispiel Nr.** | **Struktur** | **Analytische Daten** |
|---|---|---|
| **2** | 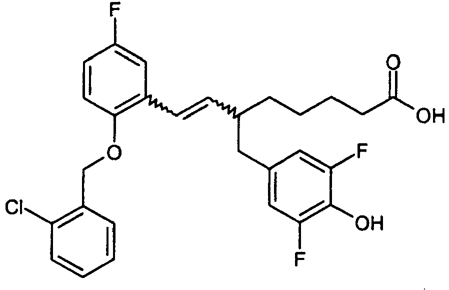 | ¹H-NMR (300 MHz, CDCl₃, δ/ppm): 1.3-1.7 (m, 6H), 2.25 (m, 2H), 2.45 (m, 1H), 2.6 (m, 2H), 5.05 (s, 2H (*Z*)), 5.1 (s, 2H (*E*))*,* 5.45 (t, 1H (*Z*)), 5.95 (dd, 1H (*E*))*,* 6.55 (m, 2H), 6.7 (m, 2H), 6.85 (m, 2H), 7.1 (dd, 1H (*E*))*,* 7.3 (m, 2H), 7.4 (m, 2H). |
| | | LC-MS (Methode 2): Rₜ 2.70 min; m/z 536 (M+NH₄)⁺. |
| 3 | 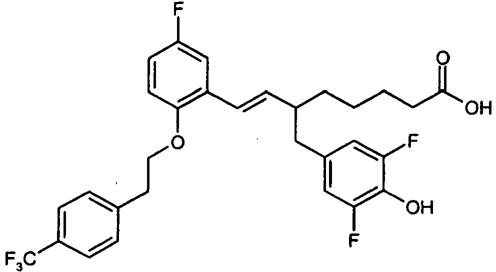 | ¹H-NMR (400 MHz, CDCl₃, δ/ppm): 1.3-1.65 (m, 6H), 2.3 (m, 3H), 2.55 (m, 2H), 3.15 (m, 2H), 4.15 (t, 2H), 5.85 (dd, 1H), 6.35 (d, 1H), 6.7 (m, 2H), 6.75 (m, 1H), 6.85 (m, 1H), 7.05 (dd, 1H), 7.4 (m, 2H), 7.6 (m, 2H). |
| | | LC-MS (Methode 1): Rₜ 2.95 min; m/z 565 (M-H)⁻. |

333 mg (0.53 mmol) *E*/*Z*-6-(3,5-Difluor-4-hydroxybenzyl)-8-[5-fluor-2-(4'-trifluormethyl-biphenyl-4-ylmethoxy)phenyl]oct-7-ensäure (Beispiel 1) werden mittels präparativer HPLC an chiraler Phase weiter aufgetrennt. Es werden jeweils enantiomerenrein 60 mg bzw. 46 mg der beiden E-Isomere sowie 42 mg bzw. 38 mg der beiden *Z*-Isomere als farblose Feststoffe erhalten (siehe Beispiele 4-7).

### Beispiel 4

### E-6-(3,5-Difluor-4-hydroxybenzyl)-8-[5-fluor-2-(4'-trifluormethyl-biphenyl-4-ylmethoxy)phenyl]-oct-7-ensäure (Enantiomer 1)

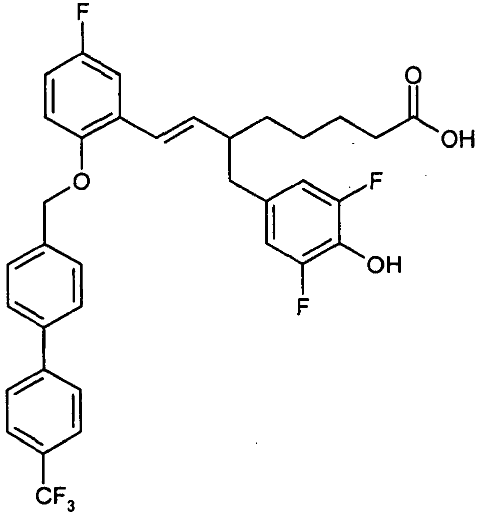

### Methode Enantiomerentrennung:

Säule: Daicel Chiralpak AD-H 250 mm x 20 mm; Elutionsmittel: Isopropanol (mit 1% Wasser und 0.2% Trifluoressigsäure) / Isohexan 30:70 (v/v); Fluss: 15 ml/min; UV-Detektion: 220 nm; Temperatur: 24°C.

Rₜ 8.79 min; Reinheit 99%; >99% ee

Ausbeute: 60 mg

¹H-NMR (400 MHz, CDCl₃, δ/ppm): 1.25-1.7 (m, 6H), 2.3 (t, 2H), 2.4 (m, 1H), 2.6 (ddd, 2H), 5.1 (s, 2H), 5.95 (dd, 1H), 6.6 (d, 1H), 6.7 (d, 2H), 6.85 (m, 2H), 7.1 (dd, 1H), 7.45 (d, 2H), 7.65 (d, 2H), 7.7 (s, 4H).

### Beispiel 5

### E-6-(3,5-Difluor-4-hydroxybenzyl)-8-[5-fluor-2-(4'-trifluormethyl-biphenyl-4-ylmethoxy)phenyl]-oct-7-ensäure (Enantiomer 2)

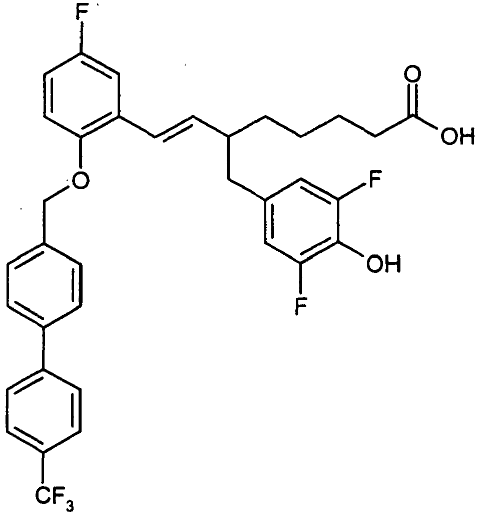

Methode Enantiomerentrennung: siehe Beispiel 4.

Rₜ 9.35 min; Reinheit 99%; >98% ee

Ausbeute: 46 mg

¹H-NMR (400 MHz, CDCl₃, δ/ppm): 1.25-1.7 (m, 6H), 2.3 (t, 2H), 2.4 (m, 1H), 2.6 (ddd, 2H), 5.1 (s, 2H), 5.95 (dd, 1H), 6.6 (d, 1H), 6.7 (d, 2H), 6.85 (m, 2H), 7.1 (dd, 1H), 7.45 (d, 2H), 7.65 (d, 2H), 7.7 (s, 4H).

### Beispiel 6

### Z-6-(3,5-Difluor-4-hydroxybenzyl)-8-[5-fluor-2-(4'-trifluormethyl-biphenyl-4-ylmethoxy)phenyl]-oct-7-ensäure (Enantiomer 1)

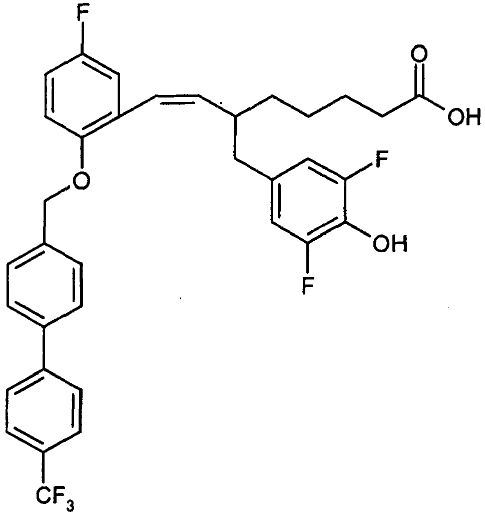

Methode Enantiomerentrennung: siehe Beispiel 4.

Rₜ 6.59 min; Reinheit 100%; 100% ee

Ausbeute: 42 mg

¹H-NMR (400 MHz, CDCl₃, δ/ppm): 1.3 (m, 2H), 1.45 (m, 2H), 1.55 (m, 2H), 2.25 (t, 2H), 2.45 (dd, 1H), 2.65 (dd, 1H), 2.75 (m, 1H), 5.0 (s, 2H), 5.45 (t, 1H), 6.55 (m, 2H), 6.6 (d, 2H), 6.8 (dd, 1H), 6.85 (ddd, 1H), 7.45 (d, 2H), 7.65 (d, 2H), 7.7 (s, 4H).

### Beispiel 7

### Z-6-(3,5-Difluor-4-hydroxybenzyl)-8-[5-fluor-2-(4'-trifluormethyl-biphenyl-4-ylmethoxy)phenyl]-oct-7-ensäure (Enantiomer 2)

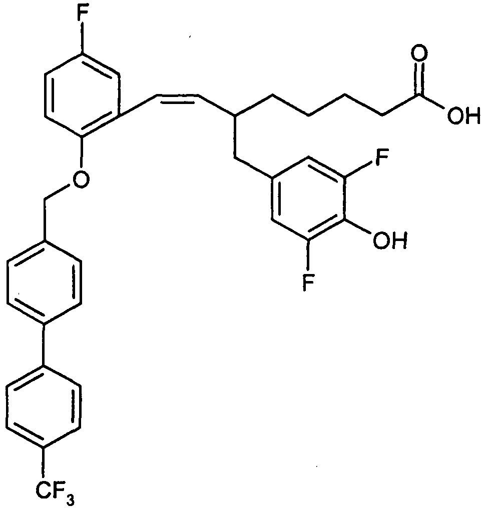

Methode Enantiomerentrennung: siehe Beispiel 4.

Rₜ 7.14 min; Reinheit 99.6%; >99% ee

Ausbeute: 38 mg

¹H-NMR (400 MHz,⁻CDCl₃, δ/ppm): 1.3 (m, 2H), 1.45 (m, 2H), 1.55 (m, 2H), 2.25 (t, 2H), 2.45 (dd, 1H), 2.65 (dd, 1H), 2.75 (m, 1H), 5.0 (s, 2H), 5.45 (t, 1H), 6.55 (m, 2H), 6.6 (d, 2H), 6.8 (dd, 1H), 6.85 (ddd, 1H), 7.45 (d, 2H), 7.65 (d, 2H), 7.7 (s, 4H).

293 mg (0.56 mmol) *E*/*Z*-8-[2-(2-Chlorbenzyloxy)-5-fluorphenyl]-6-(3,5-difluor-4-hydroxybenzyl)-oct-7-ensäure (Beispiel 2) werden mittels präparativer HPLC an chiraler Phase weiter aufgetrennt. Es werden jeweils enantiomerenrein 50 mg bzw. 34 mg der beiden E-Isomere sowie 56 mg bzw. 26 mg der beiden Z-Isomere als farblose Feststoffe erhalten (Beispiele 8-11).

### Beispiel 8

### E-8-[2-(2-Chlorbenzyloxy)-5-fluorphenyl]-6-(3,5-difluor-4-hydroxybenzyl)oct-7-ensäure (Enantiomer 1)

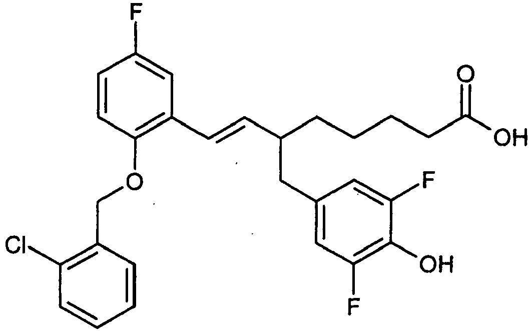

### Methode Enantiomerentrennung:

Säule: Daicel Chiralpak AD-H 250 mm x 20 mm; Elutionsmittel: Isopropanol (mit 1% Wasser und 0.2% Essigsäure) / Isohexan 20:80 (v/v); Fluss: 15 ml/min; UV-Detektion: 220 nm; Temperatur: 25°C.

Rₜ 7.60 min; Reinheit >99%; >99% ee

Ausbeute: 50 mg

¹H-NMR (400 MHz, CDCl₃, δ/ppm): 1.25-1.7 (m, 6H), 2.3 (t, 2H), 2.4 (m, 1H), 2.6 (m, 2H), 5.1 (s, 2H), 5.95 (dd, 1H), 6.6 (d, 1H), 6.7 (d, 2H), 6.85 (m, 2H), 7.1 (dd, 1H), 7.3 (m, 2H), 7.45 (m, 2H).

### Beispiel 9

### E-8-[2-(2-Chlorbenzyloxy)-5-fluorphenyl]-6-(3,5-difluor-4-hydroxybenzyl)oct-7-ensäure (Enantiomer 2)

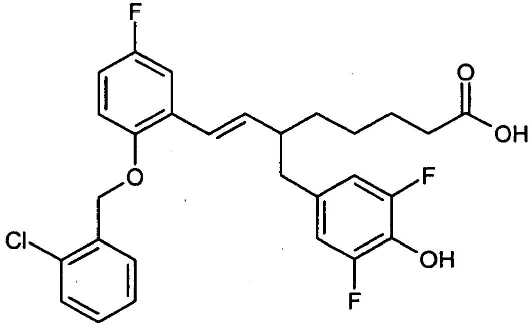

Methode Enantiomerentrennung: siehe Beispiel 8.

Rₜ 8.43 min; Reinheit >99%; >98.5% ee

Ausbeute: 34 mg

¹H-NMR (400 MHz, CDCl₃, δ/ppm): 1.25-1.7 (m, 6H), 2.3 (t, 2H), 2.4 (m, 1H), 2.6 (m, 2H), 5.1 (s, 2H), 5.95 (dd, 1H), 6.6 (d, 1H), 6.7 (d, 2H), 6.85 (m, 2H), 7.1 (dd, 1H), 7.3 (m, 2H), 7.45 (m, 2H).

### Beispiel 10

*Z*-8-[2-(2-Chlorbenzyloxy)-5-fluorphenyl]-6-(3,5-difluor-4-hydroxybenzyl)oct-7-ensäure (*Enantiomer 1*)

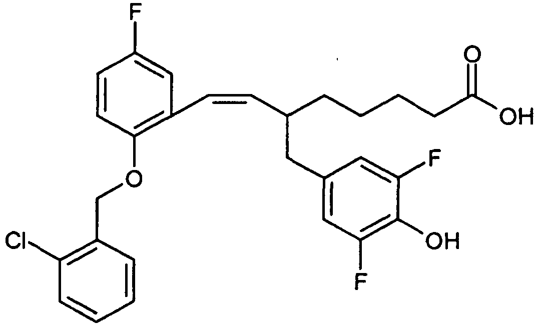

Methode Enantiomerentrennung: siehe Beispiel 8.

Rₜ 6.17 min; Reinheit >99%; >99% ee

Ausbeute: 56 mg

¹H-NMR (400 MHz, CDCl₃, δ/ppm): 1.3 (m, 2H), 1.45 (m, 2H), 1.55 (m, 2H), 2.25 (t, 2H), 2.45 (dd, 1H), 2.65 (dd, 1H), 2.75 (m, 1H), 5.05 (s, 2H), 5.45 (t, 1H), 6.5 (m, 2H), 6.6 (d, 2H), 6.8 (m, 1H), 6.9 (m, 1H), 7.3 (m, 2H), 7.4 (dd, 1H), 7.5 (dd, 1H).

### Beispiel 11

### Z-8-[2-(2-Chlorbenzyloxy)-5-fluorphenyl]-6-(3,5-difluor-4-hydroxybenzyl)oct-7-ensäure (Enantiomer 2)

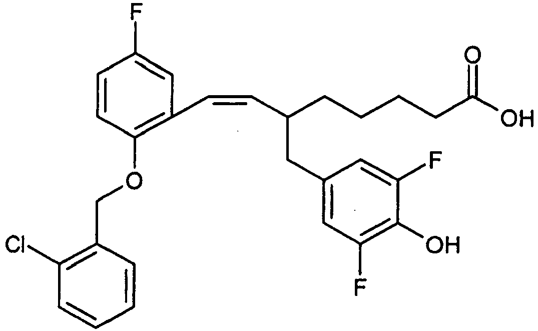

Methode Enantiomerentrennung: siehe Beispiel 8.

Rₜ 7.17 min; Reinheit >99%; >99% ee

Ausbeute: 26 mg

¹H-NMR (400 MHz, CDCl₃, δ/ppm): 1.3 (m, 2H), 1.45 (m, 2H), 1.55 (m, 2H), 2.25 (t, 2H), 2.45 (dd, 1H), 2.65 (dd, 1H), 2.75 (m, 1H), 5.05 (s, 2H), 5.45 (t, 1H), 6.5 (m, 2H), 6.6 (d, 2H), 6.8 (m, 1H), 6.9 (m, 1H), 7.3 (m, 2H), 7.4 (dd, 1H), 7.5 (dd, 1H).

### Beispiel 12

### E-4-[5-[2-(4-tert.-Butylbenzyloxy)phenyl]-3-(3,5-difluor-4-hydroxybenzyl)pent-4-enyl]benzoesäure

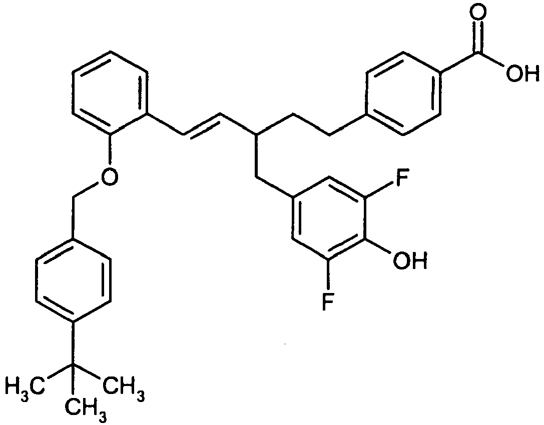

229 mg (0.39 mmol) *E*-4-[5-[2-(4-*tert*.-Butylbenzyloxy)phenyl]-3-(3,5-difluor-4-hydroxybenzyl)-pent-4-enyl]benzoesäuremethylester in 10 ml Methanol werden mit 2 ml 45%-iger Natronlauge über Nacht bei Raumtemperatur gerührt. Nach vollständigem Umsatz wird mit verdünnter Salzsäure angesäuert und mit Essigsäureethylester extrahiert. Die vereinigten organischen Phasen werden über Natriumsulfat getrocknet und eingeengt. Das erhaltene Rohprodukt wird mittels präparativer HPLC gereinigt. Es werden 154 mg (67% d. Th.) der Titelverbindung erhalten.

LC-MS (Methode 2): Rₜ 3.08 min; m/z 569 (M-H)⁻.

154 mg (0.27 mmol) *E*-4-[5-[2-(4-*tert*.-Butylbenzyloxy)phenyl]-3-(3,5-difluor-4-hydroxybenzyl)-pent-4-enyl]benzoesäure werden mittels präparativer HPLC an chiraler Phase weiter aufgetrennt. Es werden jeweils enantiomerenrein 55 mg bzw. 51 mg der beiden E-Isomere als farblose Feststoffe erhalten (siehe Beispiele 13 und 14).

### Beispiel 13

### E-4-[5-[2-(4-tert.-Butylbenzyloxy)phenyl]-3-(3,5-difluor-4-hydroxybenzyl)pent-4-enyl]benzoesäure (Enantiomer 1)

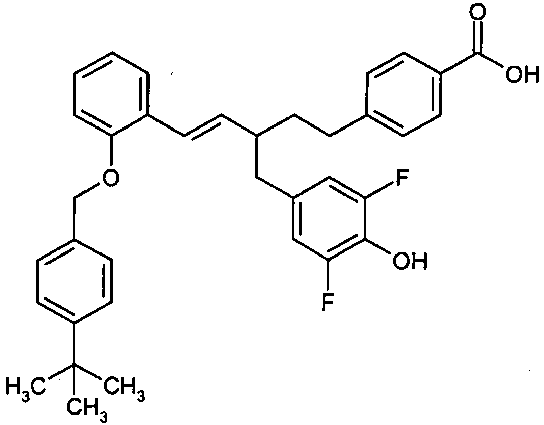

### Methode Enantiomerentrennung:

Säule: Daicel Chiralpak AD-H 250 mm x 20 mm; Elutionsmittel: Isopropanol (mit 1% Wasser und 0.2% Trifluoressigsäure) / Isohexan 30:70 (v/v); Fluss: 15 ml/min; UV-Detektion: 220 nm; Temperatur: 24°C.

Rₜ 5.79 min; Reinheit 99.5%; >99% ee

Ausbeute: 55 mg

¹H-NMR (400 MHz, DMSO-d₆, δ/ppm): 12.82-12.71 (1H, breit), 9.81 (1H, s), 7.82 (2H, d), 7.42 (1H, d), 7.39-7.29 (4H, m), 7.25 (2H, d), 7.19 (1H, t), 7.06 (1H, d), 6.90 (1H, t), 6.82 (2H, d), 6.53 (1H, d), 6.13-6.03 (1H, m), 5.07 (2H, s), 2.77-2.63 (3H, m), 2.62-2.35 (2H, m), 1.80-1.68 (1H, m), 1.67-1.52 (1H, m), 1.24 (9H, s).

MS (EI): 569 (M-H)⁻.

### Beispiel 14

### E-4-[5-[2-(4-tert.-Butylbenzyloxy)phenyl]-3-(3,5-difluor-4-hydroxybenzyl)pent-4-enyl]benzoesäure (Enantiomer 2)

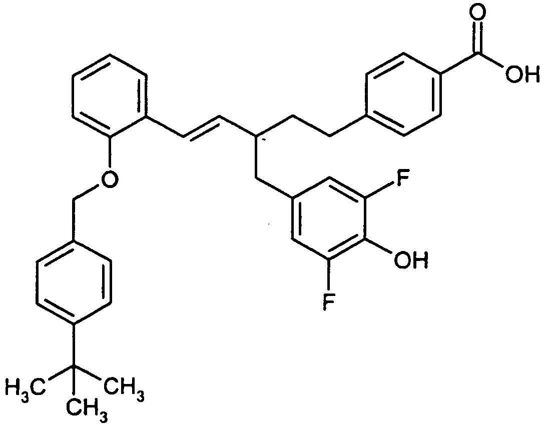

Methode Enantiomerentrennung: siehe Beispiel 13.

Rₜ 6.33 min; Reinheit 99%; >99% ee

Ausbeute: 51 mg

MS (EI): 569 (M-H)⁻.

### Beispiel 15

### E-4-(4-[2-(4-tert.-Butylbenzyloxy)phenyl]-2-{2-[4-(1H-tetrazol-5-yl)phenyl]ethyl}but-3-enyl)-2,6-difluorphenol

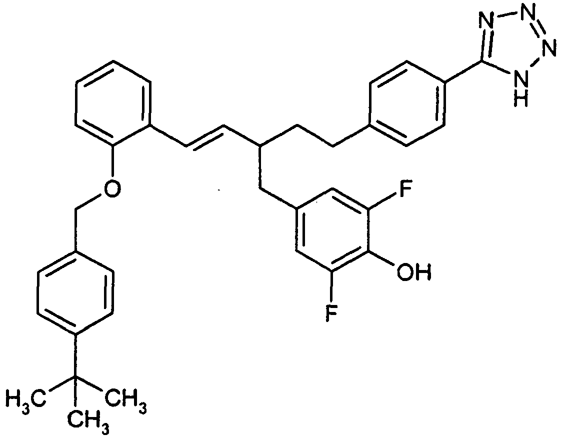

442 mg (0.51 mmol, 86%-ig) *E*-5-{4-[5-[2-(4-*tert*.-Butylbenzyloxy)phenyl]-3-(3,5-difluor-4-triiso-propylsilanyloxy-benzyl)pent-4-enyl]phenyl}-1*H*-tetrazol werden in 12 ml THF vorgelegt, auf 0°C abgekühlt, unter Sauerstoffausschluss mit 1.18 ml (1.18 mmol) einer 1 M Lösung von Tetra-*n-*butylammoniumfluorid in THF versetzt und 2 h bei Raumtemperatur gerührt. Der Ansatz wird anschließend mit Ammoniumchlorid-Lösung versetzt und mit Essigsäureethylester extrahiert. Die organische Phase wird über Natriumsulfat getrocknet und eingeengt. Es werden 297 mg (0.5 mmol, 98% d. Th.) der Titelverbindung erhalten.

LC-MS (Methode 1): Rₜ = 3.3 min

MS (ESIpos): m/z = 595 (M+H)⁺.

297 mg (0.5 mmol) 4-((3*E*)-4-{2-[(4-*tert*.-Butylbenzyl)oxy]phenyl}-2-{2-[4-(1*H*-tetrazol-5-yl)-phenyl]ethyl}but-3-en-1-yl)-2,6-difluorphenol werden mittels präparativer HPLC an chiraler Phase weiter aufgetrennt. Es werden jeweils enantiomerenrein 140 mg bzw. 52 mg der beiden E-Isomere erhalten (siehe Beispiele 16 und 17).

### Beispiel 16

### E-4-(4-[2-(4-tert.-Butylbenzyloxy)phenyl]-2-{2-[4-(1H-tetrazol-5-yl)phenyl]ethyl}but-3-enyl)-2,6-difluorphenol (Enantiomer 1)

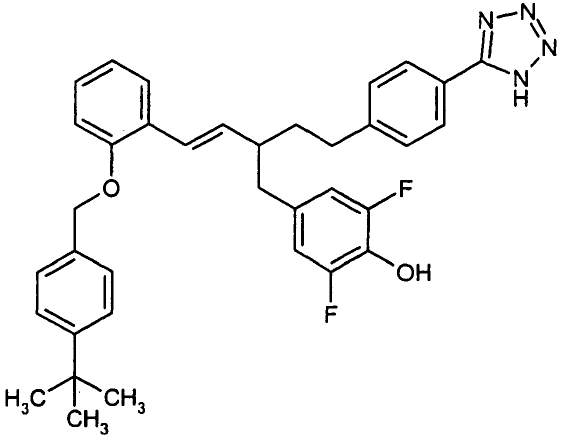

### Methode Enantiomerentrennung:

Säule: Daicel Chiralcel OD-H 250 mm x 20 mm; Elutionsmittel: Isopropanol (mit 1% Wasser und 0.2% Trifluoressigsäure) /Isohexan 30:70 (v/v); Fluss: 15 ml/min; UV-Detektion: 220 nm; Temperatur: 30°C.

Rₜ 5.16 min; Reinheit >80%; >95% ee

Ausbeute: 140 mg

LC-MS (Methode 3): Rₜ = 3.19 min

MS (ESIpos): m/z = 595 (M+H)⁺.

### Beispiel 17

### E-4-(4-[2-(4-tert.-Butylbenzyloxy)phenyl]-2-{2-[4-(1H-tetrazol-5-yl)phenyl]ethyl}but-3-enyl)-2,6-difluorphenol (Enantiomer 2)

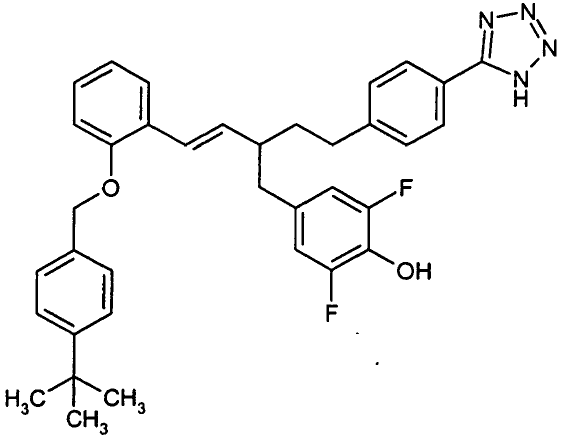

Methode Enantiomerentrennung: siehe Beispiel 16.

Rₜ 5.81 min; Reinheit >80%; >60% ee

Ausbeute: 52 mg.

### Beispiel 18

### 5-{(3,5-Difluor-4-hydroxybenzyl)[2-(5-fluor-2-{[4'-(trifluormethyl)-biphenyl-4-yl]methoxy}-phenyl)ethyl]amino}pentansäure

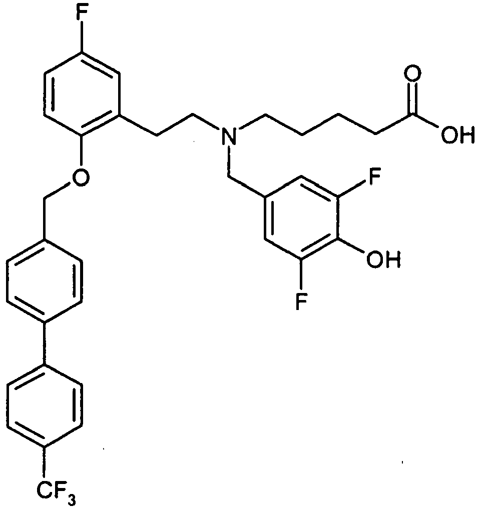

Eine Lösung von 40 mg (0.08 mmol) 5-{[2-(5-Fluor-2-{[4'-(trifluormethyl)biphenyl-4-yl]methoxy}phenyl)ethyl]amino}pentansäure in 5 ml Aceton wird mit 54.5 mg (0.09 mmol) [4-(Brommethyl)-2,6-difluorphenoxy](triisopropyl)silan (Beispiel 16A), 22.6 mg (0.16 mmol) Kaliumcarbonat und 20.3 mg (0.12 mmol) Kaliumiodid versetzt und 12 Stunden unter Rückfluss erhitzt. Nach dem Abkühlen wird der Ansatz mit Wasser versetzt und mit Essigsäureethylester extrahiert. Die organische Phase wird mit gesättigter Natriumchlorid-Lösung gewaschen, über Magnesiumsulfat getrocknet und das Lösungsmittel im Vakuum abdestilliert. Der erhaltene Rückstand wird durch präparative HPLC gereinigt. Es werden 15.3 mg (0.024 mmol, 30% d. Th.) der Titelverbindung erhalten.

¹H-NMR (300 MHz, DMSO-d₆): δ = 7.89 (2H, d), 7.80 (2H, d), 7.73 (2H, d), 7.5 (2H, d), 7.05 (3H, m), 6.82 (2H, d), 5.09 (2H, s), 3.44 (2H, s), 2.77-2.70 (4H, m), 2.65-2.58 (1H, m), 2.43-2.36 (2H, m), 2.29-2.25 (1H, m), 2.12-2.05 (2H, m), 1.45-1.34 (2H, m).

MS (ESIpos): m/z = 632 (M+H)⁺.

### B. Bewertung der pharmakologischen Wirksamkeit

Die pharmakologische Wirkung der erfindungsgemäßen Verbindungen kann in folgenden Assays gezeigt werden:

### B-1. Gefäßrelaxierende Wirkung in vitro:

Kaninchen werden durch intravenöse Injektion von Thiopental-Natrium narkotisiert bzw. getötet (ca. 50 mg/kg) und entblutet. Die Arteria Saphena wird entnommen und in 3 mm breite Ringe geteilt. Die Ringe werden einzeln auf je einem triangelförmigen, am Ende offenen Häkchenpaar aus 0.3 mm starkem Spezialdraht (Remanium^{®}) montiert. Jeder Ring wird unter Vorspannung in 5 ml-Organbäder mit 37°C warmer, carbogenbegaster Krebs-Henseleit-Lösung folgender Zusammensetzung gebracht: NaCl 119 mM; KCl 4.8 mM; CaCl₂ x 2 H₂O 1 mM; MgSO₄ x 7 H₂O 1.4 mM; KH₂PO₄ 1.2 mM; NaHCO₃ 25 mM; Glucose 10 mM; Rinderserumalbumin 0.001%. Die Kontraktionskraft wird mit Statham UC2-Zellen erfasst, verstärkt und über A/D-Wandler (DAS-1802 HC, Keithley Instruments, München) digitalisiert sowie parallel auf Linienschreibern registriert. Kontraktionen werden durch Zugabe von Phenylephrin induziert.

Nach mehreren (allgemein 4) Kontrollzyklen wird die zu untersuchende Substanz in jedem weiteren Durchgang in steigender Dosierung zugesetzt und die Höhe der unter dem Einfluss der Testsubstanz erzielten Kontraktion mit der Höhe der im letzten Vordurchgang erreichten Kontraktion verglichen. Daraus wird die Konzentration errechnet, die erforderlich ist, um die in der Vorkontrolle erreichte Kontraktion auf 50% zu reduzieren (IC₅₀-Wert). Das Standard-Applikationsvolumen beträgt 5 µl. Der DMSO-Anteil in der Badlösung entspricht 0.1%.

Repräsentative Ergebnisse zu den erfindungsgemäßen Verbindungen sind in Tabelle 1 aufgeführt:

**Tabelle 1: Gefäßrelaxierende Wirkung in vitro**

| **Beispiel Nr.** | **IC₅₀ [nM]** |
|---|---|
| 4 | 960 |
| 5 | 300 |
| 6 | 2700 |
| 7 | 2600 |
| 8 | 647 |
| 13 | 24.5 |
| 14 | 1218 |
| 18 | 43 |

### B-2. Stimulation der rekombinanten löslichen Guanylatcyclase (sGC) in vitro:

Die Untersuchungen zur Stimulation der rekombinanten löslichen Guanylatcyclase (sGC) durch die erfindungsgemäßen Verbindungen mit und ohne Natriumnitroprussid sowie mit und ohne den Häm-abhängigen sGC-Inhibitor 1*H*-1,2,4-Oxadiazol-(4,3a)-chinoxalin-1-on (ODQ) werden nach der in folgender Literaturstelle im Detail beschriebenen Methode durchgeführt: M. Hoenicka, E.M. Becker, H. Apeler, T. Sirichoke, H. Schroeder, R. Gerzer und J.-P. Stasch, "Purified soluble guanylyl cyclase expressed in a baculovirus/Sf9 system: Stimulation by YC-1, nitric oxide, and carbon oxide", J. Mol. Med. 77 (1999), 14-23. Die Häm-freie Guanylatcyclase wird durch Zugabe von Tween 20 zum Probenpuffer (0.5% in der Endkonzentration) erhalten.

Die Aktivierung der sGC durch eine Prüfsubstanz wird als n-fache Stimulation der Basalaktivität angegeben. Das Ergebnis für Beispiel 13 ist in Tabelle 2 gezeigt:

**Tabelle 2: Stimulation (n-fach) der rekombinanten löslichen Guanylatcyclase (sGC) in vitro durch Beispiel 13**

| **Konzentration Beispiel 13 [µM]** | **Häm-haltige sGC** | | | **Häm-freie sGC** | |
|---|---|---|---|---|---|
| | **Basal** | **+ 0.1 µM DEA/NO** | + **10 µM ODQ** | **Basal** | **+ 10 µM ODQ** |
| 0.0 | 1.0 | 26.5 | 35.6 | 1.0 | 0.8 |
| 0.001 | 1.7 | 26.3 | 37.5 | 4.1 | 4.0 |
| 0.01 | 6.7 | 31.2 | 38.8 | 30.6 | 27.1 |
| 0.1 | 19.2 | 40.2 | 43.8 | 94.4 | 88.6 |
| 1 | 24.2 | 43.2 | 52.2 | 122.8 | 118.5 |
| 10 | 36.2 | 62.0 | 63.7 | 126.3 | 118.4 |

| | | | | | |
|---|---|---|---|---|---|
| [DEA/NO = 2-(*N,N*-Diethylamino)diazenolat-2-oxid; ODQ = 1*H*-1,2,4-Oxadiazol-(4,3a)-chinoxa-lin-1-on]. | | | | | |

Aus Tabelle 2 ist ersichtlich, dass eine Stimulation sowohl des Häm-haltigen als auch des Hämfreien Enzyms erreicht wird. Weiterhin zeigt die Kombination aus Beispiel 13 und 2-(*N,N-*Diethyl-amino)diazenolat-2-oxid (DEA/NO), einem NO-Donor, keinen synergistischen Effekt, d.h. die Wirkung von DEA/NO wird nicht potenziert, wie dies bei einem über einen Häm-abhängigen Mechanismus wirkenden sGC-Aktivator zu erwarten wäre. Darüber hinaus wird die Wirkung des erfindungsgemäßen sGC-Aktivators durch den Häm-abhängigen Inhibitor der löslichen Guanylatcyclase ODQ nicht blockiert, sondern sogar gesteigert. Die Ergebnisse aus Tabelle 2 belegen somit den Wirkmechanismus der erfindungsgemäßen Verbindungen als Aktivatoren der löslichen Guanylatcyclase.

### B-3. Radiotelemetrische Messung von Blutdruck und Herzfrequenz an wachen SH-Ratten

Für die im Folgenden beschriebenen Messungen an wachen SH-Ratten wird ein im Handel erhältliches Telemetriesystem der Firma Data Sciences International DSI, USA, eingesetzt.

Das System besteht aus 3 Hauptkomponenten: (*1*) Implantierbare Sender, (*2*) Empfänger, die über einen Multiplexer mit einem (*3*) Datenakquisitionscomputer verbunden sind. Die Telemetrieanlage ermöglicht eine kontinuierliche Erfassung von Blutdruck und Herzfrequenz an wachen Tieren in ihrem gewohnten Lebensraum.

Die Untersuchungen werden an ausgewachsenen weiblichen, spontan-hypertensiven Ratten (SH-Ratten) mit einem Körpergewicht von >200 g durchgeführt. Die Versuchstiere werden nach Senderimplantation einzeln in Makrolon-Käfigen Typ 3 gehalten. Sie haben freien Zugang zu Standardfutter und Wasser. Der Tag/Nacht-Rhythmus im Versuchslabor wird per Raumbeleuchtung um 6:00 Uhr morgens und um 19:00 Uhr abends gewechselt.

Die eingesetzten Telemetriesender (TAM PA-C40, DSI) werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobarbital (Nembutal, Sanofi, 50 mg/kg i.p.) narkotisiert und an der Bauchseite weiträumig rasiert und desinfiziert. Nach Eröffnung des Bauchraumes entlang der Linea alba wird der flüssigkeitsgefüllte Messkatheter des Systems oberhalb der Bifurcation nach cranial in die Aorta descendens eingesetzt und mit Gewebekleber (VetBonD™, 3M) befestigt. Das Sendergehäuse wird intraperitoneal an der Bauchwandmuskulatur fixiert und die Wunde schichtweise verschlossen. Postoperativ wird zur Infektionsprophylaxe ein Antibiotikum verabreicht (Tardomyocel COMP, Bayer, 1 ml/kg s.c.).

### Versuchsablauf:

Die zu untersuchenden Substanzen werden jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert. Eine Lösungsmittel-behandelte Gruppe von Tieren wird als Kontrolle eingesetzt.

Die Telemetrie-Messeinrichtung ist für 24 Tiere konfiguriert. Jeder Versuch wird unter einer Versuchsnummer registriert.

Den in der Anlage lebenden instrumentierten Ratten ist jeweils eine eigene Empfangsantenne zugeordnet (1010 Receiver, DSI). Die implantierten Sender sind über einen eingebauten Magnetschalter von außen aktivierbar und werden bei Versuchsvorlauf auf Sendung geschaltet. Die ausgestrahlten Signale können durch ein Datenakquisitionssystem (Dataquest™ A.R.T. for Windows, DSI) online erfasst und entsprechend aufgearbeitet werden. Die Ablage der Daten erfolgt jeweils in einem hierfür eröffneten Ordner, der die Versuchsnummer trägt.

Im Standardablauf werden über je 10 Sekunden Dauer gemessen: (*1*) systolischer Blutdruck (SBP), (*2*) diastolischer Blutdruck (DBP), (*3*) arterieller Mitteldruck (MAP) und (*4*) Herzfrequenz (HR).

Die Messwerterfassung wird rechnergesteuert in 5 Minuten-Abständen wiederholt. Die als Absolutwert erhobenen Quelldaten werden im Diagramm mit dem aktuell gemessenen Barometerdruck korrigiert und in Einzeldaten abgelegt. Weitere technische Details sind in der Dokumentation der Herstellerfirma (DSI) aufgeführt.

Die Verabreichung der Prüfsubstanzen erfolgt am Versuchstag um 9:00 Uhr. Im Anschluss an die Applikation werden die oben beschriebenen Parameter über 24 Stunden gemessen. Nach Versuchsende werden die erhobenen Einzeldaten mit der Analysis-Software (Dataquest™ A.R.T. Analysis) sortiert. Als Leerwert wird der Zeitpunkt 2 Stunden vor Substanz-Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten-Mittelwert, 30 Minuten-Mittelwert) und als Textdatei auf einen Datenträger übertragen. Die so vorsortierten und komprimierten Messwerte werden in Excel-Vorlagen übertragen und tabellarisch dargestellt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 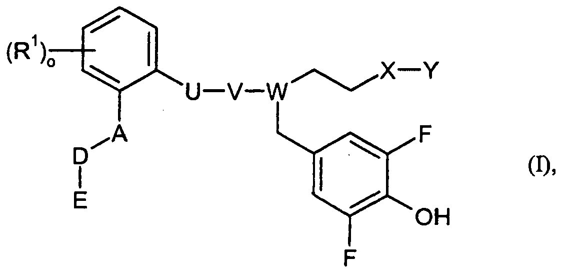 in welcher
U, V und W zusammen eine Gruppe der Formel *-CH=CH-CH<, *-CH₂-CH₂-CH< oder *-CH₂-CH₂-N< bilden, worin * die Verknüpfungsstelle mit dem Phenylring bedeutet,
A für O oder CH₂ steht,
D für eine Bindung oder für (C₁-C₇)-Alkandiyl, (C₂-C₇)-Alkendiyl oder (C₂-C₇)-Alkindiyl, welche jeweils ein- oder mehrfach mit Fluor substituiert sein können, steht,
E für Wasserstoff, Trifluormethyl oder für eine Gruppe der Formel 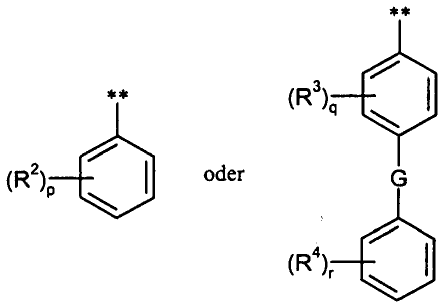 steht, worin ** die Verknüpfungsstelle mit der Gruppe D und
G eine Bindung, CH₂, -CH₂-CH₂- oder -CH=CH- bedeutet,
X für -CH₂-CH₂- oder eine Gruppe der Formel 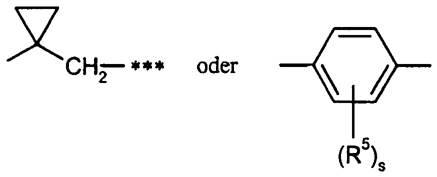 steht, worin *** die Verknüpfungsstelle mit der Gruppe Y bedeutet,
Y für Carboxyl oder eine Gruppe der Formel 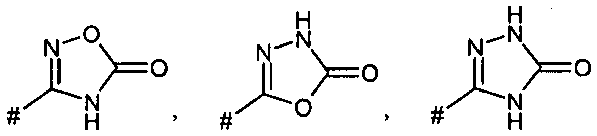 oder 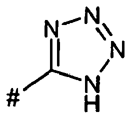 steht, worin # die jeweilige Verknüpfungsstelle bedeutet,
R¹, R², R³, R⁴ und R⁵ unabhängig voneinander für einen Substituenten ausgewählt aus der Reihe Halogen, (C₁-C₆)-Alkyl, Trifluormethyl, (C₁-C₆)-Alkoxy, Trifluormethoxy, Cyano und Nitro stehen,
und
o, p, q, r und s unabhängig voneinander jeweils für die Zahl 0, 1, 2, 3 oder 4 stehen,
wobei für den Fall, dass R¹, R², R³, R⁴ oder R⁵ mehrfach auftreten, ihre Bedeutungen jeweils gleich oder verschieden sein können,
sowie ihre Salze, Solvate und Solvate der Salze.

2. Verbindung der Formel (I) nach Anspruch 1, in welcher
U, V und W zusammen eine Gruppe der Formel *-CH=CH-CH< oder *-CH₂-CH₂-N<
bilden, worin * die Verknüpfungsstelle mit dem Phenylring bedeutet,
A für O steht,
D für (C₁-C₇)-Alkandiyl, welches ein- oder mehrfach mit Fluor substituiert sein kann, steht,
E für Wasserstoff, Trifluormethyl oder für eine Gruppe der Formel 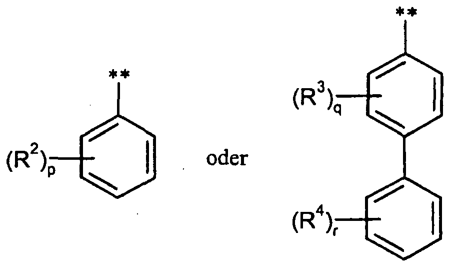 steht, worin ** die Verknüpfungsstelle mit der Gruppe D bedeutet,
X für -CH₂-CH₂- oder eine Gruppe der Formel 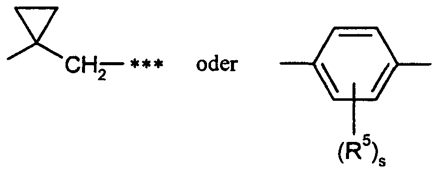 steht, worin *** die Verknüpfungsstelle mit der Gruppe Y bedeutet,
Y für Carboxyl oder eine Gruppe der Formel 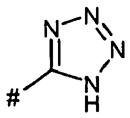 steht, worin # die Verknüpfungsstelle bedeutet,
R¹, R², R³ und R⁴ unabhängig voneinander für einen Substituenten ausgewählt aus der Reihe Fluor, Chlor, Brom, (C₁-C₄)-Alkyl, Trifluormethyl, (C₁-C₄)-Alkoxy und Trifluormethoxy stehen,
o, p, q und r unabhängig voneinander jeweils für die Zahl 0, 1 oder 2 stehen, wobei für den Fall, dass R¹, R², R³ oder R⁴ mehrfach auftreten, ihre Bedeutungen jeweils gleich oder verschieden sein können,
R⁵ für Fluor
und
s für die Zahl 0 oder 1 steht,
sowie ihre Salze, Solvate und Solvate der Salze.

3. Verbindung der Formel (I-A) nach Anspruch 1 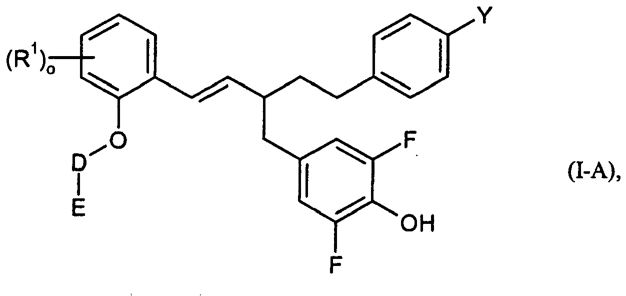 in welcher
D für (C₁-C₇)-Alkandiyl, welches ein- oder mehrfach mit Fluor substituiert sein kann, steht,
E für Wasserstoff, Trifluormethyl oder für eine Gruppe der Formel 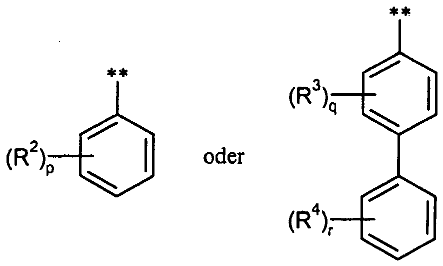 steht, worin ** die Verknüpfungsstelle mit der Gruppe D bedeutet,
Y für Carboxyl oder eine Gruppe der Formel 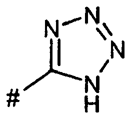 steht, worin # die Verknüpfungsstelle bedeutet,
R¹, R², R³ und R⁴ unabhängig voneinander für einen Substituenten ausgewählt aus der Reihe Fluor, Chlor, Brom, Methyl, *tert*.-Butyl, Trifluormethyl und Methoxy stehen,
und
o, p, q und r unabhängig voneinander jeweils für die Zahl 0, 1 oder 2 stehen, wobei für den Fall, dass R¹, R², R³ oder R⁴ mehrfach auftreten, ihre Bedeutungen jeweils gleich oder verschieden sein können,
sowie ihre Salze, Solvate und Solvate der Salze.

4. Verbindung der Formel (I-B) nach Anspruch 1 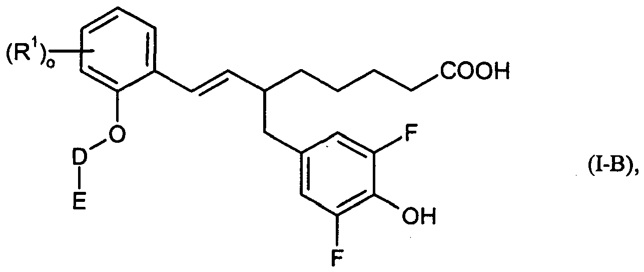 in welcher
D für (C₁-C₇)-Alkandiyl, welches ein- oder mehrfach mit Fluor substituiert sein kann, steht,
E für Wasserstoff, Trifluormethyl oder für eine Gruppe der Formel 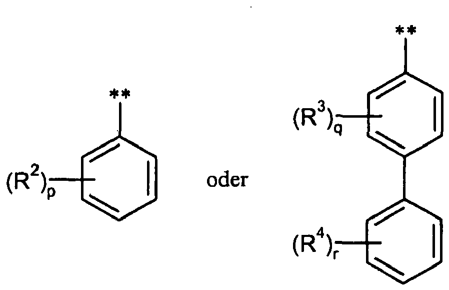 steht, worin ** die Verknüpfungsstelle mit der Gruppe D bedeutet,
R¹, R², R³ und R⁴ unabhängig voneinander für einen Substituenten ausgewählt aus der Reihe Fluor, Chlor, Brom, Methyl, *tert*.-Butyl, Trifluormethyl und Methoxy stehen,
und
o, p, q und r unabhängig voneinander jeweils für die Zahl 0, 1 oder 2 stehen,
wobei für den Fall, dass R¹, R², R³ oder R⁴ mehrfach auftreten, ihre Bedeutungen jeweils gleich oder verschieden sein können, sowie ihre Salze, Solvate und Solvate der Salze.

5. Verfahren zur Herstellung von Verbindungen der Formel (I), (I-A) bzw. (I-B), wie in den Ansprüchen 1 bis 4 definiert, in welchen U, V und W zusammen eine Gruppe der Formel *-CH=CH-CH< bilden, worin * die Verknüpfungsstelle mit dem Phenylring bedeutet, und Y für Carboxyl steht,
**dadurch gekennzeichnet, dass** man Verbindungen der Formel (II) 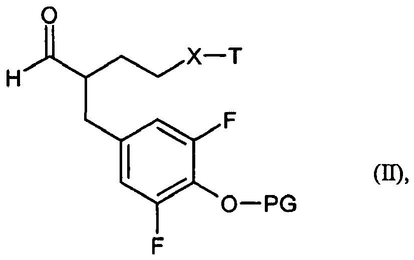 in welcher X die in den Ansprüchen 1 bis 4 angegebene Bedeutung hat und
PG für eine Hydroxy-Schutzgruppe
und
T für Cyano oder (C₁-C₄)-Alkoxycarbonyl steht,
entweder
[A-1] in einem inerten Lösungsmittel in Gegenwart einer Base mit einer Verbindung der
Formel (III-A) 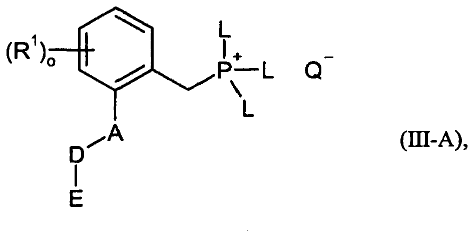 in welcher A, D, E, R¹ und o jeweils die in den Ansprüchen 1 bis 4 angegebenen Bedeutungen haben und
L für Phenyl oder o-, m- oder p-Tolyl
und
Q für Halogenid oder Tosylat steht,
zu Verbindungen der Formel (IV-A) 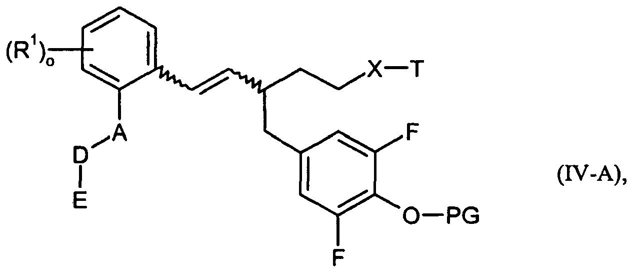 in welcher A, D, E, X, R¹, o, PG und T jeweils die oben angegebenen Bedeutungen haben,
umsetzt
oder
[A-2] in einem inerten Lösungsmittel in Gegenwart einer Base mit einer Verbindung der
Formel (III-B) 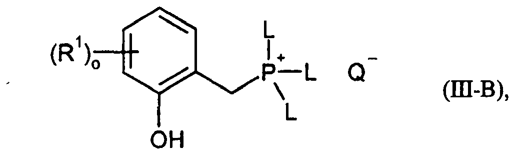 in welcher R¹, o, L und Q jeweils die oben angegebenen Bedeutungen haben, zunächst zu Verbindungen der Formel (IV-B) 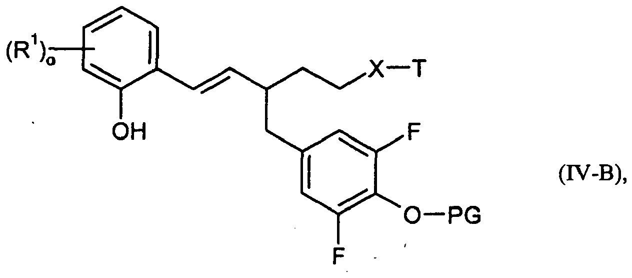 in welcher X, R¹, o, PG und T jeweils die oben angegebenen Bedeutungen haben, umsetzt und diese anschließend in einem inerten Lösungsmittel in Gegenwart einer Base mit einer Verbindung der Formel (V)
E-D*-Z¹ (V),
in welcher E die in den Ansprüchen 1 bis 4 angegebene Bedeutung hat,
D* die in den Ansprüchen 1 bis 4 angegebene Bedeutung von D hat, jedoch nicht für eine Bindung steht,
und
Z¹ für eine Abgangsgruppe, wie beispielsweise Halogen, Tosylat oder Mesylat, steht,
zu Verbindungen der Formel (IV-C) 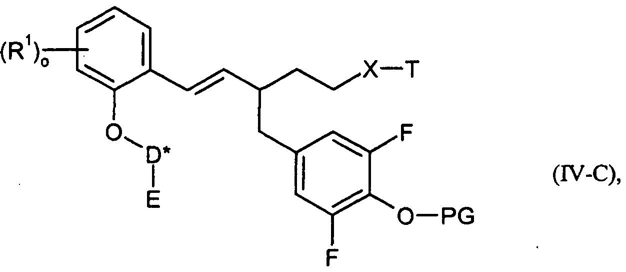 in welcher D*, E, X, R¹, o, PG und T jeweils die oben angegebenen Bedeutungen haben,
alkyliert,
die resultierenden Verbindungen der Formel (IV-A) bzw. (IV-C) dann durch Abspaltung der Schutzgruppe PG in Verbindungen der Formel (VI) 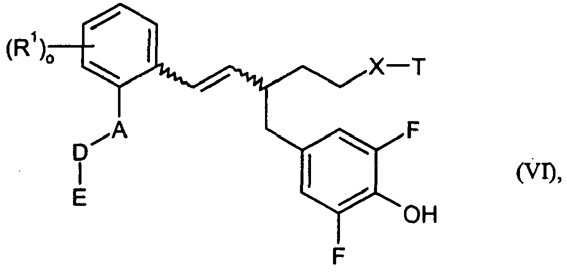 in welcher A, D, E, X, R¹, o und T jeweils die oben angegebenen Bedeutungen haben, überführt und diese durch Hydrolyse der Ester- bzw. Nitril-Gruppe T zu Carbonsäuren der Formel (I-C) 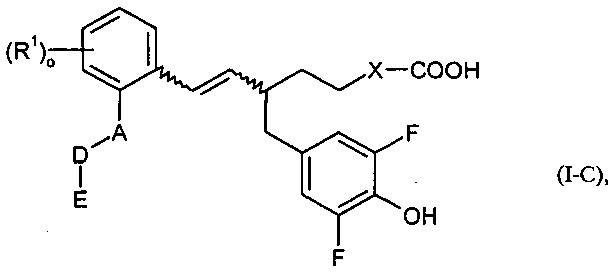 in welcher A, D, E, X, R¹ und o jeweils die oben angegebenen Bedeutungen haben,
umsetzt
und die Verbindungen der Formel (I-C) gegebenenfalls nach dem Fachmann bekannten Methoden in ihre Enantiomere und/oder Diastereomere trennt und/oder gegebenenfalls mit den entsprechenden (i) Lösungsmitteln und/oder (ii) Basen oder Säuren in ihre Solvate, Salze und/oder Solvate der Salze überführt.

6. Verfahren zur Herstellung von Verbindungen der Formel (I), wie in den Ansprüchen 1 und 2 definiert, in welcher U, V und W zusammen eine Gruppe der Formel *-CH₂-CH₂-N< bilden, worin * die Verknüpfungsstelle mit dem Phenylring bedeutet, und Y für Carboxyl steht,
**dadurch gekennzeichnet, dass** man Verbindungen der Formel (XII) 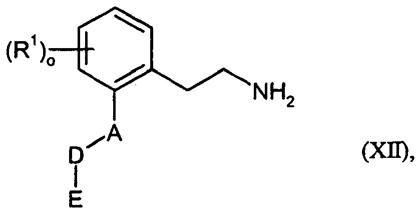 in welcher A, D, E, R¹ und o jeweils die in Anspruch 1 oder 2 angegebenen Bedeutungen haben,
zunächst in einem inerten Lösungsmittel in Gegenwart einer Base mit einer Verbindung der Formel (VII) 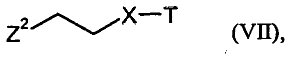 in welcher X die in Anspruch 1 oder 2 angegebene Bedeutung hat und
T für Cyano oder (C₁-C4)-Alkoxycarbonyl
und
Z² für eine Abgangsgruppe, wie beispielsweise Halogen, Mesylat oder Tosylat, steht,
zu Verbindungen der Formel (XIII) 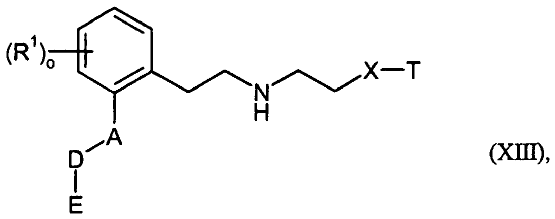 in welcher A, D, E, X, R¹, o und T jeweils die oben angegebenen Bedeutungen haben, alkyliert, anschließend in einem inerten Lösungsmittel in Gegenwart einer Base mit einer
Verbindung der Formel (VIII) 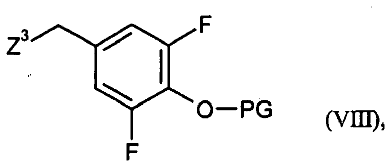 in welcher
PG für eine Hydroxy-Schutzgruppe
und
Z³ für eine Abgangsgruppe, wie beispielsweise Halogen, Mesylat oder Tosylat, steht, zu Verbindungen der Formel (XIV) 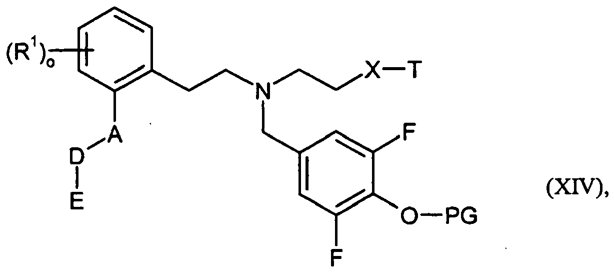 in welcher A, D, E, X, R¹, o, PG und T jeweils die oben angegebenen Bedeutungen haben, umsetzt, dann durch Abspaltung der Schutzgruppe PG in Verbindungen der Formel (XV) 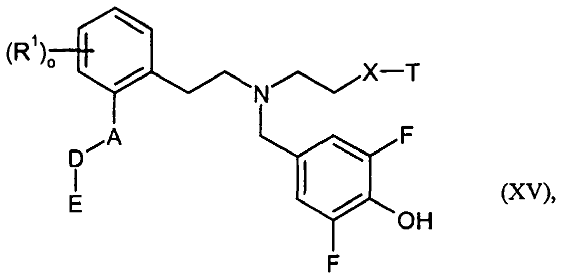 in welcher A, D, E, X, R¹, o und T jeweils die oben angegebenen Bedeutungen haben, überführt und diese durch Hydrolyse der Ester- bzw. Nitril-Gruppe T zu Carbonsäuren der Formel (I-D) 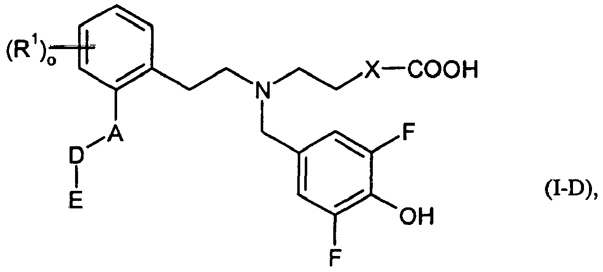 in welcher A, D, E, X, R¹ und o jeweils die oben angegebenen Bedeutungen haben, umsetzt
und die Verbindungen der Formel (I-D) gegebenenfalls mit den entsprechenden (i) Lösungsmitteln und/oder (ii) Basen oder Säuren in ihre Solvate, Salze und/oder Solvate der Salze überführt.

7. Verbindung, wie in einem der Ansprüche 1 bis 4 definiert, zur Behandlung und/oder Prävention von Krankheiten.

8. Verwendung einer Verbindung, wie in einem der Ansprüche 1 bis 4 definiert, zur Herstellung eines Arzneimittels zur Behandlung und/oder Prävention von Herzinsuffizienz, Angina pectoris, Hypertonie, pulmonaler Hypertonie, Ischämien, Gefäßerkrankungen, thromboembolischen Erkrankungen und Arteriosklerose.

9. Arzneimittel enthaltend eine Verbindung, wie in einem der Ansprüche 1 bis 4 definiert, in Kombination mit einem inerten, nicht-toxischen, pharmazeutisch geeigneten Hilfsstoff.

10. Arzneimittel enthaltend eine Verbindung, wie in einem der Ansprüche 1 bis 4 definiert, in Kombination mit einem weiteren Wirkstoff ausgewählt aus der Gruppe bestehend aus organischen Nitraten, NO-Donatoren, cGMP-PDE-Inhibitoren, Stimulatoren der Guanylatcyclase, antithrombotisch wirkenden Mitteln, den Blutdruck senkenden Mitteln sowie den Fettstoffwechsel verändernden Mitteln.

11. Arzneimittel nach Anspruch 9 oder 10 zur Behandlung und/oder Prävention von Herzinsuffizienz, Angina pectoris, Hypertonie, pulmonaler Hypertonie, Ischämien, Gefäßerkrankungen, thromboembolischen Erkrankungen und Arteriosklerose.

## Claims

1. Compound of the formula (I) 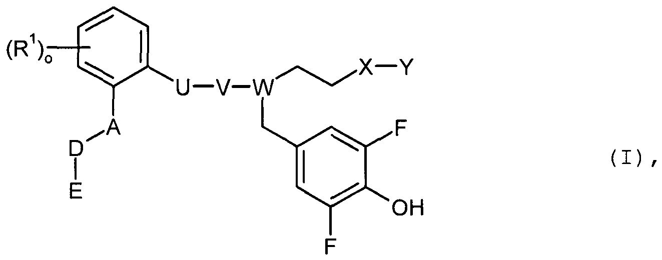 in which
U, V and W together form a group of the formula *-CH=CH-CH<, *-CH₂-CH₂-CH< or *-CH₂-CH₂-N<, in which * means the point of linkage to the phenyl ring,
A is O or CH₂,
D is a bond or is (C₁-C₇)-alkanediyl, (C₂-C₇)-alkenediyl or (C₂-C₇)-alkynediyl, each of which may be substituted one or more times by fluorine,
E is hydrogen, trifluoromethyl or a group of the formula 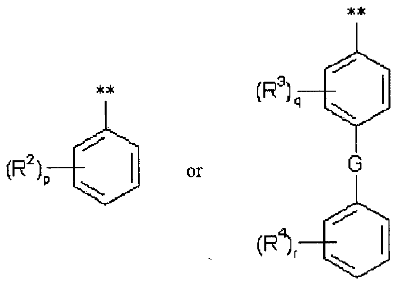 in which ** means the point of linkage to the group D and
G is a bond, CH₂, -CH₂-CH₂- or -CH=CH-,
X is -CH₂-CH₂- or a group of the formula 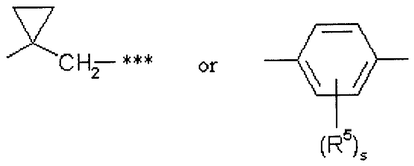 the in which *** means the point of linkage to group Y,
Y is carboxyl or a group of the formula 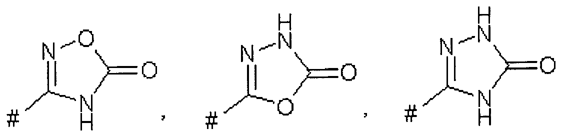 or 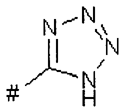 in which # means the respective point of linkage,
R¹, R², R³ und R⁴ are independently of one another substituents selected from the series halogen, (C₁-C₆)-alkyl, trifluoromethyl, (C₁-C₆)-alkoxy, trifluoromethoxy, cyano and nitro,
and
o, p, q, r and s are independently of one another each the number 0, 1, 2, 3 or 4.
where in the case where R¹, R², R³, R⁴ or R⁵occur more than once, their meanings may in each case be identical or different,
the salts, solvates and solvates of the salts thereof.

2. Compound of the formula (I) according to Claim 1, in which
U, V and W together form a group of the formula *-CH=CH-CH< or *-CH₂-CH₂-N< in which * means the point of linkage to the phenyl ring,
A is O,
D is (C₁-C₇)-alkanediyl which may be substituted one or more times by fluorine,
E is hydrogen, trifluoromethyl or is a group of the formula 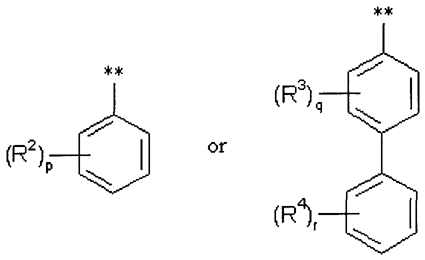 in which ** means the point of linkage to the group D,
X is -CH₂-CH₂- or a group of the formula 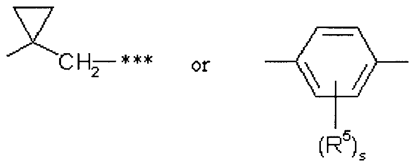 the in which *** means the point of linkage to group Y,
Y is carboxyl or a group of the formula 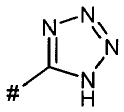 in which # means the respective point of linkage,
R¹, R², R³ and R⁴ are independently of one another a substituent selected from the series fluorine, chlorine, bromine, (C₁-C₄) -alkyl, trifluoromethyl, (C₁-C₄)-alkoxy and trifluoromethoxy,
o, p, q and r are independently of one another each the number 0, 1 or 2, where in the case where R¹, R², R³ or R⁴ occur more than once, their meanings may in each case be identical or different,
R⁵ is fluorine,
and
s is the number 0 or 1,
and the salts, solvates and solvates of the salts thereof.

3. Compound of the formula (I-A) according to Claim 1 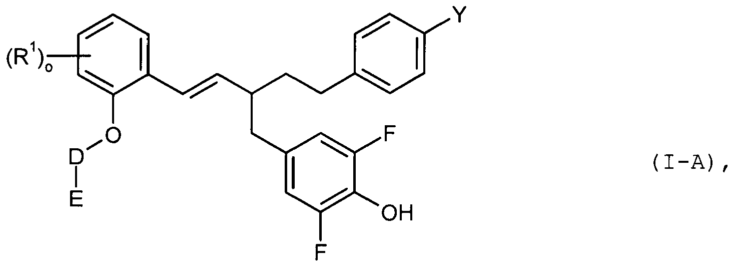 in which
D is (C₁-C₇)-alkanediyl which may be substituted one or more times by fluorine,
E is hydrogen, trifluoromethyl or is a group of the formula 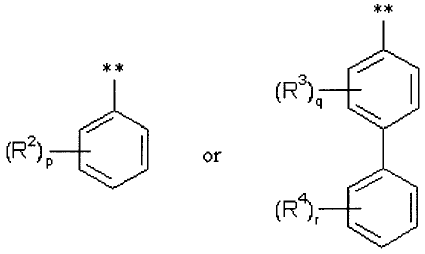 in which ** means the point of linkage to the group D,
Y is carboxyl or a group of the formula 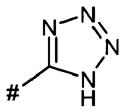 in which # means the point of linkage,
R¹, R², R³ and R⁴ are independently of one another a substituent selected from the series fluorine, chlorine, bromine, methyl, tert-butyl, trifluoromethyl and methoxy,
and
o, p, q and r are independently of one another each the number 0, 1 or 2, where in the case that R¹, R², R³ or R⁴ occur more than once, their meanings may in each case be identical or different,
and the salts, solvates and solvates of the salts thereof.

4. Compound of the formula (I-B) according to Claim 1 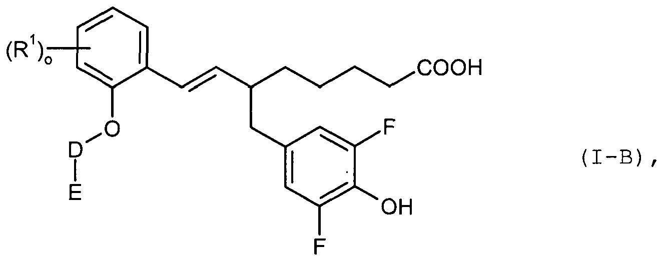 in which
D is (C₁-C₇)-alkanediyl which may be substituted one or more times by fluorine,
E is hydrogen, trifluoromethyl or is a group of the formula 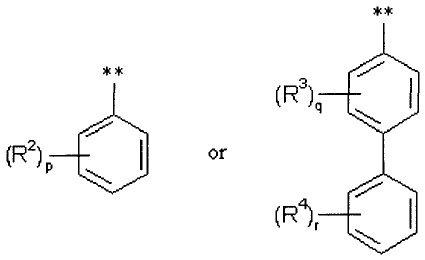 in which ** means the point of linkage to the group D,
R¹, R², R³ and R⁴ are independently of one another a substituent selected from the series fluorine, chlorine, bromine, methyl, *tert-*butyl, trifluoromethyl and methoxy,
and
o, p, q and r are independently of one another each the number 0, 1 or 2, where in the case that R¹, R², R³ or R⁴ occur more than once, their meanings may in each case be identical or different,
and the salts, solvates and solvates of the salts thereof.

5. Process for preparing compounds of the formula (I), (I-A) or (I-B) as defined in Claims 1 to 4, in which U, V and W together form a group of the formula *-CH=CH-CH<, in which * means the point of linkage to the phenyl ring, and Y is carboxyl,
**characterized in that** compounds of the formula (II) 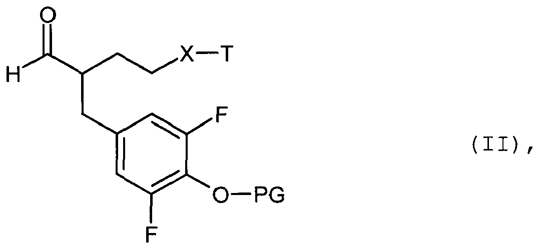 in which X has the meaning indicated in Claims 1 to 4, and
PG is a hydroxy protective group
and T is cyano or (C₁-C₄)-alkoxycarbonyl,
are either
[A-1] reacted in an inert solvent in the presence of a base with a compound of the formula (III-A) 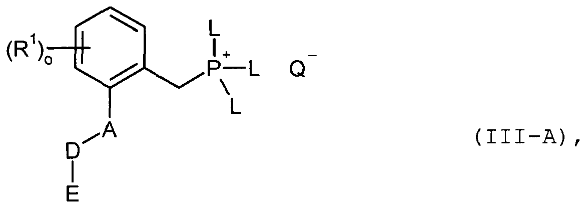 in which A, D, E, R¹ and o each have the meanings indicated in Claims 1 to 4, and
L is phenyl or o-, m- or p-tolyl
and
Q is halide or tosylate,
to give compounds of the formula (IV-A) 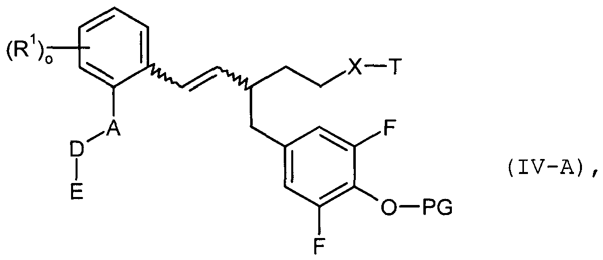 in which A, D, E, X, R¹, o, PG and T each have the meanings indicated above,
or
[A-2] reacted in an inert solvent in the presence of a base with a compound of the
formula (III-B) 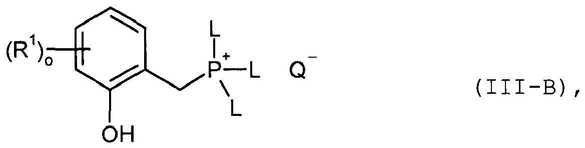 in which R¹, o, L and Q each have the meanings indicated above,
initially to give compounds of the formula (IV-B) 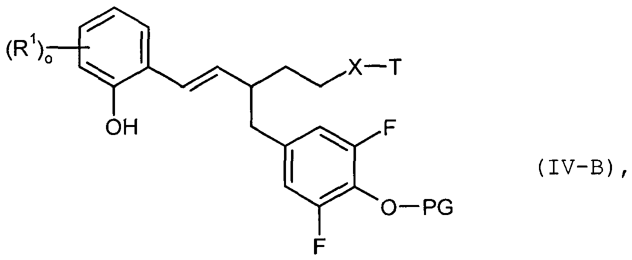 in which X, R¹, o, PG and T each have the meanings indicated above,
and these are then alkylated in an inert solvent in the, presence of a base with a compound of the formula (V)
E-D*-Z¹ (V),
in which E has the meaning indicated in Claims 1 to 4,
D* has the meaning of D indicated in Claims 1 to 4, but is not a bond,
and
Z¹ is a leaving group, such as, for example, halogen, tosylate or mesylate,
to give compounds of the formula (IV-C) 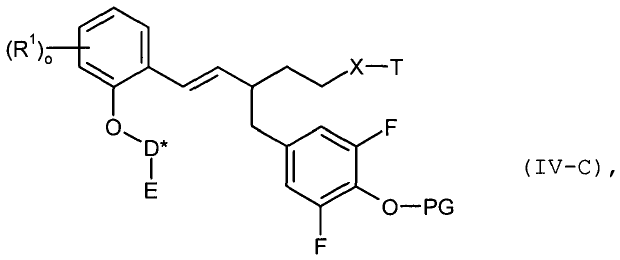 in which D*, E, X, R¹, o, PG and T each have the meanings indicated above,
the resulting compounds of the formula (IV-A) or (IV-C) are then converted by eliminating the protective group PG into compounds of the formula (VI) 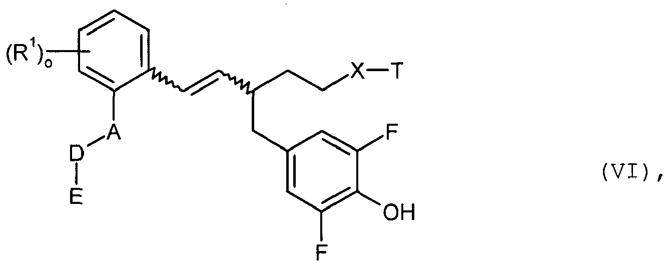 in which A, D, E, X, R¹, o and T each have the meanings indicated above,
and these are reacted by hydrolysis of the ester or nitrile group T to give carboxylic acids of the formula (I-C) 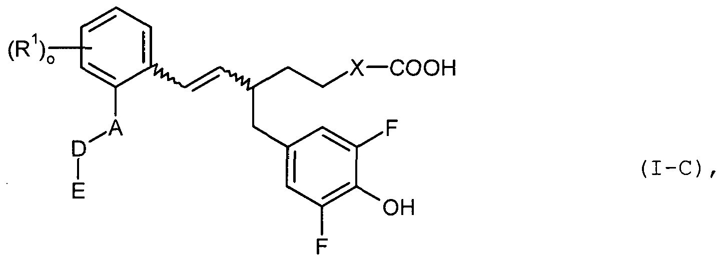 in which A, D, E, X, R¹ and o each have the meanings indicated above,
and the compounds of the formula (I-C) are separated where appropriate by methods known to the skilled person into their enantiomers and/or
diastereomers, and/or where appropriate converted with the appropriate (i) solvents and/or (ii) bases or acids into the solvates, salts and/or solvates of the salts thereof.

6. Process for preparing compounds of the formula (I) as defined in Claims 1 and 2, in which U, V and W together form a group of the formula *-CH₂-CH₂-N<, in which * means the point of linkage to the phenyl ring, and Y is carboxyl,
**characterized in that** compounds of the formula (XII) 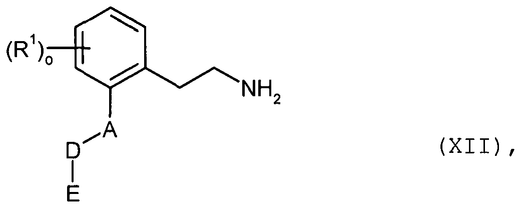 in which A, D, E, R¹ and o each have the meanings indicated in Claim 1 or 2,
are initially alkylated in an inert solvent in the presence of a base with a compound of the formula (VII) 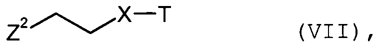 in which X has the meaning indicated in Claim 1 or 2, and
T is cyano or (C₁-C₄)-alkoxycarbonyl
and
Z² is a leaving group such as, for example, halogen, mesylate or tosylate,
to give compounds of the formula (XIII) 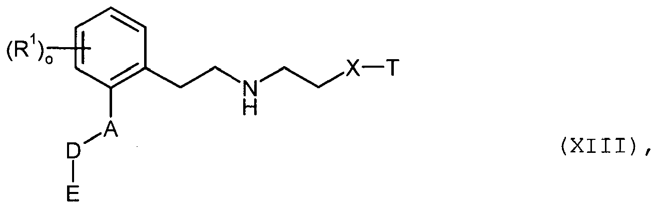 in which A, D, E, X, R¹, o and T each have the meanings indicated above,
subsequently reacted in an inert solvent in the presence of a base with a compound of the formula (VIII) 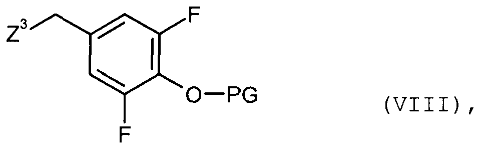 in which
PG is a hydroxy protective group
and
Z³ is a leaving group such as, for example, halogen, mesylate or tosylate,
to give compounds of the formula (XIV) 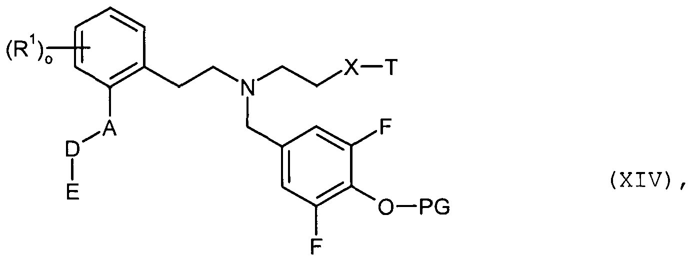 in which A, D, E, X, R¹, o, PG and T each have the meanings indicated above,
then converted by eliminating the protective group PG into compounds of the formula (XV) 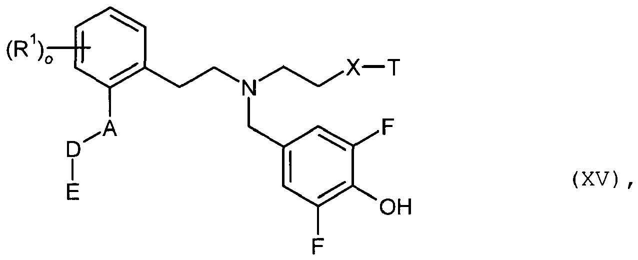 in which A, D, E, X, R¹, o and T each have the meanings indicated above,
and the latter are reacted by hydrolysis of the ester or nitrile group T to give carboxylic acids of the formula (I-D) 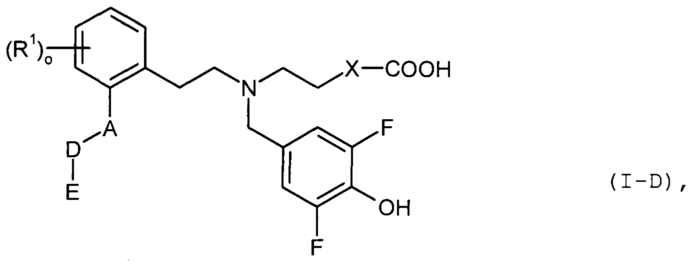 in which A, D, E, X, R¹ and o each have the meanings indicated above,
and the compounds of the formula (I-D) are where appropriate converted with the appropriate (i) solvents and/or (ii) bases or acids into the solvates, salts and/or solvates of the salts thereof.

7. Compound as defined in any of Claims 1 to 4 for the treatment and/or prevention of diseases.

8. Use of a compound as defined in any of Claims 1 to 4 for producing a medicament for the treatment and/or prevention of heart failure, angina pectoris, hypertension, pulmonary hypertension, ischemias, vascular disorders, thromboembolic disorders and arteriosclerosis.

9. Medicament comprising a compound as defined in any of Claims 1 to 4 in combination with an inert, nontoxic, pharmaceutically suitable excipient.

10. Medicament comprising a compound as defined in any of Claims 1 to 4 in combination with a further active ingredient selected from the group consisting of organic nitrates, NO donors, cGMP-PDE inhibitors, stimulators of guanylate cyclase, agents having antithrombotic activity, agents lowering blood pressure, and agents altering lipid metabolism.

11. Medicament according to Claim 9 or 10 for the treatment and/or prevention of heart failure, angina pectoris, hypertension, pulmonary hypertension, ischemias, vascular disorders, thromboembolic disorders and arteriosclerosis.

## Revendications

1. Composé de formule (I) 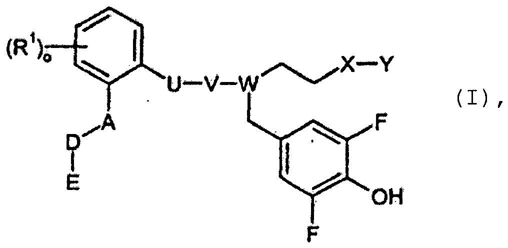 dans laquelle
U, V et W forment ensemble un groupe de formule *-CH=CH-CH<, *-CH₂-CH₂-CH< ou *-CH₂-CH₂-N<, où * représente le point d'attachement au cycle phényle,
A représente O ou CH₂,
D représente une liaison ou un groupe alcane(C₁-C₇)diyle, alcène(C₂-C₇)diyle ou alcyne(C₂-C₇)diyle, qui peuvent être substitués chacun une ou plusieurs fois par le fluor,
E représente un atome d'hydrogène, le groupe trifluorométhyle ou un groupe de formule 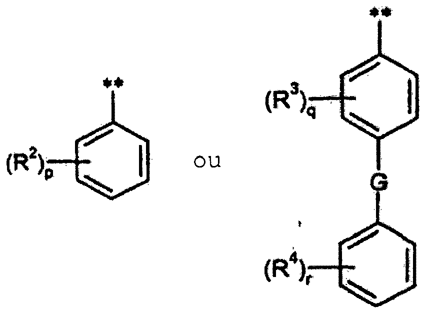 où ** représente le point d'attachement au groupe D et
G représente une liaison, CH₂, -CH₂-CH₂- ou -CH=CH-,
X représente -CH₂-CH₂- ou un groupe de formule 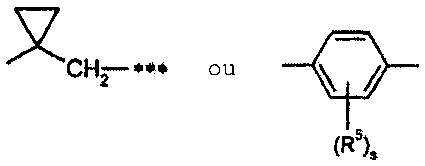 où *** représente le point d'attachement au groupe Y,
Y représente le groupe carboxy ou un groupe de formule 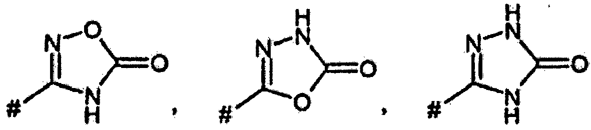 ou 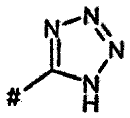 où # représente le point d'attachement respectif, R¹, R², R³, R⁴ et R⁵ représentent, indépendamment les uns des autres, un substituant choisi dans la série halogéno, alkyle en C₁-C₆, trifluorométhyle, alcoxy en C₁-C₆, trifluorométhoxy, cyano et nitro,
et
o, p, q, r et s représentent chacun, indépendamment les uns des autres, le nombre 0, 1, 2, 3 ou 4, dans le cas où R¹, R², R³, R⁴ ou R⁵ apparaissent plusieurs fois, leurs significations pouvant être les mêmes ou différentes,
ainsi que ses sels, produits de solvatation et produits de solvatation des sels.

2. Composé de formule (I) selon la revendication 1, dans lequel
U, V et W forment ensemble un groupe de formule *-CH=CH-CH< ou *-CH₂-CH₂-N<, où * représente le point d'attachement au cycle phényle,
A représente O,
D représente un groupe alcane(C₁-C₇)diyle qui peut être substitué une ou plusieurs fois par le fluor,
E représente un atome d'hydrogène, le groupe trifluorométhyle ou un groupe de formule 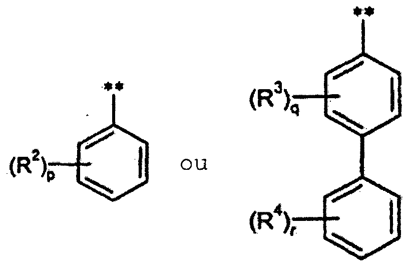 où ** représente le point d'attachement au groupe D,
X représente -CH₂-CH₂- ou un groupe de formule 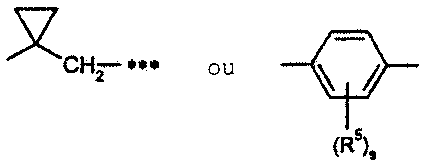 où *** représente le point d'attachement au groupe Y,
Y représente le groupe carboxy ou un groupe de formule 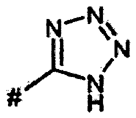 où # représente le point d'attachement,
R¹, R², R³ et R⁴ représentent, indépendamment les uns des autres, un substituant choisi dans la série fluoro, chloro, bromo, alkyle en C₁-C₄, trifluorométhyle, alcoxy en C₁-C₄ et trifluorométhoxy,
o, p, q et r représentent chacun, indépendamment les uns des autres, le nombre 0, 1 ou 2, dans le cas où R¹, R², R³ ou R⁴ apparaissent plusieurs fois, leurs significations pouvant être les mêmes ou différentes,
R⁵ représente un atome de fluor
et
s représente le nombre 0 ou 1,
ainsi que ses sels, produits de solvatation et produits de solvatation des sels.

3. Composé de formule (I-A) selon la revendication 1, 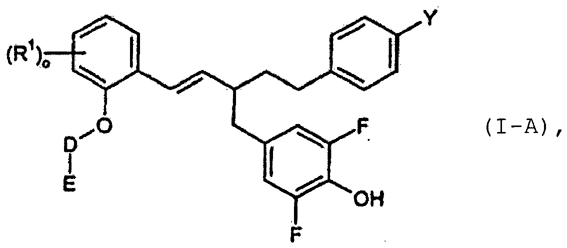 dans laquelle
D représente un groupe alcane(C₁-C₇)diyle qui peut être substitué une ou plusieurs fois par le fluor,
E représente un atome d'hydrogène, le groupe trifluorométhyle ou un groupe de formule 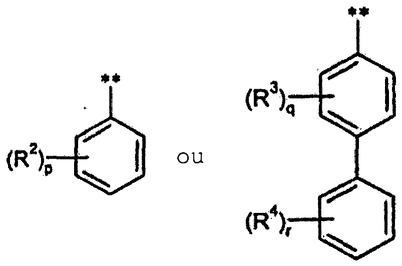 où ** représente le point d'attachement au groupe D,
Y représente le groupe carboxy ou un groupe de formule 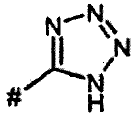 où # représente le point d'attachement,
R¹, R², R³ et R⁴ représentent, indépendamment les uns des autres, un substituant choisi dans la série fluoro, chloro, bromo, méthyle, tert-butyle, trifluorométhyle et méthoxy,
et
o, p, q et r représentent chacun, indépendamment les uns des autres, le nombre 0, 1 ou 2, dans le cas où R¹, R², R³ ou R⁴ apparaissent plusieurs fois, leurs significations pouvant être les mêmes ou différentes,
ainsi que ses sels, produits de solvatation et produits de solvatation des sels.

4. Composé de formule (I-B) selon la revendication 1, 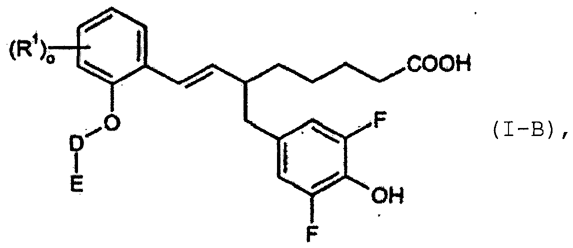 dans laquelle
D représente un groupe alcane(C₁-C₇)diyle qui peut être substitué une ou plusieurs fois par le fluor,
E représente un atome d'hydrogène, le groupe trifluorométhyle ou un groupe de formule 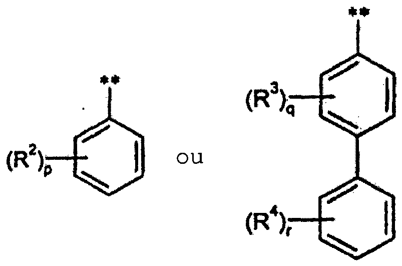 où ** représente le point d'attachement au groupe D,
R¹, R², R³ et R⁴ représentent, indépendamment les uns des autres, un substituant choisi dans la série fluoro, chloro, bromo, méthyle, tert-butyle, trifluorométhyle et méthoxy,
et
o, p, q et r représentent chacun, indépendamment les uns des autres, le nombre 0, 1 ou 2, dans le cas où R¹, R², R³ ou R⁴ apparaissent plusieurs fois, leurs significations pouvant être les mêmes ou différentes,
ainsi que ses sels, produits de solvatation et produits de solvatation des sels.

5. Procédé pour la préparation de composés de formule (I), (I-A) ou (I-B), tels que définis dans les revendications 1 à 4, dans lesquels U, V et W forment ensemble un groupe de formule *-CH=CH-CH<, où * représente le point d'attachement au cycle phényle, et Y représente le groupe carboxy,
**caractérisé en ce qu'**on fait réagir des composés de formule (II) 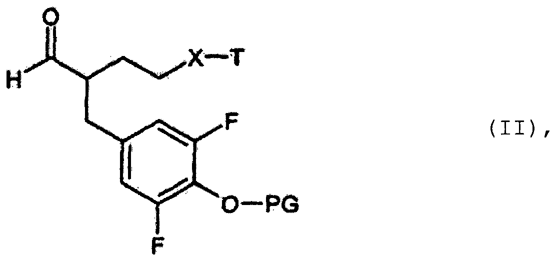 dans laquelle X a la signification indiquée dans les revendications 1 à 4 et
PG représente un groupe protecteur de fonction hydroxy
et
T représente le groupe cyano ou un groupe alcoxy(C₁-C₄)carbonyle,
soit
[A-1] dans un solvant inerte, en présence d'une base, avec un composé de formule (III-A) 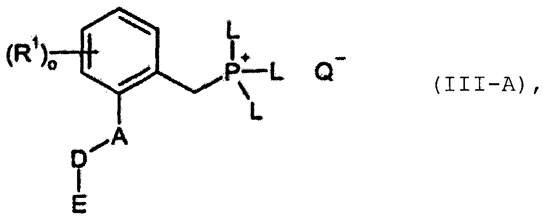 dans laquelle A, D, E, R¹ et o ont chacun les significations indiquées dans les revendications 1 à 4 et
L représente le groupe phényle ou o-, m- ou
p-tolyle
et
Q représente un halogénure ou tosylate,
pour aboutir à des composés de formule (IV-A) 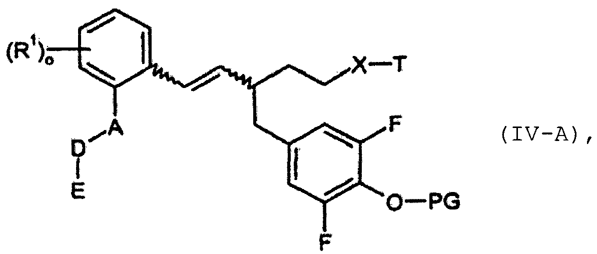 dans laquelle A, D, E, X, R¹, o, PG et T ont chacun les significations indiquées plus haut, soit
[A-2] d'abord, dans un solvant inerte, en présence d'une base, avec un composé de formule (III-B) 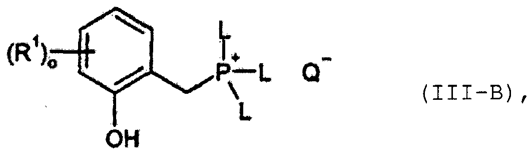 dans laquelle R¹, o, L et Q ont chacun les significations indiquées plus haut,
pour aboutir à des composés de formule (IV-B) 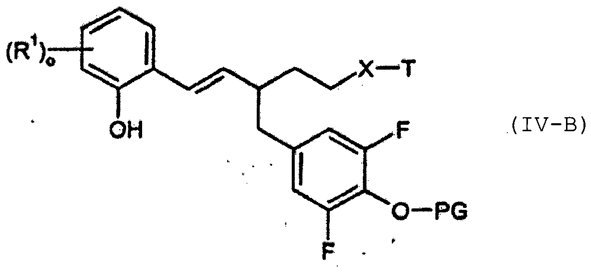 dans laquelle X, R¹, o, PG et T ont chacun les significations indiquées plus haut,
et ensuite, dans un solvant inerte, en présence d'une base, on soumet ces derniers à une alkylation avec un composé de formule (V)
E-D*-Z¹ (V),
dans laquelle E a la signification indiquée dans les revendications 1 à 4,
D* a la signification de D indiquée dans les revendications 1 à 4, mais ne représente pas une liaison,
et
Z¹ représente un groupe partant, comme par exemple halogéno, tosylate ou mésylate,
pour aboutir à des composés de formule (IV-C) 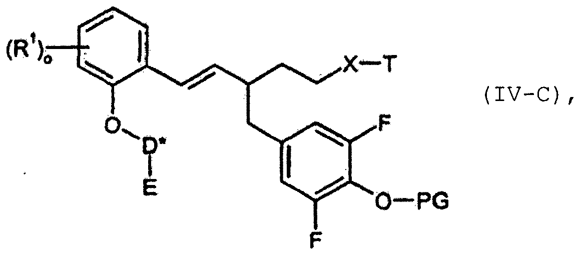 dans laquelle D*, E, X, R¹, o, PG et T ont chacun les significations indiquées plus haut,
ensuite on convertit les composés de formule (IV-A) ou respectivement (IV-C) résultants, par élimination du groupe protecteur PG, en composés de formule (VI) 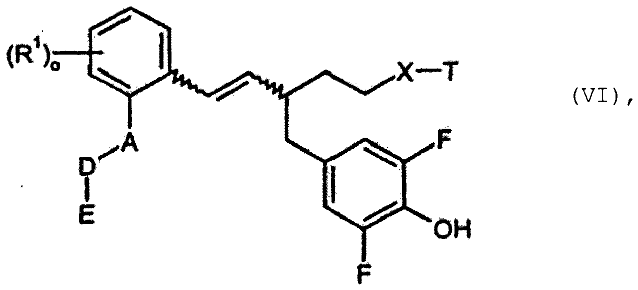 dans laquelle A, D, E, X, R¹, o et T ont chacun les significations indiquées plus haut,
et on convertit ces derniers, par hydrolyse du groupe ester ou respectivement nitrile T, en acides carboxyliques de formule (I-C) 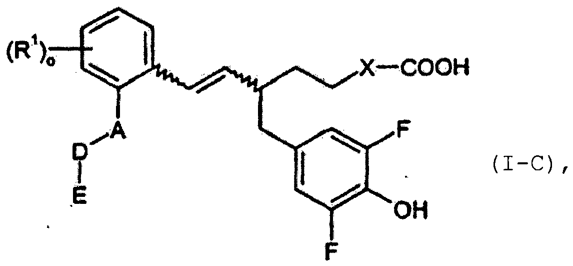 dans laquelle A, D, E, X, R¹ et o ont chacun les significations indiquées plus haut,
et éventuellement on fractionne les composés de formule (I-C), selon des méthodes connues de l'homme de métier, en leurs énantiomères et/ou diastéréoisomères et/ou éventuellement on les convertit, à l'aide (i) des solvants correspondants et/ou (ii) des bases correspondantes ou des acides correspondants, en leurs produits de solvatation, sels et/ou produits de solvatation des sels.

6. Procédé pour la préparation de composés de formule (I), tels que définis dans les revendications 1 et 2, dans laquelle U, V et W forment ensemble un groupe de formule *-CH₂-CH₂-N<, où * représente le point d'attachement au cycle phényle, et Y représente le groupe carboxyle,
**caractérisé en ce que** d'abord on soumet à une alkylation des composés de formule (XII) 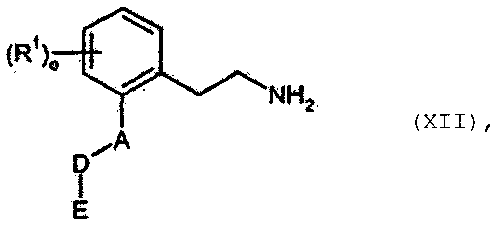 dans laquelle A, D, E, R¹ et o ont chacun les significations indiquées dans la revendication 1 ou 2, dans un solvant inerte, en présence d'une base, avec un composé de formule (VII) 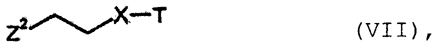 dans laquelle X a la signification indiquée dans la
revendication 1 ou 2 et
T représente un groupe cyano ou alcoxy(C₁-C₄)-carbonyle
et
Z² représente un groupe partant, comme par exemple halogéno, mésylate ou tosylate,
pour aboutir à des composés de formule (XIII) 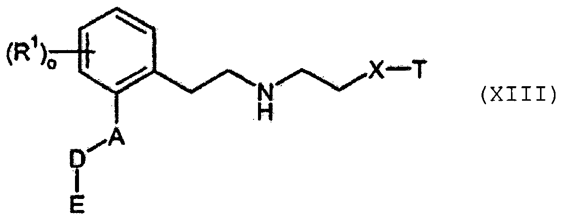 dans laquelle A, D, E, X, R¹, o et T ont chacun les significations indiquées plus haut,
ensuite on les fait réagir, dans un solvant inerte, en présence d'une base, avec un composé de formule (VIII) 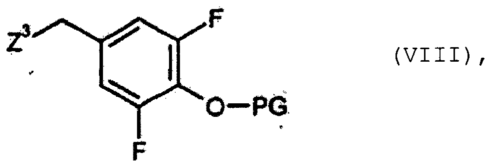 dans laquelle
PG représente un groupe protecteur de fonction hydroxy
et
Z³ représente un groupe partant, comme par exemple halogéno, mésylate ou tosylate,
pour aboutir à des composés de formule (XIV) 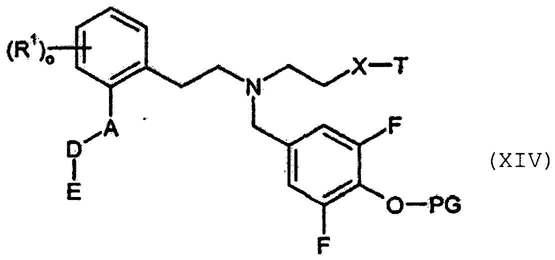 dans laquelle A, D, E, X, R¹, o, PG et T ont chacun les significations indiquées plus haut,
puis, on les convertit, par élimination du groupe protecteur PG, en composés de formule (XV) 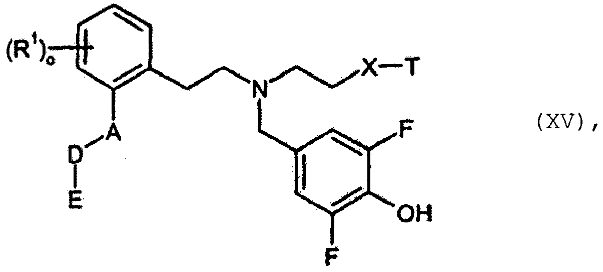 dans laquelle A, D, E, X, R¹, o et T on chacun les significations indiquées plus haut,
et on convertit ces derniers, par hydrolyse du groupe ester ou respectivement nitrile T, en acides carboxyliques de formule (I-D) 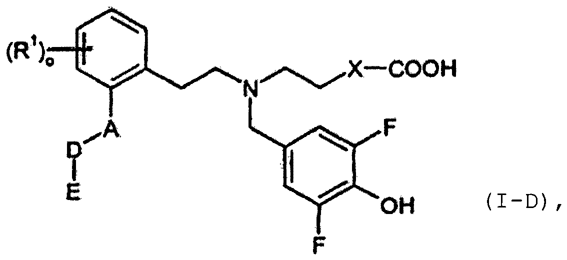 dans laquelle A, D, E, X, R¹ et o ont chacun les significations indiquées plus haut,
et éventuellement on convertit les composés de formule (I-D), à l'aide (i) des solvants correspondants et/ou (ii) des bases correspondantes ou des acides correspondants, en leurs produits de solvatation, sels et/ou produits de solvatation des sels.

7. Composé, tel que défini dans les revendications 1 à 4, destiné au traitement et/ou à la prévention de maladies.

8. Utilisation d'un composé, tel que défini dans l'une quelconque des revendications 1 à 4, pour la fabrication d'un médicament destiné au traitement et/ou à la prévention de l'insuffisance cardiaque, de l'angine de poitrine, de l'hypertonie, de l'hypertonie pulmonaire, d'ischémies, de maladies vasculaires, de maladies thromboemboliques et de l'artériosclérose.

9. Médicament contenant un composé, tel que défini dans l'une quelconque des revendications 1 à 4, en association avec un adjuvant inerte, non toxique, pharmaceutiquement convenable.

10. Médicament contenant un composé, tel que défini dans l'une quelconque des revendications 1 à 4, en association avec une autre substance active choisie dans le groupe constitué par des nitrates organiques, des donneurs de NO, des inhibiteurs de cGMP-PDE, des stimulants de la guanylate-cyclase, des agents à action antithrombotique, des agents abaissant la tension artérielle ainsi que des agents modifiant le métabolisme lipidique.

11. Médicament selon la revendication 9 ou 10, destiné au traitement et/ou à la prévention de l'insuffisance cardiaque, de l'angine de poitrine, de l'hypertonie, de l'hypertonie pulmonaire, d'ischémies, de maladies vasculaires, de maladies thromboemboliques et de l'artériosclérose.
